# EUROPEAN PATENT SPECIFICATION

(11) **EP 2 874 049 B1**
(45) Date of publication and mention of the grant of the patent: **17.10.2018**
(21) Application number: 13816298.7
(22) Date of filing: 27.06.2013
(51) Int. Cl.: G06F 3/041, H01B 5/14, C07D 401/14, C07D 409/14, C07D 471/04, C07D 491/04, C07D 519/00, C07F 7/10

(54) **TRANSPARENT ELECTRODE FOR TOUCH PANEL, TOUCH PANEL, AND DISPLAY DEVICE**
TRANSPARENTE ELEKTRODE FÜR BERÜHRUNGSBILDSCHIRM, BERÜHRUNGSBILDSCHIRM UND ANZEIGEVORRICHTUNG
ÉLECTRODE TRANSPARENTE POUR PANNEAU TACTILE, PANNEAU TACTILE, ET DISPOSITIF D'AFFICHAGE

(30) Priority: 11.07.2012 JP 2012155611; 27.08.2012 JP 2012187055
(43) Date of publication of application: 20.05.2015
(73) Proprietor: Konica Minolta, Inc., Tokyo 100-7015 (JP)
(72) Inventor: HAKII Takeshi, Tokyo 100-7015 (JP); ISHIDAI Hiroshi, Tokyo 100-7015 (JP); KINOSHITA Toshiyuki, Tokyo 100-7015 (JP); YOSHIDA Kazuhiro, Tokyo 100-7015 (JP); TADA Kazunari, Tokyo 100-7015 (JP)
(74) Representative: Gille Hrabal
(86) International application number: PCT/JP2013/067690
(87) International publication number: WO 2014/010433

(56) References cited:
- WO-A1-2010/110164
- WO-A1-2011/093309
- WO-A2-2007/022226
- JP-A- H06 278 244
- JP-A- 2001 164 382
- JP-A- 2010 146 944
- JP-A- 2010 283 003
- JP-A- 2012 022 460
- JP-A- 2012 089 361

## Description

### Technical Field

The present invention relates to a transparent electrode for a touch panel, a touch panel, and a display device, and particularly to a transparent electrode for a touch panel, which is suitable for thickness reduction, and a touch panel and a display device including the same.

### Background Art

There are various types of touch panels to be disposed on a display surface side of a display panel, such as a resistant film type, a surface capacitance type, a projected capacitance type, an optical type, and an ultrasonic type; in particular, the projected capacitance type has a feature in that the multipoint input is possible and therefore, the practical application thereof for smart phones and the like has been advanced.

Incidentally, in a touch panel with a structure in which electrodes are disposed across the entire surface of the panel like in the projected capacitance type, the visibility of the display image disposed through the patch panel is secured by structuring thin-film electrodes using a transparent conductive material. For the electrode of such a touch panel (i.e., transparent electrode), a metal oxide such as indium tin oxide (ITO) has been used mainly. The metal oxide such as ITO, however, has the excellent light-transmitting property but the conductivity thereof is not sufficient, whereby the voltage drop easily occurs around the center of the panel and the size increase of the touch panel is interrupted. For suppressing the resistance value of the transparent electrode as above, a certain degree of thickness is necessary. Therefore, in the case where the electrode has a pattern like in the projected capacitance type, the pattern easily becomes visible and in this case, the visibility of the display image to be the base is deteriorated.

In recent years, therefore, a structure including the metal nanowire with higher conductivity than ITO has been suggested as the transparent electrode for a touch panel (see, for example, Patent Literature 1 below).

### Citation List

### Patent Literature

Patent Literature 1: JP 2012-33466 A

Further reference may be made to JP2001/164382, JP2012/022460, JPH6278244, JP2012/89361, WO2011/093309, JP2010/283003, WO2010/110164, JP2010/146944 and WO2007/022226.

### Summary of Invention

### Technical Problem

The transparent electrode formed using the metal nanowire, however, has a problem in that when the amount of metal nanowire to be added in order to decrease the resistance is increased, the visibility of the display image to be the base is deteriorated due to the light scattering in the metal nanowire.

In view of this, an object of the present invention is to provide a transparent electrode for a touch panel, which enables the further size increase, and a touch panel that can be further increased in size by the use thereof, and a display device including the same.

### Solution to Problem

The above object of the present invention is achieved by the items as below.
1. A transparent electrode 1, 1a, 1b for a touch panel, comprising:
   a nitrogen-containing layer 3 formed using a compound containing nitrogen atoms; and
   an electrode layer 5 mainly containing silver and provided stacking on the nitrogen-containing layer 3,
   wherein the transparent electrode 1, 1a, 1b for a touch panel is characterized in that the nitrogen-containing layer (3) contains a compound represented by General Formula (1) below 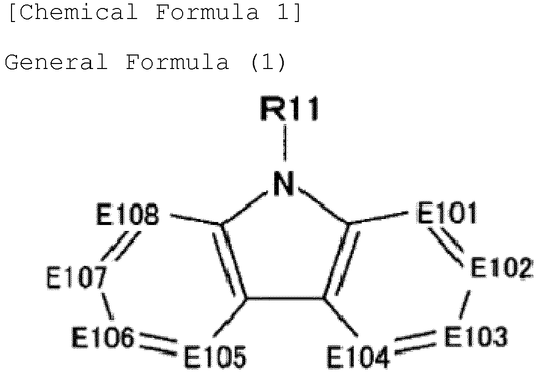
   [In General Formula (1), each of E101 to E108 represents -C(R12)= or -N=, at least one of E101 to E108 represents -N=, and R11 and R12 represent a hydrogen atom or a substituent.]
2. The transparent electrode 1, 1a, 1b for a touch panel according to item 1, characterized in that the nitrogen-containing layer 3 contains a compound represented by General Formula (2) below. 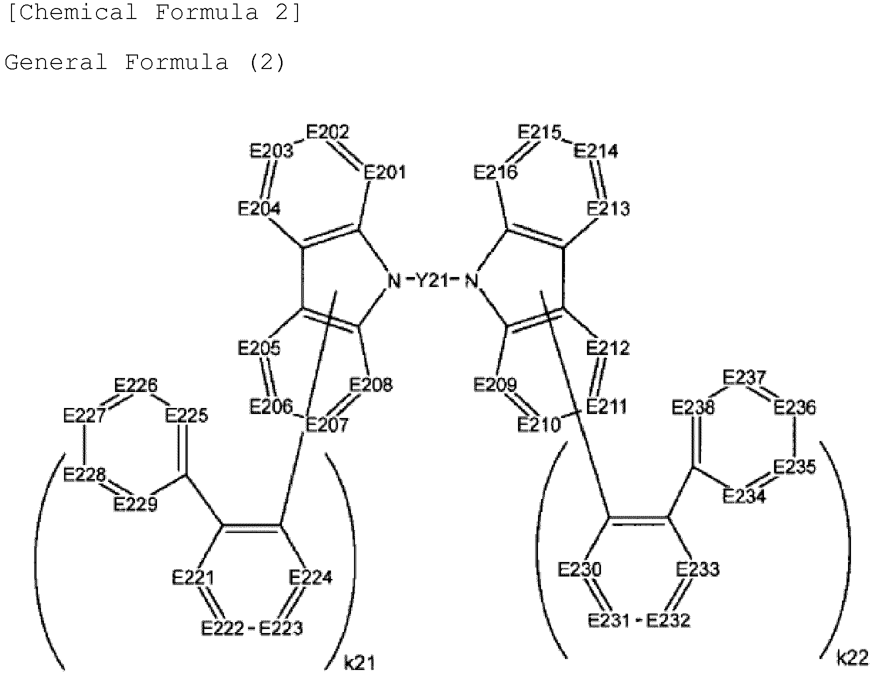 [In General Formula (2), Y21 represents an arylene group, a heteroarylene group, or a divalent connecting group including a combination thereof, each of E201 to E216 and E221 to E238 represents -C(R21)= or -N=, R21 represents a hydrogen atom or a substituent, at least one of E221 to E229 and at least one of E230 to E238 represent -N=, k21 and k22 represent an integer of 0 to 4 and k21 + k22 is an integer of 2 or more.]
3. The transparent electrode 1, 1a, 1b for a touch panel according to item 1, characterized in that the nitrogen-containing layer 3 contains a compound represented by General Formula (3) below. 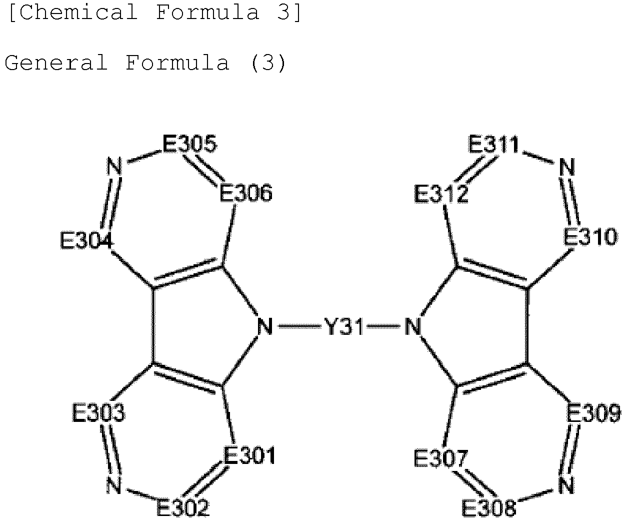 [In General Formula (3), each of E301 to E312 represents -C (R31) =, R31 represents a hydrogen atom or a substituent, and Y31 represents an arylene group, a heteroarylene group, or a divalent connecting group including a combination thereof.]
4. The transparent electrode 1, 1a, 1b for a touch panel according to item 1, characterized in that the nitrogen-containing layer 3 contains a compound represented by General Formula 4 below. 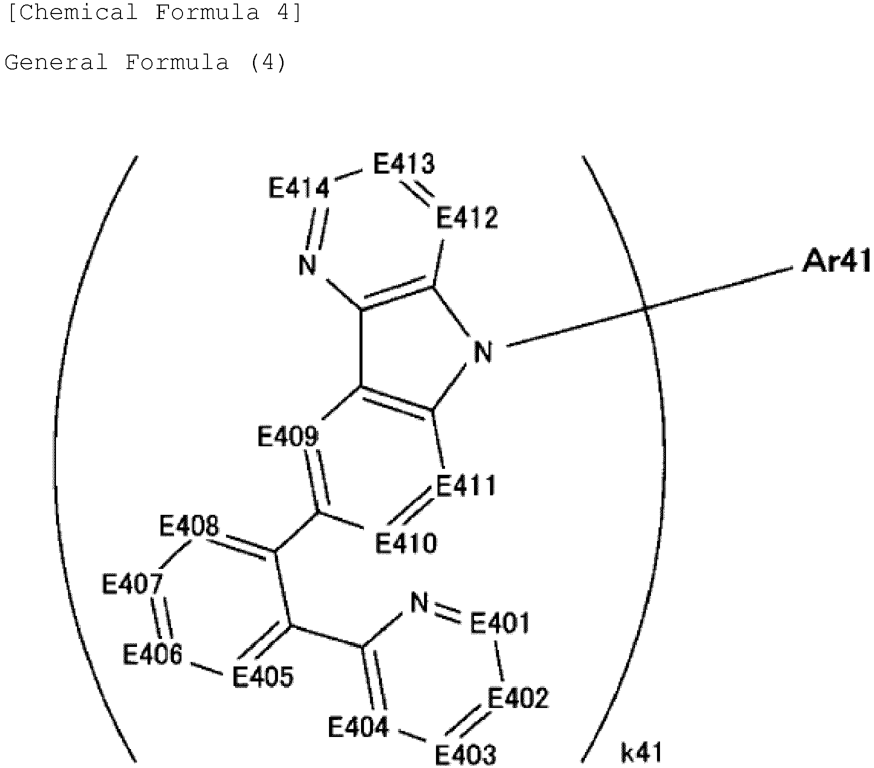 [In General Formula (4), each of E401 to E414 represents -C(R41) =, R41 represents a hydrogen atom or a substituent, Ar41 represents a substituted or unsubstituted aromatic hydrocarbon ring or heteroaromatic ring, and k41 represents an integer of 3 or more.]
5. The transparent electrode 1, 1a, 1b for a touch panel according to any one of items 1 to 4, characterized in that an effective unshared electron pair content ratio [n/M] of the compound containing nitrogen atoms satisfies 2.0 × 10⁻³ ≤ [n/M] , where n represents the number of unshared electron pairs which are not related to an aromatic property and are not coordinated to metal among unshared electron pairs of the nitrogen atoms and M represents the molecular weight of the compound.
6. The transparent electrode 1, 1a, 1b for a touch panel according to any one of items 1 to 4, characterized in that the effective unshared electron pair content ratio [n/M] of the compound satisfies 3.9 × 10⁻³ ≤ [n/M].
7. The transparent electrode 1, 1a, 1b for a touch panel according to one any of items 1 to 4, characterized in that the nitrogen-containing layer 3 is formed using the compound containing nitrogen atoms and using a mixture of such compound with another compound, and an average value of the effective unshared electron pair content ratio [n/M] considering a mixing ratio of these compounds satisfies 2.0 × 10⁻³ ≤ [n/M].
8. The transparent electrode 1, 1a, 1b for a touch panel according to any of items 1 to 4, characterized in that it further comprises a high-refractive-index layer H provided with the nitrogen-containing layer 3 interposed between the electrode layer 5 and the high-refractive-index layer H and having a higher refractive index than the nitrogen-containing layer 3.
9. The transparent electrode 1, 1a, 1b for a touch panel according to any one of items 1 to 4, characterized in that the high-refractive-index layer H is formed of titanium oxide or niobium oxide.
10. The transparent electrode 1, 1a, 1b for a touch panel according to any one of items 1 to 4, characterized in that the nitrogen-containing layer 3 has a thickness of 5 nm or less.
11. The transparent electrode 1, 1a, 1b for a touch panel according to any of items 1 to 4, characterized in that the nitrogen-containing layer 3 and the electrode layer 5 are provided adjacent to each other.
12. A touch panel 21, 23, 25, 27, 29 characterized in that it comprises the transparent electrode 1, 1a, 1b for a touch panel according to any of items 1 to 4.
13. A display device 31 characterized in that it comprises:
   the touch panel 21, 23, 25, 27, 29 according to any one of items 1 to 4; and
   a display panel 33 disposed overlapping with the touch panel.

The transparent electrode for a touch panel with the above structure is formed by stacking an electrode layer mainly containing silver on a nitrogen-containing layer formed using a compound containing nitrogen atoms, wherein the nitrogen-containing layer 3 contains a compound represented by any of the general Formulae (1) to (4) above. Thus, the silver atom included in the electrode layer mutually operates with the compound containing nitrogen atoms included in the nitrogen-containing layer, thereby reducing the diffusion distance of the silver atom on the nitrogen-containing layer surface and suppressing the aggregation of silver. Therefore, the electrode layer is formed by the film growth of the single layer growth type (Frank-van der Merwe: FM type) of a silver film, which is generally formed in an isolated island-shape due to the film growth of the nucleus growth type (Volume-Weber: VW type). Thus, the uniformly thin electrode layer can be obtained.

As a result, the transparent electrode for a touch panel with the electrode layer whose conductivity is secured by the uniform thickness while the light-transmitting property is secured by the small thickness can be achieved.

### Advantageous Effects of Invention

As thus described, in the present invention, both the conductivity and the light-transmitting property of the electrode layer mainly containing silver can be improved; thus, the size of the transparent electrode for a touch panel including this electrode layer can be increased. Further, the touch panel and the display device including the transparent electrode for a touch panel can be increased in size.

### Brief Description of Drawings

Fig. 1 is a schematic sectional diagram illustrating a transparent electrode for a touch panel according to an embodiment of the present invention.
Fig. 2 is a schematic sectional diagram illustrating a first modified example of the transparent electrode for a touch panel.
Fig. 3 is a schematic sectional diagram illustrating a second modified example of the transparent electrode for a touch panel.
Fig. 4 is a perspective diagram illustrating a structure of a touch panel according to the embodiment.
Fig. 5 is a plan diagram illustrating each transparent electrode of the touch panel according to the embodiment.
Fig. 6 is a schematic plan diagram illustrating an electrode portion of the touch panel according to the embodiment.
Fig. 7 is a schematic sectional diagram illustrating a structure of the touch panel according to the embodiment.
Fig. 8 is a schematic sectional diagram illustrating a first modified example of the touch panel.
Fig. 9 is a schematic sectional diagram illustrating a second modified example of the touch panel.
Fig. 10 is a schematic sectional diagram illustrating a third modified example of the touch panel.
Fig. 11 is a schematic sectional diagram illustrating a fourth modified example of the touch panel.
Fig. 12 is a perspective diagram illustrating a structure of a display device.
Fig. 13 is a graph representing the relation between the sheet resistance and the effective unshared electron pair content ratio [n/M] of the nitrogen-containing layer included in the transparent electrode.

### Description of Embodiments

One embodiment of the present invention is hereinafter described with reference to the drawings in the following order:
1. Transparent electrode for touch panel
2. First modified example of transparent electrode for touch panel (example in which intermediate layer is provided)
3. Second modified example of transparent electrode for touch panel (example in which high-refractive-index layer is provided)
4. Touch panel (Structure 1 in which two-layer transparent electrode is provided on transparent substrate)
5. First modified example of touch panel (Structure 2 in which two-layer transparent electrode is provided on transparent substrate)
6. Second modified example of touch panel (structure in which two transparent substrates are used)
7. Third modified example of touch panel (structure in which transparent electrode is provided on each surface of transparent substrate)
8. Fourth modified example of touch panel (structure in which two patterns of transparent electrodes are provided on the same plane of transparent substrate)
9. Display device (structure in which touch panel is used)

### <<1. Transparent electrode for touch panel>>

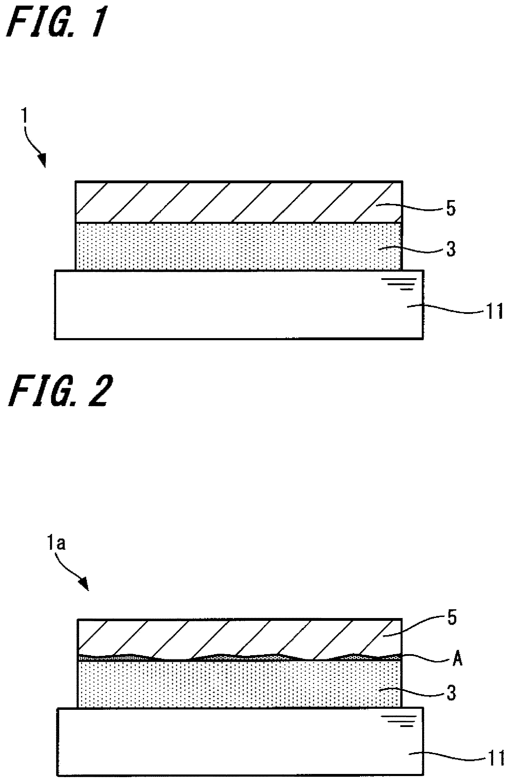

Fig. 1 is a schematic sectional diagram illustrating a structure of a transparent electrode for a touch panel (hereinafter referred to as transparent electrode) according to an embodiment of the present invention. As illustrated in this drawing, a transparent electrode 1 has a two-layer structure in which a nitrogen-containing layer 3 and an electrode layer 5 are stacked, and the nitrogen-containing layer 3 and the electrode layer 5 are provided in this order on a transparent substrate 11, for example. Among these, the electrode layer 5 constituting a substantial electrode portion in the transparent electrode 1 is the layer formed to contain silver (Ag) mainly and is stacked in the state being adjacent to the nitrogen-containing layer 3. On the other hand, the nitrogen-containing layer 3 disposed adjacent to the electrode layer 5 is formed using a compound containing nitrogen atoms (N) stably coupled with silver, which is the main material of the electrode layer 5. The nitrogen-containing layer 3 contains a compound represented by any General Formulae (1) to (4) above.

Detailed description is hereinafter made of the structures of the transparent substrate 11, on which the transparent electrode 1 with the above multilayer structure is provided, the nitrogen-containing layer 3 included in the transparent electrode 1, and the electrode layer 5 in this order. Note that the transparency of the transparent electrode 1 of the present invention refers to a light transmittance of 50% or more at a wavelength of 550 nm.

### <Transparent substrate 11>

The transparent substrate 11 where the transparent electrode 1 of the present invention is formed may be the substrate that also serves as a front plate of a display panel, for example. The transparent substrate 11 as above may be, for example, glass, quartz, or a transparent resin film.

The glass may be, for example, silica glass, soda-lime silica glass, lead glass, borosilicate glass, non-alkaline glass, or the like. A surface of the glass material is subjected to a physical process such as polishing as necessary from the viewpoint of the adhesion with the nitrogen-containing layer 3, the durability, and the smoothness or is provided with an inorganic or organic film or with a hybrid film in which those films are combined.

As the resin film, for example, any of polyesters such as polyethylene (PET) and polyethylene naphthalate (PEN), polyethylene, polypropylene, cellulose esters such as cellophane, cellulose diacetate, cellulose triacetate (TAC), cellulose acetate butylate, cellulose acetate propionate (CAP), cellulose acetate phthalate, and cellulose nitrate, and derivatives thereof, polyvinylidene fluoride, polyvinyl alcohol, polyethylene alcohol, syndiotactic polystyrene, polycarbonate, a norbornene resin, polymethylene pentene, polyether ketone, polyimide, polyether sulfone (PES), polyphenylene sulfide, polysulfones, polyether imide, polyether ketone imide, polyamide, a fluorine resin, nylon, polymethyl methacrylate, acrylic, polyarylates, ARTON (product name, manufactured by JSR Corporation) or APEL (product name, manufactured by Mitsui Chemicals, Inc.) or other cycloolefin-based resins are given.

A surface of the resin film may be provided with an inorganic or organic film or a hybrid film in which these films are combined. Those films and the hybrid film are preferably the films with the barrier property (also called barrier films or the like) whose water vapor permeation (25±0.5°C, relative humidity 90±2% RH) is 0.01 g (m²·24 hours) or less that is measured by a method based on JIS-K-7129-1992. Moreover, the film with the high barrier property whose oxygen permeation is 10⁻³ ml/ (m²·24 hours·atm) or less and water vapor permeation is 10⁻⁵ g/ (m²·24 hours) or less that is measured by a method based on JIS-K-7126-1987 is preferable.

The material of the aforementioned film with the barrier property may be the material with a function of suppressing the intrusion of substances that deteriorate the element, such as moisture or oxygen; for example, silicon oxide, silicon dioxide, silicon nitride, or the like can be used. Moreover, it is more preferable that these inorganic layers and layer of an organic material (organic layer) are stacked for overcoming the weakness of the film with the barrier property. The order of stacking the inorganic layer and the organic layer is not particularly limited; however, the both are preferably stacked alternately a plurality of times.

A method of forming the film with the barrier property is not particularly limited; for example, a vacuum evaporation method, a sputtering method, a reactive sputtering method, a molecular beam epitaxy method, a cluster ion beam method, an ion plating method, a plasma polymerization method, an atmospheric plasma polymerization method, a plasma CVD method, a laser CVD method, a thermal CVD method, a coating method, or the like can be employed. In particular, the atmospheric plasma polymerization method according to JP-A-2004-68143 is preferable.

### <Nitrogen-containing layer 3>

The nitrogen-containing layer 3 is provided stacked on the electrode layer 5, and here is provided adjacent to the electrode layer 5. The nitrogen-containing layer 3 as above is formed using a compound containing nitrogen atoms (N) represented by any of the general Formulae (1) to (4) above. As the compound containing nitrogen atoms, [Compounds 1 to 3] as below are given.

### [Compound-1]

A preferred example of the compound included in the nitrogen-containing layer 3 is a compound in which: the content ratio of [effective unshared electron pair], which is the unshared electrode pair of nitrogen atoms that are stably coupled with silver serving as the main material of the electrode layer 5 out of the nitrogen atoms contained in the compound, is within a predetermined range.

Here, [effective unshared electron pair] refers to the unshared electron pair that is not related to the aromatic property and is not coordinated to metal, out of the unshared electron pairs of the nitrogen atoms contained in the compound. Here, the aromatic property refers to the unsaturated cyclic structure in which the atoms with π electrons are arranged in the cyclic manner, and based on Huckel's rule, satisfies the condition that the number of electrons included in the π electron system on the cycle is [4n + 2] (n = 0 or a natural number).

The aforementioned [effective unshared electron pair] is selected depending on whether the unshared electron pair of the nitrogen atom is related to the aromatic property regardless of whether the nitrogen atom having the unshared electron pair is the hetero atom constituting a part of the aromatic ring or not. For example, even though a certain nitrogen atom is a hetero atom of the aromatic ring, if that nitrogen atom has the unshared electron pair that is not related to the aromatic property, the unshared electron pair is counted as one of [effective unshared electron pairs]. On the other hand, even though a certain nitrogen atom is not the hetero atom of the aromatic ring, if all the unshared electron pairs of the nitrogen atom are related to the aromatic property, the unshared electron pair of that nitrogen atom is not counted as the [effective unshared electron pair]. In each compound, the number n of [effective unshared electron pairs] coincides with the number of nitrogen atoms having [effective unshared electron pair].

In this embodiment, in particular, the number n of [effective unshared electron pairs] relative to the molecular weight M of this compound is defined as, for example, the effective unshared electron pair content ratio [n/M]. It is preferable that the nitrogen-containing layer 3 is formed by using the compound selected so that this [n/M] satisfies 2.0 × 10⁻³ ≤ [n/M]. Moreover, it is more preferable that the effective unshared electron pair content ratio [n/M] of the nitrogen-containing layer 3 that is defined as above is in the range of 3.9 × 10⁻³ ≤ [n/M].

The nitrogen-containing layer 3 may be formed using a compound whose effective unshared electron pair content ratio [n/M] is in the aforementioned range, and may be formed using such a compound only or using a mixture of such a compound with another compound. The other compound may or may not contain the nitrogen atoms and the effective unshared electron pair content ratio [n/M] thereof may be out of the aforementioned range.

When the nitrogen-containing layer 3 is formed using a plurality of compounds, it is preferable that the molecular weight M of a mixture compound in which these compounds are mixed is calculated based on the mixing ratio of the compounds, the total number n of [effective unshared electron pairs] relative to this molecular weight M is calculated as the average value of the effective unshared electron pair content ratio [n/M], and this value is in the aforementioned predetermined range. In other words, it is preferable that the effective unshared electron pair content ratio [n/M] of the nitrogen-containing layer 3 itself is in the predetermined range.

Note that when the nitrogen-containing layer 3 is formed using a plurality of compounds and the mixing ratio (content ratio) of the compounds is different in the film thickness direction, the effective unshared electron pair content ratio [n/M] on the surface layer of the nitrogen-containing layer 3 on the side in contact with the electrode layer 5 is preferably in the predetermined range.

As the compound included in the nitrogen-containing layer 3, specific examples of the compound whose effective unshared electron pair content ratio [n/M] satisfies 2.0 × 10⁻³ ≤ [n/M] are shown (No. 1 to No. 37). Each of Compounds No. 1 to No. 37 has a circular mark for the nitrogen atom with the [effective unshared electron pair] . Table 1 below shows the molecular weight M, the number n of [effective unshared electron pairs], and the effective unshared electron pair content ratio [n/M] of Compounds No. 1 to No. 37. In copper phthalocyanine of Compound 33, the unshared electron pair that is not coordinated to copper among the unshared electron pairs of the nitrogen atoms is counted as the [effective unshared electron pair] . The compounds in Chemical Formulae 7 to 11 which are not according to General formula 1 of claim 1 are: No. 1, No.2, No. 3, No. 4, No. 5, No. 6, No. 9, No. 11, No. 12, No. 14, No. 15, No. 16, No. 17, No. 18, No. 21, No. 24, No. 25, No. 28, No. 29, No. 32, No. 33, No. 34, No. 35, No. 36, No. 37 (see also table 1 below).

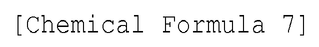

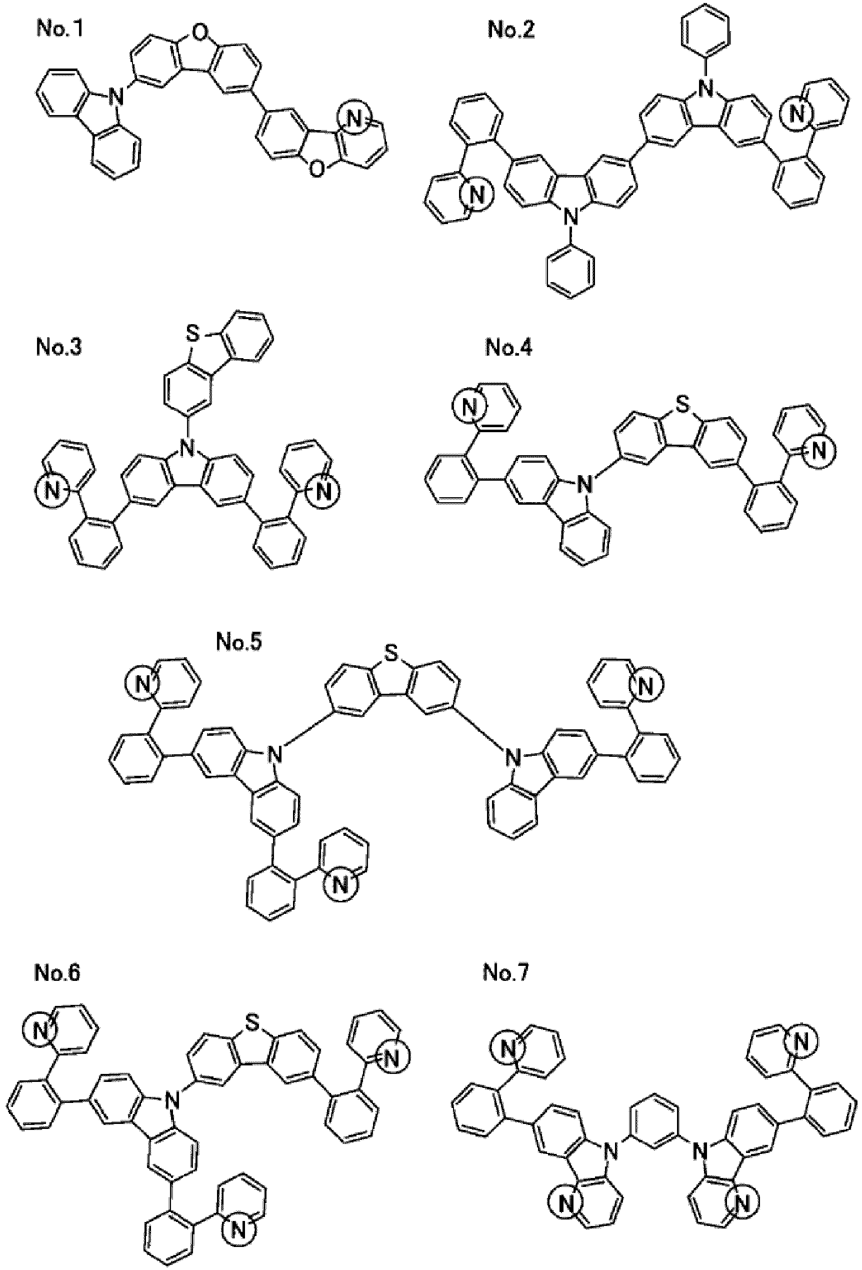

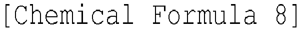

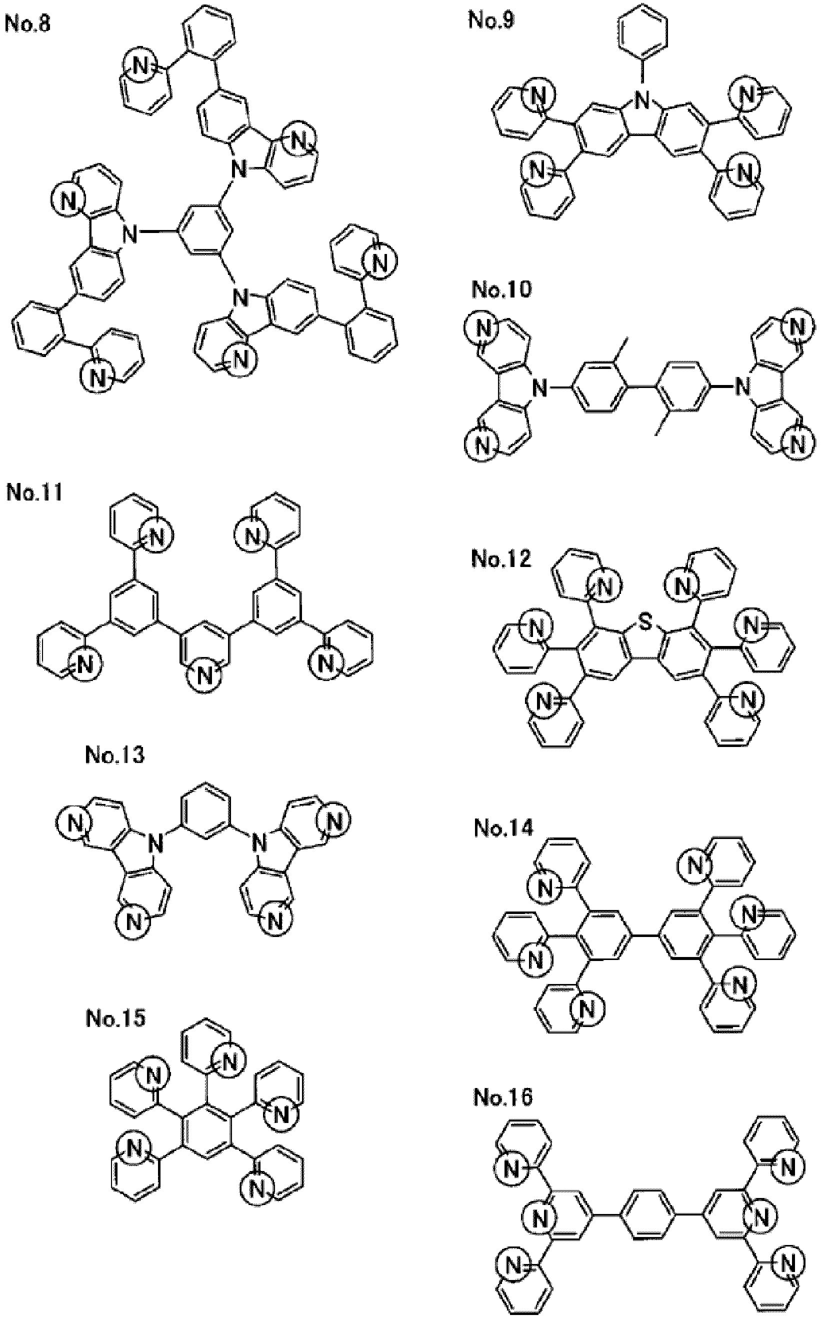

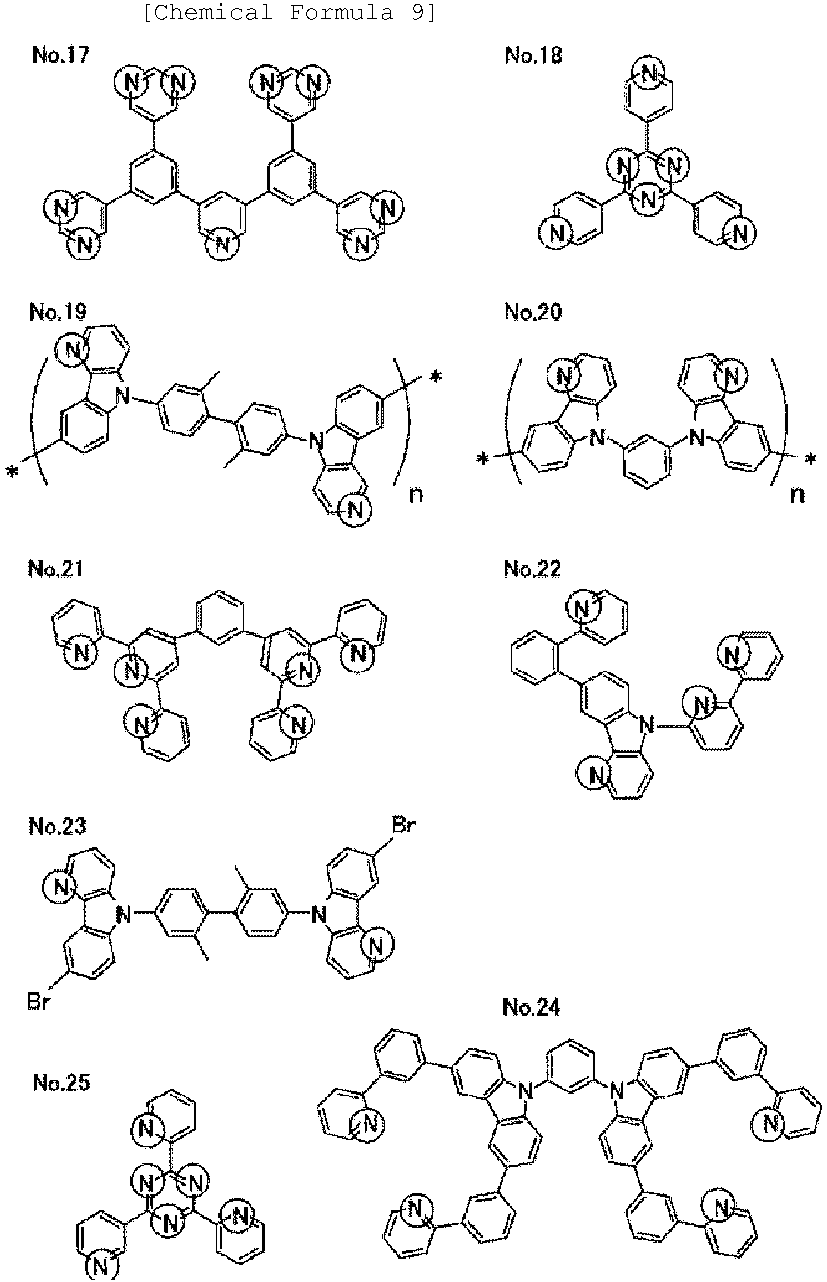

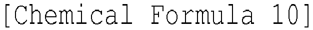

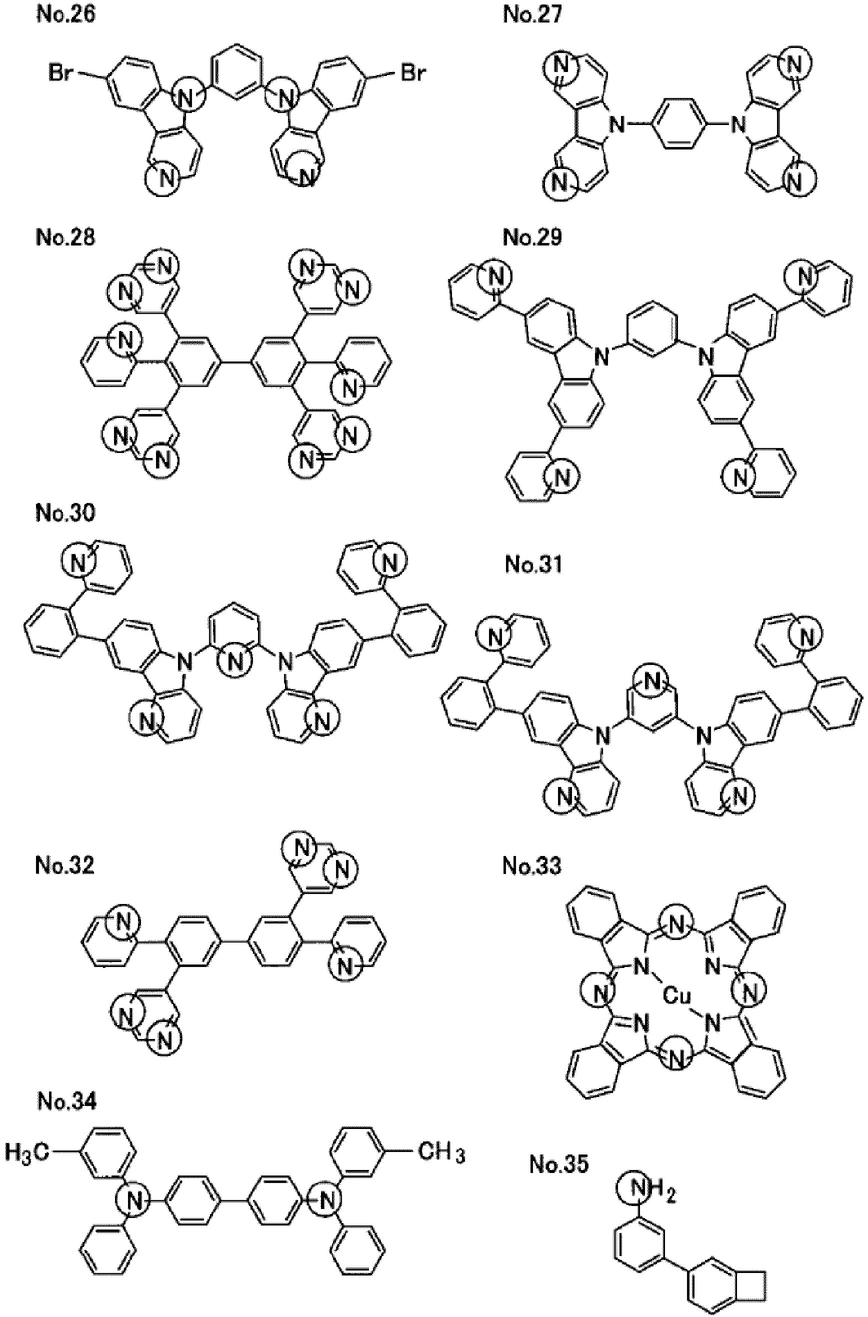

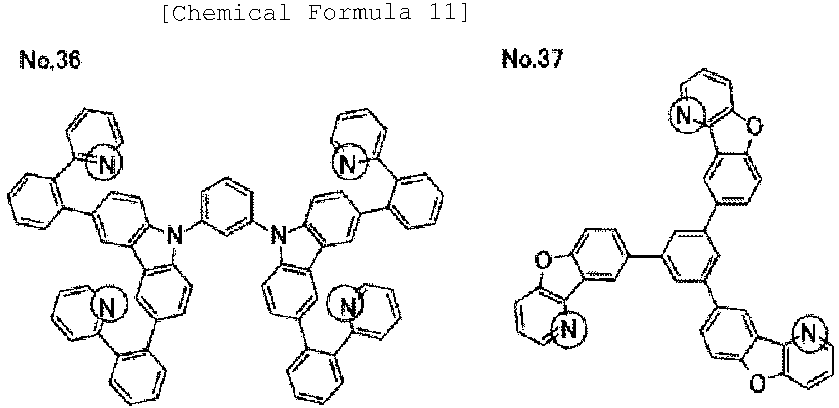

**[Table 1]**

| Compound | Number [n] of effective unshared electron pairs | Molecular weight [M] | [n/M] | Corresponding General Formula |
|---|---|---|---|---|
| No. 1 | 1 | 500.55 | 2.0E-03 | * |
| No. 2 | 2 | 790.95 | 2.5E-03 | * |
| No. 3 | 2 | 655.81 | 3.0E-03 | * |
| No. 4 | 2 | 655.81 | 3.0E-03 | * |
| No. 5 | 3 | 974.18 | 3.1E-03 | * |
| No. 6 | 3 | 808.99 | 3.7E-03 | * |
| No. 7 | 4 | 716.83 | 5.6E-03 | (1), (2) |
| No. 8 | 6 | 1036.19 | 5.8E-03 | (1), (4) |
| No. 9 | 4 | 551.64 | 7.3E-03 | * |
| No. 10 | 4 | 516.60 | 7.7E-03 | (1), (3) |
| No. 11 | 5 | 539.63 | 9.3E-03 | * |
| No. 12 | 6 | 646.76 | 9.3E-03 | * |
| No. 13 | 4 | 412.45 | 9.7E-03 | (1), (3) |
| No. 14 | 6 | 616.71 | 9.7E-03 | * |
| No. 15 | 5 | 463.53 | 1.1E-02 | * |
| No. 16 | 6 | 540.62 | 1.1E-02 | * |
| No. 17 | 9 | 543.58 | 1.7E-02 | * |
| No. 18 | 6 | 312.33 | 1.9E-02 | * |
| No. 19 | 2 | 512.60 | 3.9E-03 | (1) |
| No. 20 | 2 | 408.45 | 4.9E-03 | (1) |
| No. 21 | 6 | 540.62 | 1.1E-02 | * |
| No. 22 | 4 | 475.54 | 8.4E-03 | (1) |
| No. 23 | 2 | 672.41 | 3.0E-03 | (1) |
| No. 24 | 4 | 1021.21 | 3.9E-03 | * |
| No. 25 | 6 | 312.33 | 1.9E-02 | * |
| No. 26 | 4 | 568.26 | 7.0E-03 | (1) |
| No. 27 | 4 | 412.45 | 9.7E-03 | (1), (3) |
| No. 28 | 10 | 620.66 | 1.6E-02 | * |
| No. 29 | 4 | 716.83 | 5.6E-03 | * |
| No. 30 | 5 | 717.82 | 7.0E-03 | (1), (2) |
| No. 31 | 5 | 717.82 | 7.0E-03 | (1), (2) |
| No. 32 | 6 | 464.52 | 1.3E-02 | * |
| No. 33 | 4 | 576.10 | 6.9E-03 | * |
| No. 34 | 2 | 516.67 | 3.9E-03 | * |
| No. 35 | 1 | 195.26 | 5.1E-03 | * |
| No. 36 | 4 | 1021.21 | 3.9E-03 | * |
| No. 37 | 3 | 579.60 | 5.2E-03 | * |

| | | | | |
|---|---|---|---|---|
| *: These compounds are not according to General formula 1 of claim 1. | | | | |

Note that Table 1 shows, in the case where the compound given therein belongs to any of General Formulae (1) to (4) representing [Compound-2] described below, the corresponding general formula.

### [Compound-2]

As another example of the compound included in the nitrogen-containing layer 3, in addition to the aforementioned compound whose effective unshared electron pair content ratio [n/M] is in the above range, the compound represented by any of General Formulae (1) to (4) given below is applicable from the viewpoint of the film-forming property.

The compounds represented by General Formulae (1) to (4) include the compound whose effective unshared electron pair content ratio [n/M] is in the above range, and such compounds can be used alone preferably as the compound included in the nitrogen-containing layer 3 (see Table 1) . On the other hand, if the compounds represented by General Formulae (1) to (4) are the compounds whose effective unshared electron pair content ratio [n/M] is out of the above range, the compound may be mixed with the compound whose effective unshared electron pair content ratio [n/M] is in the above range; thus, the obtained compound can be suitably used as the compound included in the nitrogen-containing layer 3.

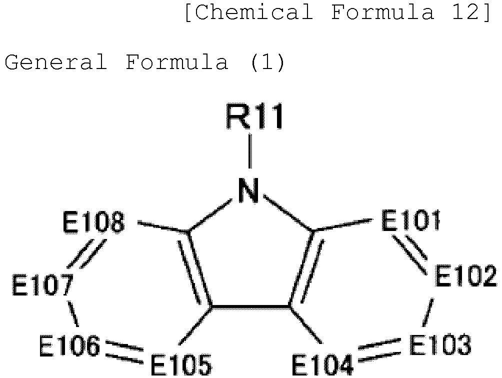

In the above General Formula (1), each of E101 to E108 represents -C(R12)= or -N=, and at least one of E101 to E108 is -N=. Moreover, in the above General Formula (1), R11 and R12 represent a hydrogen atom or a substituent.

Examples of the substituent include an alkyl group (such as a methyl group, an ethyl group, a propyl group, an isopropyl group, a tert-butyl group, a pentyl group, a hexyl group, an octyl group, a dodecyl group, a tridecyl group, a tetradecyl group, or a pentadecyl group), a cycloalkyl group (such as a cyclopentyl group or a cyclohexyl group), an alkenyl group (such as a vinyl group or an allyl group), an alkynyl group (such as an ethynyl group or a propargyl group), an aromatic hydrocarbon group (also called an aromatic carbon ring group, an aryl group or the like, typified by a phenyl group, a p-chlorophenyl group, a mesityl group, a tolyl group, a xylyl group, a naphthyl group, an anthryl group, an azulenyl group, an acenaphthenyl group, a fluorenyl group, a phenanthryl group, an indenyl group, a pyrenyl group, or a biphenyl group), a heteroaromatic ring group (such as a furyl group, a thienyl group, a pyridyl group, a pyridazinyl group, a pyrimidinyl group, a pyrazinyl group, a triazinyl group, an imidazolyl group, a pyrazolyl group, a thiazolyl group, a quinazolinyl group, a carbazolyl group, a carbolinyl group, a diaza carbazolyl group (representing the group obtained by replacing any one of carbon atoms included in a carboline ring of the carbolinyl group with a nitrogen atom), or a phthalazinyl group), a heterocycle group (such as a pyrrolidyl group, an imidazolidinyl group, a morpholyl group, or an oxazolidyl group), an alkoxy group (such as a methoxy group, an ethoxy group, a propyloxy group, a pentyloxy group, a hexyloxy group, an octyloxy group, or a dodecyloxy group), a cycloalkoxy group (such as a cyclopentyloxy group or a cyclohexyloxy group), an aryloxy group (such as a phenoxy group or a naphthyloxy group), an alkylthio group (such as a methylthio group, an ethylthio group, a propylthio group, a pentylthio group, a hexylthio group, an octylthio group, or a dodecylthio group), a cycloalkylthio group (such as a cyclopentylthio group or a cyclohexylthio group), an arylthio group (such as a phenylthio group or a naphthylthio group), an alkoxycarbonyl group (such as a methyloxycarbonyl group, an ethyloxycarbonyl group, a butyloxycarbonyl group, an octyloxycarbonyl group, or a dodecyloxycarbonyl group), an aryloxycarbonyl group (such as a phenyloxycarbonyl group or a naphthyloxycarbonyl group), a sulfamoyl group (such as an aminosulfonyl group, a methylaminosulfonyl group, a dimethylaminosulfonyl group, a butylaminosulfonyl group, a hexylaminosulfonyl group, a cyclohexylaminosulfonyl group, an octylaminosulfonyl group, a dodecylaminosulfonyl group, a phenylaminosulfonyl group, a naphthylaminosulfonyl group, or a 2-pyridylaminosulfonyl group), an acyl group (such as an acetyl group, an ethylcarbonyl group, a propylcarbonyl group, a pentylcarbonyl group, a cyclohexylcarbonyl group, an octylcarbonyl group, a 2-ethylhexylcarbonyl group, a dodecylcarbonyl group, a phenylcarbonyl group, a naphthylcarbonyl group, or a pyridylcarbonyl group), an acyloxy group (such as an acetyloxy group, an ethylcarbonyloxy group, a butylcarbonyloxy group, an octylcarbonyloxy group, a dodecylcarbonyloxy group, or a phenylcarbonyloxy group), an amide group (such as a methylcarbonyl amino group, an ethylcarbonyl amino group, a dimethylcarbonyl amino group, a propylcarbonyl amino group, a pentylcarbonyl amino group, a cyclohexylcarbonyl amino group, a 2-ethylhexylcarbonyl amino group, an octylcarbonyl amino group, a dodecylcarbonyl amino group, a phenylcarbonylamino group, or a naphthylcarbonyl amino group), a carbamoyl group (such as an aminocarbonyl group, a methylaminocarbonyl group, a dimethylaminocarbonyl group, a propylaminocarbonyl group, a pentylaminocarbonyl group, a cyclohexylaminocarbonyl group, an octylaminocarbonyl group, a 2-ethylhexylaminocarbnyl group, a dodecylaminocarbonyl group, a phenylaminocarbonyl group, a naphthylaminocarbonyl group, or a 2-pyridilaminocarbonyl group), a ureido group (such as a methylureido group, an ethylureido group, a pentylureido group, a cyclohexylureido group, an octylureido group, a dodecylureido group, a phenylureido group, a naphthylureido group, or a 2-pyridilaminoureido group), a sulfinyl group (such as a methylsulfinyl group, an ethylsulfinyl group, a butylsulfinyl group, a cyclohexylsulfinyl group, a 2-ethylhexylsulfinyl group, a dodecylsulfinyl group, a phenylsulfinyl group, a naphthylsulfinyl group, or a 2-pyridilsulfinyl group), an alkylsulfonyl group (such as a methylsulfonyl group, an ethylsulfonyl group, a butylsulfonyl group, a cyclohexylsulfonyl group, a 2-ethylhexylsulfonyl group, or a dodecylsulfonyl group), an arylsulfonyl group or a heteroarylsulfonyl group (such as a phenylsulfonyl group, a naphthylsulfonyl group, or a 2-pyridilsulfonyl group), an amino group (such as an amino group, an ethylamino group, a dimethylamino group, a butylamino group, a cyclopentylamino group, a 2-ethylhexylamino group, a dodecylamino group, an anilino group, a naphthylamino group, a 2-pyridilamino group, a piperidyl group (also called a piperidinyl group), or a 2, 2, 6, 6-tetramethylpiperidinyl group), a halogen atom (such as a fluorine atom, a chlorine atom, or a bromine atom), a hydrocarbon fluoride group (such as a fluoromethyl group, a trifluoromethyl group, a pentafluoroethyl group, or a pentafluorophenyl group), a cyano group, a nitro group, a hydroxy group, a mercapto group, a silyl group (such as a trimethylsilyl group, a triisopropylsilyl group, a triphenylsilyl group, or a phenyldiethylsilyl group), a phosphate ester group (such as a dihexylphosphoryl group), a phosphite ester group (such as a diphenylphosphinyl group), and a phosphono group.

Any of these substituents may be partly substituted further with another substituent, or a plurality of substituents may be coupled with each other to form a ring.

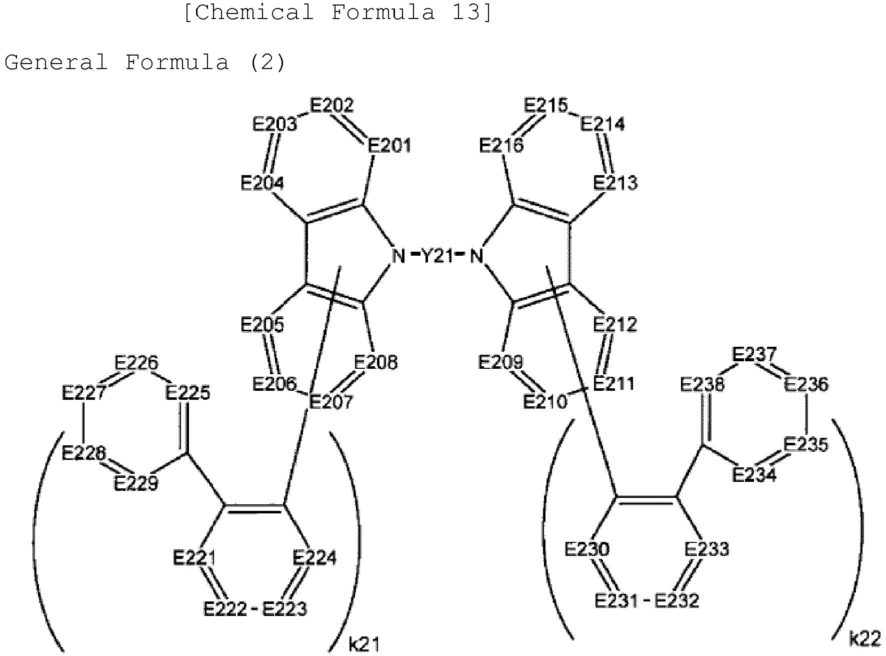

General Formula (2) also corresponds to one mode of General Formula (1). In General Formula (2), Y21 represents an arylene group, a heteroarylene group, or a divalent connecting group including a combination thereof. Each of E201 to E216 and E221 to E238 represents -C(R21)= or -N=, and R21 represents a hydrogen atom or a substituent. Note that at least one of E221 to E229 and at least one of E230 to E238 represent -N=. Moreover, k21 and k22 represent an integer of 0 to 4 and k21 + k22 is an integer of 2 or more.

In General Formula (2), the arylene group represented by Y21 is, for example, an o-phenylene group, a p-phenylene group, a naphthalene diyl group, an anthracene diyl group, a naphthacene diyl group, a pyrene diyl group, a naphthyl naphthalene diyl group, a biphenyl diyl group (such as [1,1'-biphenyl]-4,4'-diyl group, a 3-3'-biphenyl diyl group, or a 3,6-biphenyl diyl group), a tertphenyl diyl group, a quaterphenyl diyl group, a quinquephenyl diyl group, a sexiphenyl diyl group, a septiphenyl diyl group, an octyphenyl diyl group, a nobiphenyl diyl group, a deciphenyl diyl group, or the like.

In General Formula (2), examples of the heteroarylene group represented by Y21 include a divalent group derived from the group consisting of a carbazole group, a carboline group, a diazacarbazole group (also called a monoazacarboline group, representing a ring structure in which one of carbon atoms constituting the carboline group is replaced by a nitrogen atom), a triazole ring, a pyrrole ring, a pyridine ring, a pyradine group, a quinoxaline ring, a thiophene group, an oxadiazole ring, a dibenzofuran ring, a dibenzothiophene ring, and an indole ring.

As a preferred embodiment of the arylene group, the heteroarylene group, or the divalent connecting group including a combination thereof represented by Y21, the heteroarylene group derived from the condensed heteroaromatic ring formed by condensing three or more rings is preferable; moreover, as the group derived from the condensed heteroaromatic ring formed by condensing three or more rings, the group derived from the dibenzofuran ring or the group derived from the dibenzothiophene ring is preferable.

In General Formula (2), when R21 of -C(R21)= represented by each of E201 to E216 and E221 to E238 is a substituent, the substituent shown as the example of R11 and R12 in General Formula (1) is similarly applicable.

In General Formula (2), it is preferable that six or more of E201 to E208 and six or more of E209 to E216 each represent -C(R21)=.

In General Formula (2), at least one of E225 to E229 and at least one of E234 to E238 preferably represent -N=.

Moreover, in General Formula (2), at least one of E225 to E229 and at least one of E234 to E238 preferably represent -N=.

Moreover, in General Formula (2), at least one of E221 to E224 and at least one of E230 to E233 preferably represent -C(R21)=.

Moreover, in the compound represented by General Formula (2), E203 preferably represents -C(R21)=, R21 preferably represents a connecting portion, E211 preferably represents -C(R21)=, and R21 preferably represents a connecting portion.

Moreover, E225 and E234 preferably represent -N= and each of E221 to E224 and E230 to E233 preferably represents -C(R21)=.

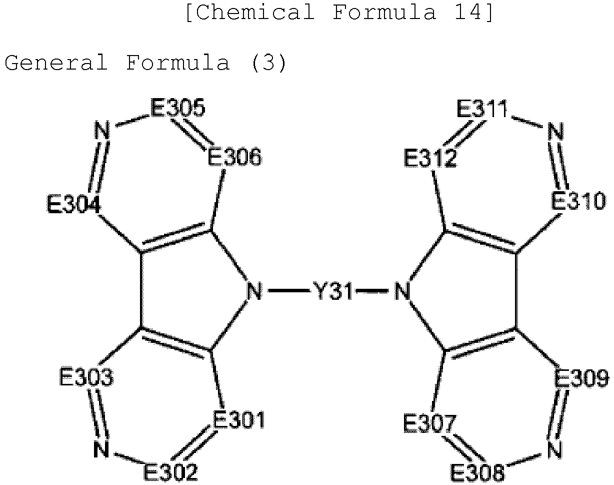

General Formula (3) also corresponds to one mode of General Formula (1). In General Formula (3), each of E301 to E312 represents -C(R31)=, and R31 represents a hydrogen atom or a substituent. Moreover, Y31 represents an arylene group, a heteroarylene group, or a divalent connecting group including a combination thereof.

In General Formula (3), when R31 of -C(R31)= represented by each of E301 to E312 is a substituent, the substituent shown as the example of R11 and R12 in General Formula (1) is similarly applicable.

In General Formula (3), a preferred embodiment of the arylene group, the heteroarylene group, or the divalent connecting group including the combination thereof, which is represented by Y31, may be similar to Y21 of General Formula (2) .

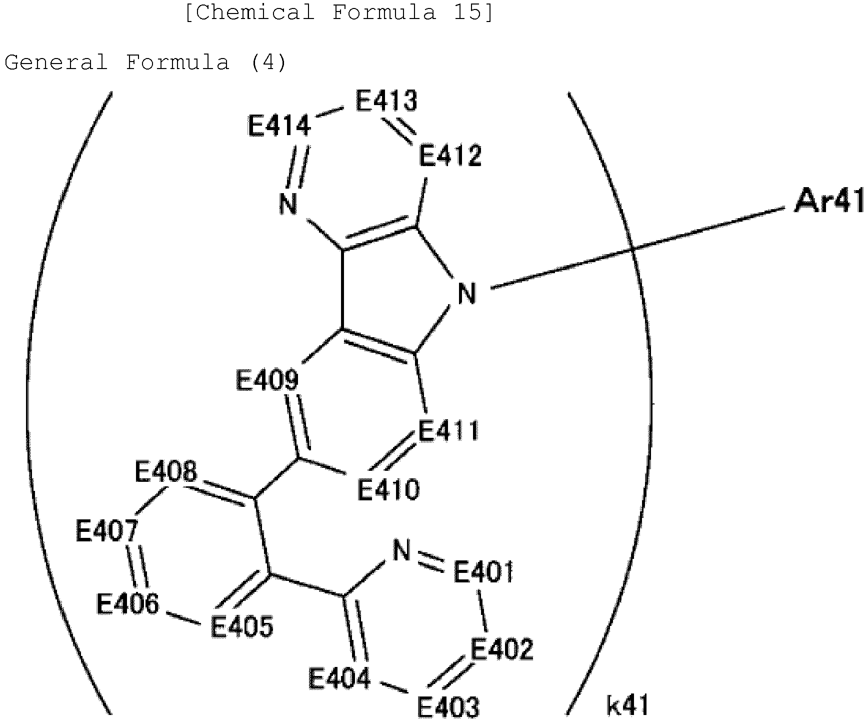

General Formula (4) also corresponds to one mode of General Formula (1). In General Formula (4), each of E401 to E414 represents -C(R41)= and R41 represents a hydrogen atom or a substituent. Ar41 represents a substituted or unsubstituted aromatic hydrocarbon ring or heteroaromatic ring. Moreover, k41 represents an integer of 3 or more.

If R41 of -C(R41)= represented by each of E401 to E414 in General Formula (4) is a substituent, the substituent shown as the example of R11 and R12 in General Formula (1) is similarly applicable.

If Ar41 represents an aromatic hydrocarbon ring in General Formula (4), this aromatic hydrocarbon ring may be a benzene ring, a biphenyl ring, a naphthalene ring, an azulene ring, an anthracene ring, a phenanthrene ring, a pyrene ring, a chrysene ring, a naphthacene ring, a triphenylene ring, an o-terphenyl ring, an m-terphenyl ring, a p-terphenyl ring, an acenaphthene ring, a coronene ring, a fluorene ring, a fluoranthrene ring, a pentacene ring, a perylene ring, a pentaphene ring, a picene ring, a pyrene ring, a pyranthrene ring, an anthra anthrene ring, or the like. Each of these rings may have the substituent shown as the example of R11 and R12 of General Formula (1).

If Ar41 represents an heteroaromatic ring in General Formula (4), this heteroaromatic ring may be a furan ring, a thiophene ring, an oxazole ring, a pyrrole ring, a pyridine ring, a pyridazine ring, a pyrimidine ring, a pyridine ring, a triazine ring, a benzoimidazole ring, an oxadiazole ring, a triazole ring, an imidazole ring, a pyrazole ring, a thiazole ring, an indole ring, a benzothiazole ring, a benzoxazole ring, a quinoxaline ring, a quinazoline ring, a phthalazine ring, a carbazole ring, an azacarbazole ring, or the like. Note that the azacarbazole ring refers to the ring in which one or more carbon atoms of the benzene ring of the carbazole ring are substituted by a nitrogen atom. Each of these rings may have the substituent shown as the example of R11 and R12 of General Formula (1).

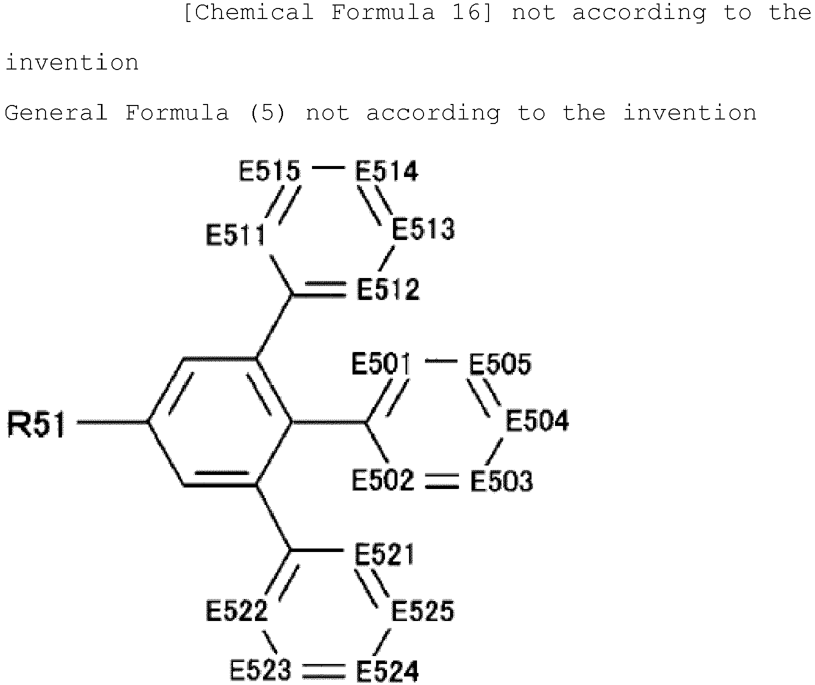

In General Formula (5), R51 represents a substituent. Each of E501, E502, E511 to E515, and E521 to E525 represents -C(R52)= or -N=. Each of E503 to E505 represents -C(R52)=. R52 represents a hydrogen atom (H) or a substituent. At least one of E501 and E502 represents a nitrogen atom (-N=), at least one of E511 to E515 represents a nitrogen atom (-N=), and at least one of E521 to E525 represents a nitrogen atom (-N=) .

If each of R51 and R52 in General Formula (5) represents a substituent, the substituent shown as the example of R11 and R12 of General Formula (1) is similarly applicable.

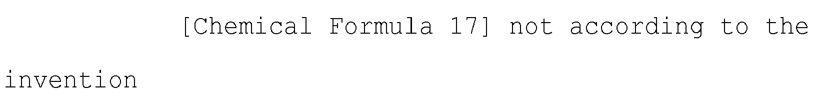

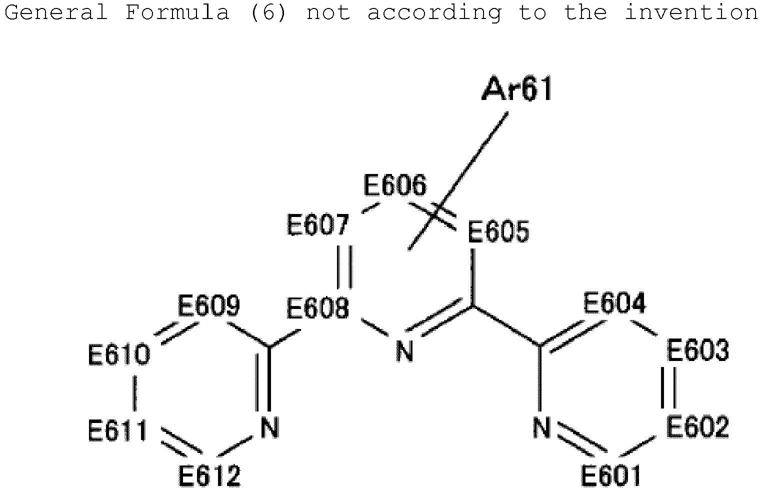

Each of E601 to E612 in General Formula (6) represents -C(R61)= or -N=, and R61 represents a hydrogen atom or a substituent. Moreover, Ar61 represents a substituted or unsubstituted aromatic hydrocarbon ring or heteroaromatic ring.

If R61 of -C(R61)= represented by each of E601 to E612 in General Formula (6) is a substituent, the substituent shown as the example of R11 and R12 of General Formula (1) is similarly applicable.

The substituted or unsubstituted aromatic hydrocarbon ring or heteroaromatic ring represented by Ar61 of General Formula (6) may be similar to that of Ar41 of General Formula (4).

### [Compound-3]

As another example of the compound included in the nitrogen-containing layer 3, Compounds 1 to 118, whose specific examples are given below, are given in addition to the compounds represented by General Formulae (1) to (6) above. These compounds are preferable because of being stable. Compounds 1 to 118 include the compound whose effective unshared electron pair content ratio [n/M] is in the above range described above, and such compounds are preferably used alone as the compound to be included in the nitrogen-containing layer 3. Moreover, Compounds 1 to 118 include the compound that satisfies any of General Formulae (1) to (6). Of the following compounds in chemical formulae 18 to 45 the following are not according to the invention: 1, 2, 3, 4, 5, 6, 7, 11, 12, 13, 15, 16, 18, 20, 30, 31, 32, 33, 34, 35, 36, 37, 38, 39, 40, 41, 42, 44, 45, 46, 47, 48, 49, 50, 51, 68, 69, 70, 71, 72, 73, 74, 76, 77, 78, 79, 80, 81, 83, 85, 86, 88, 89, 93, 97, 98, 99, 102, 103, 104, 108, 109, 110, 111, 113, 114, 115, 116, 117.

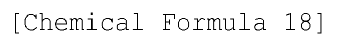

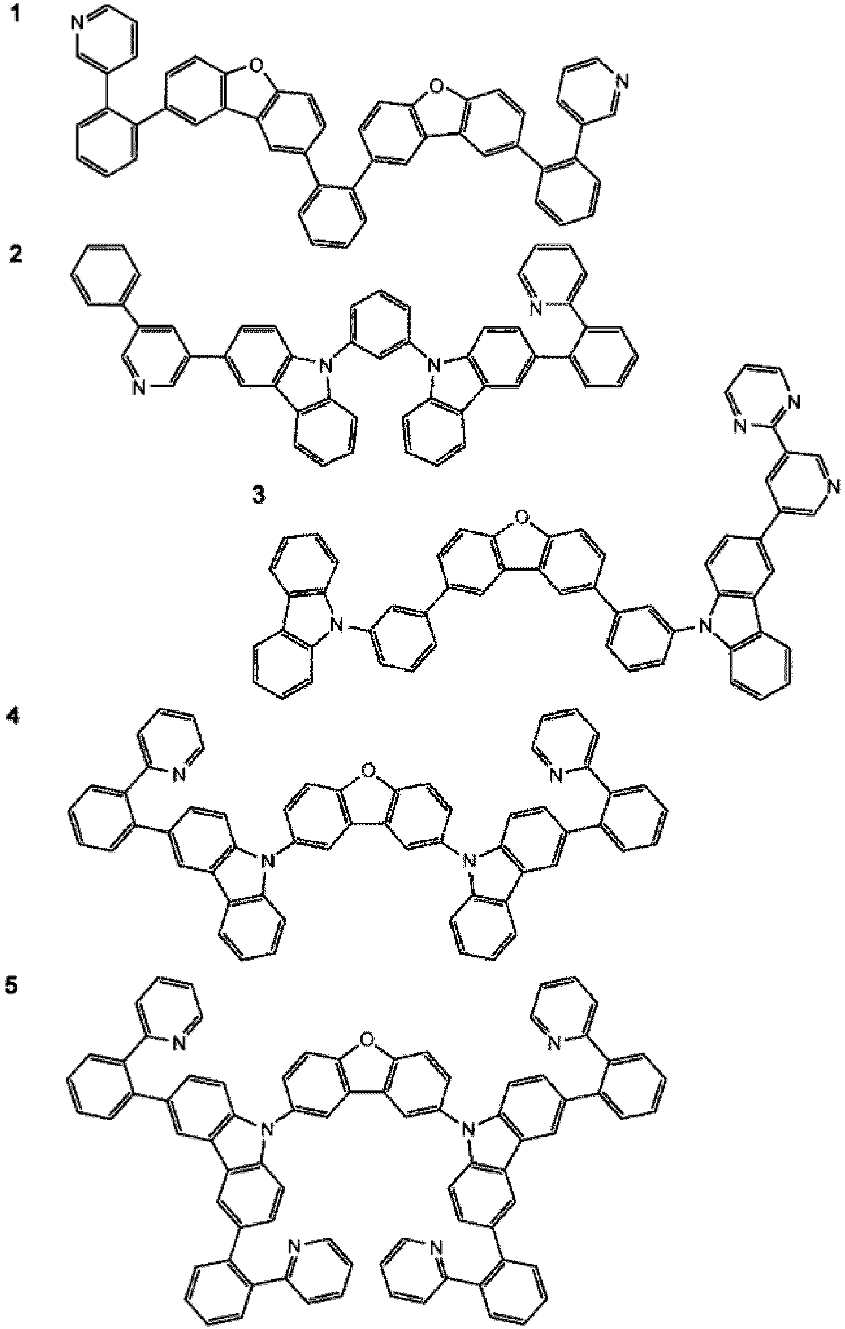

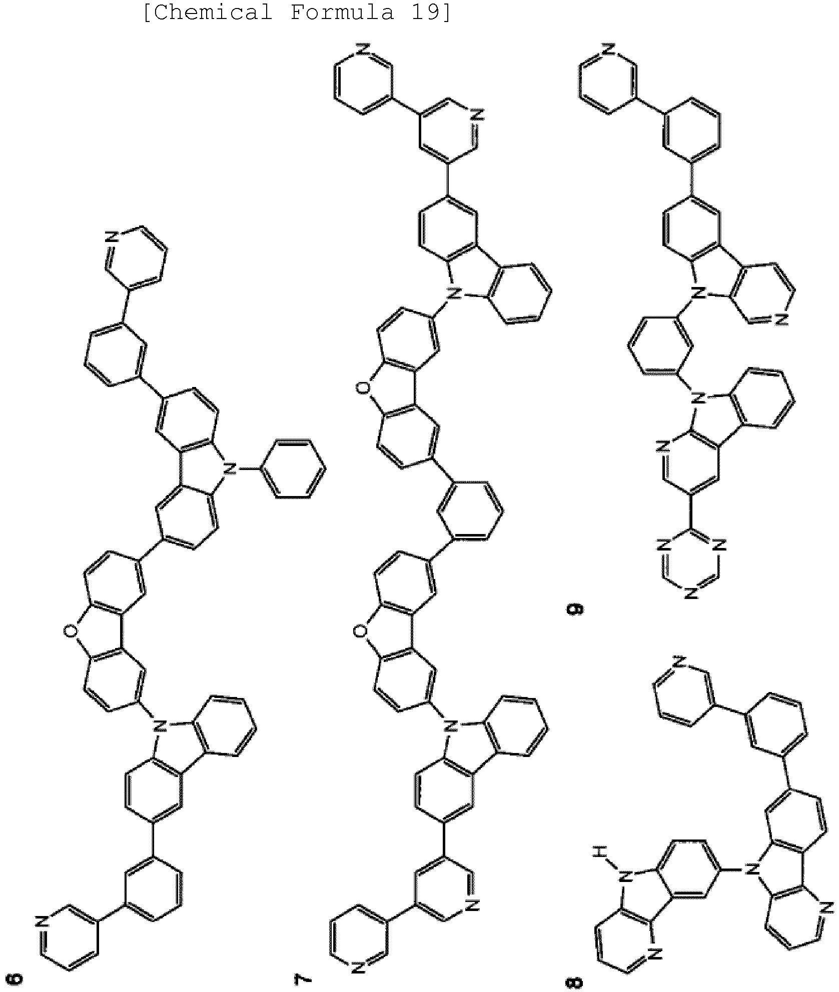

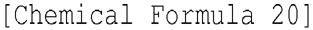

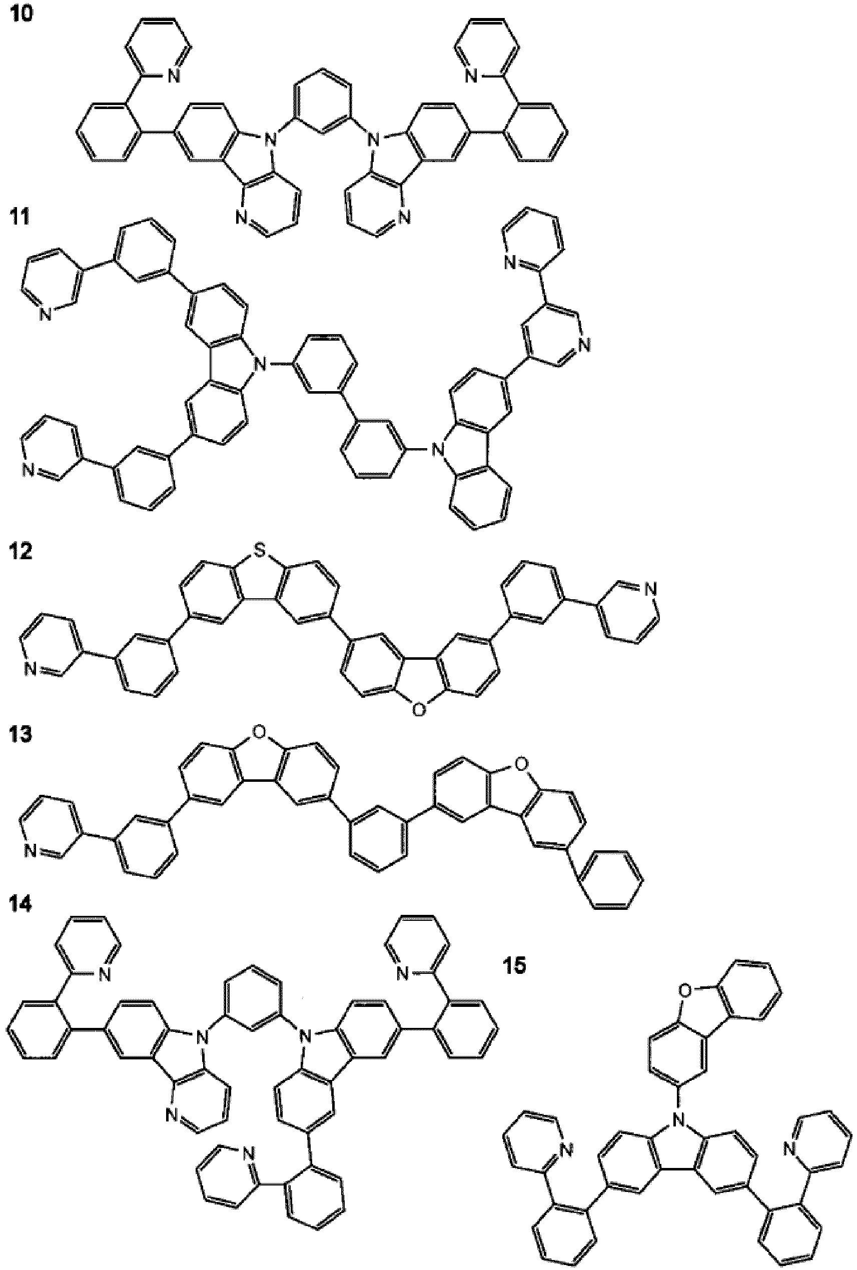

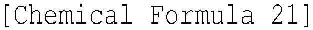

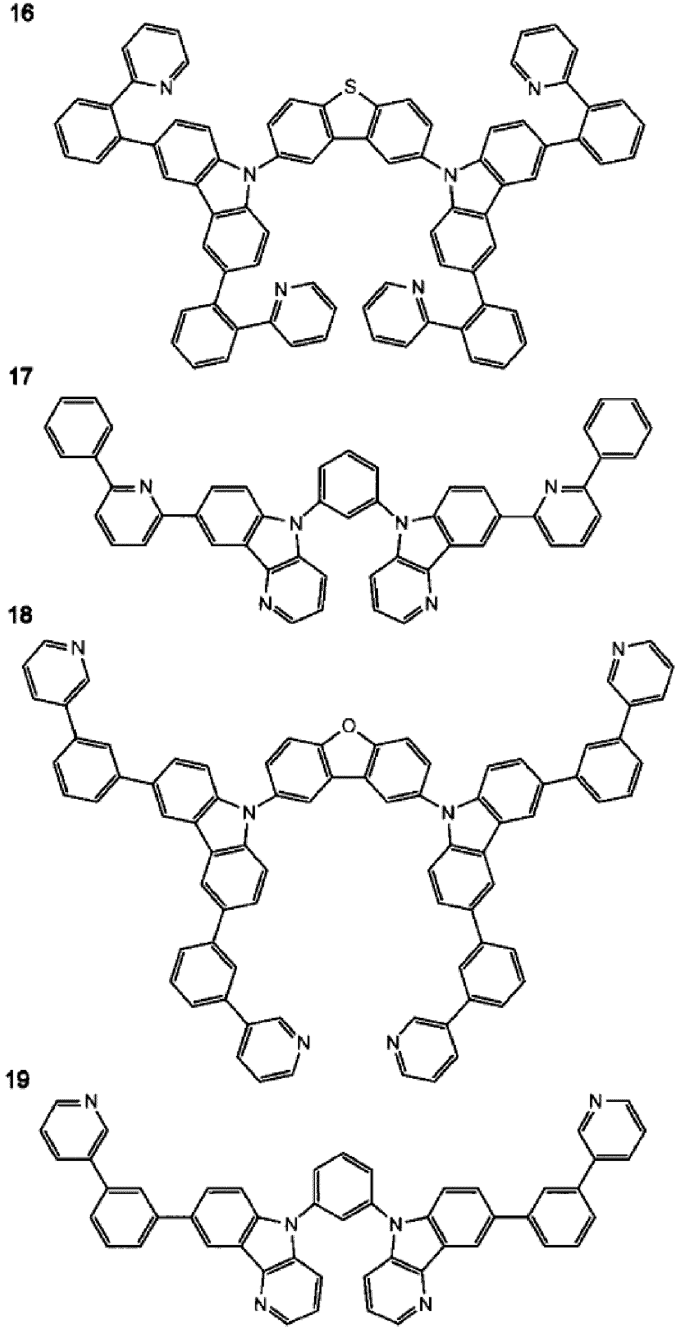

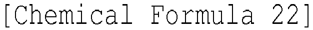

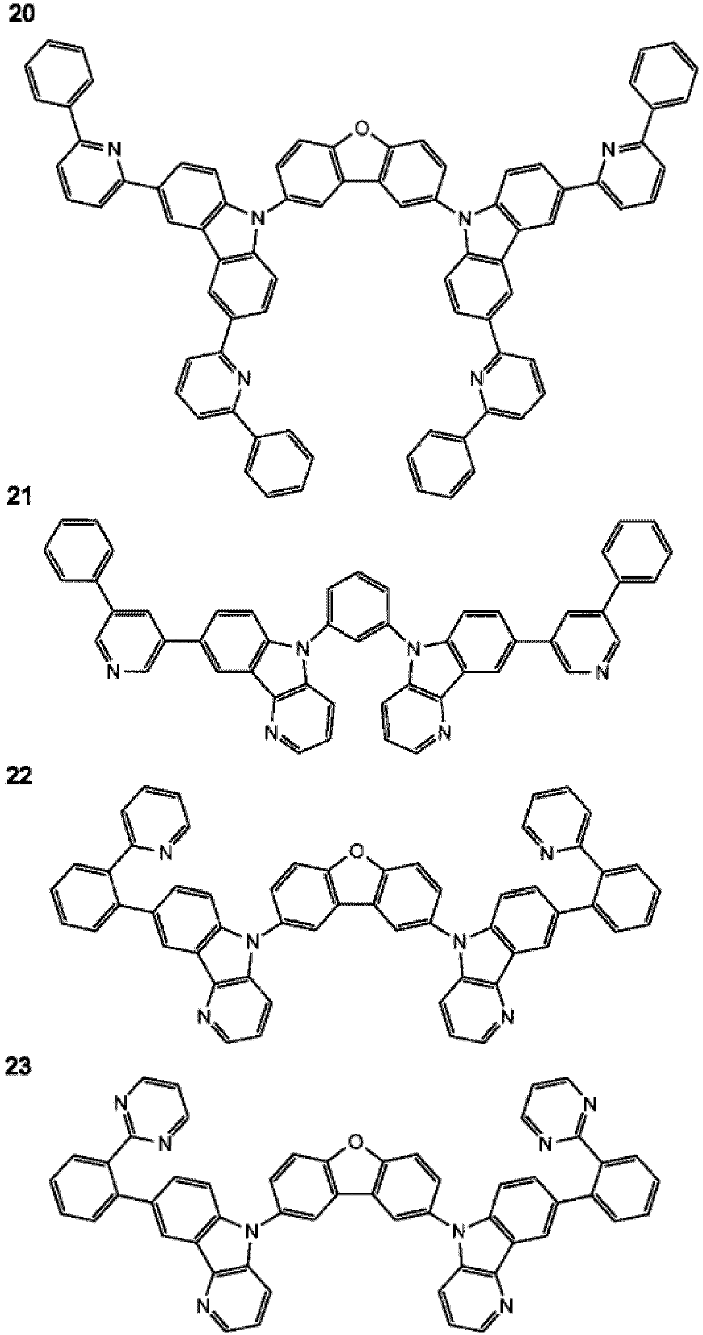

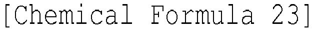

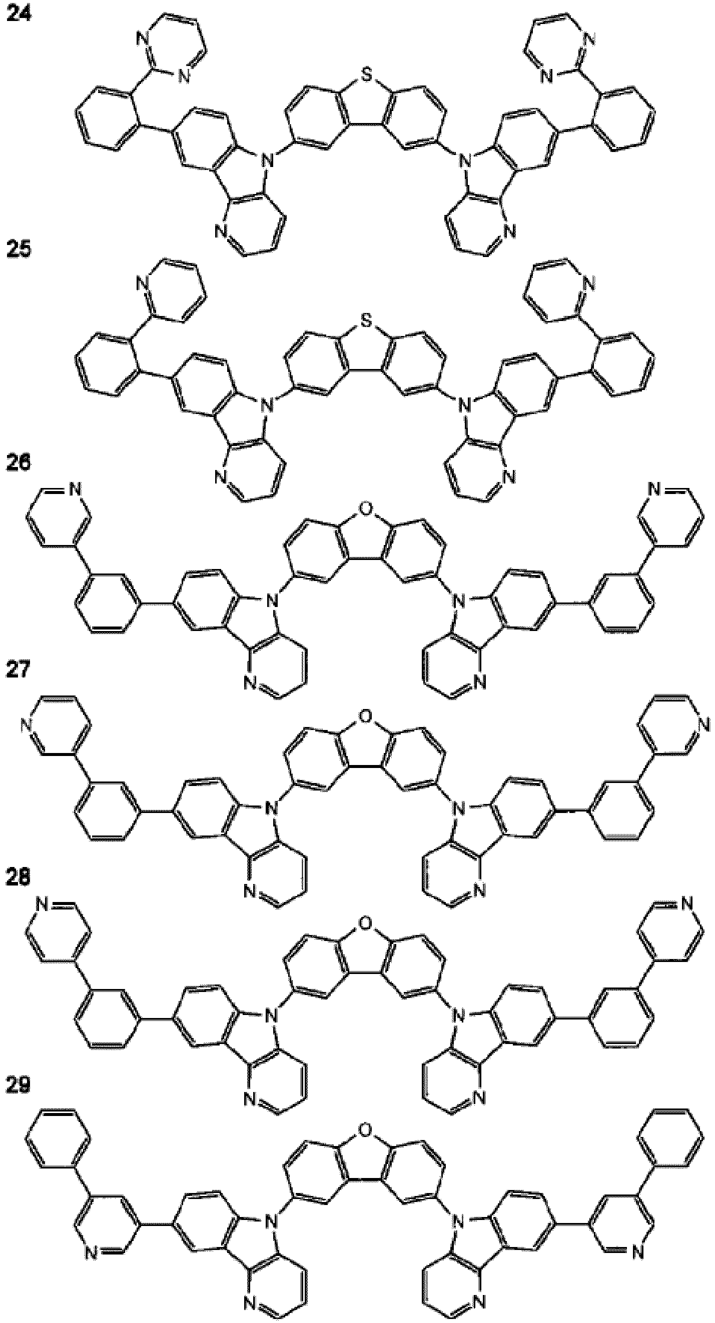

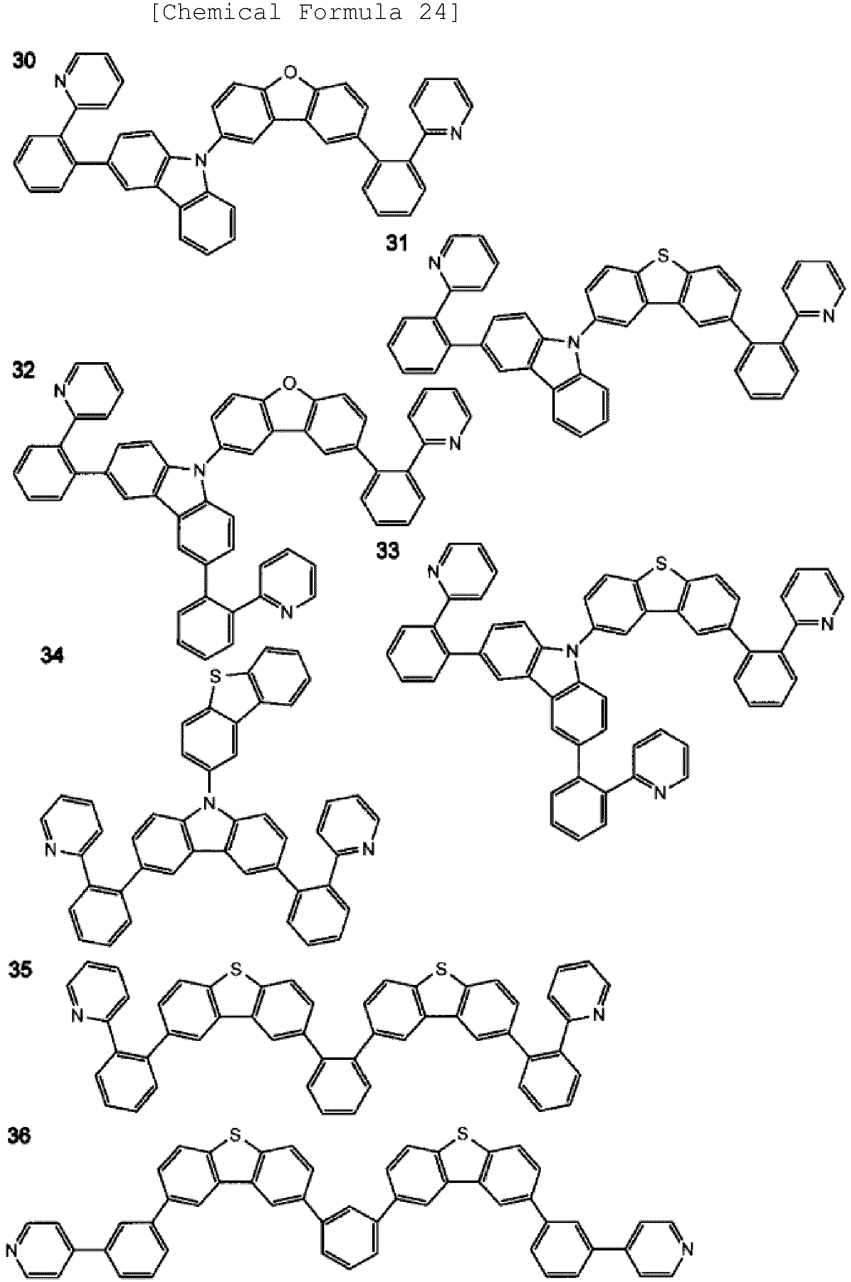

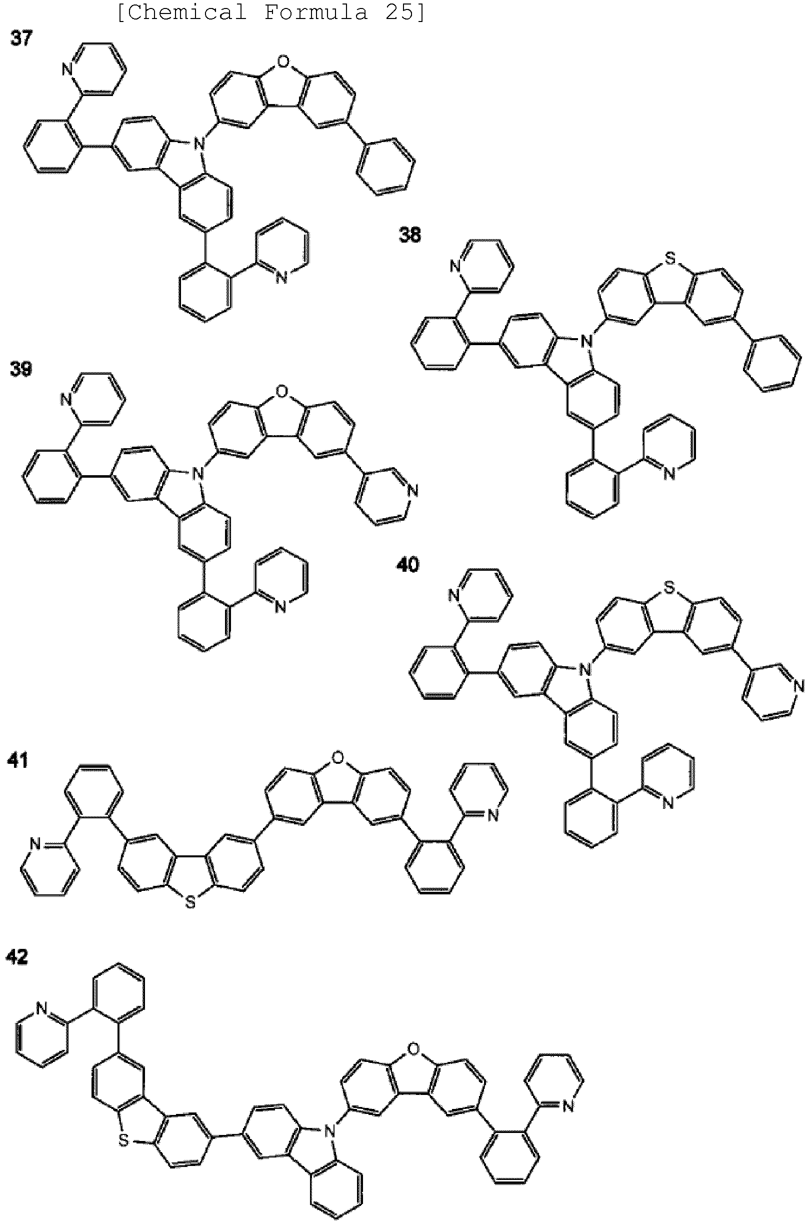

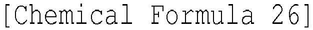

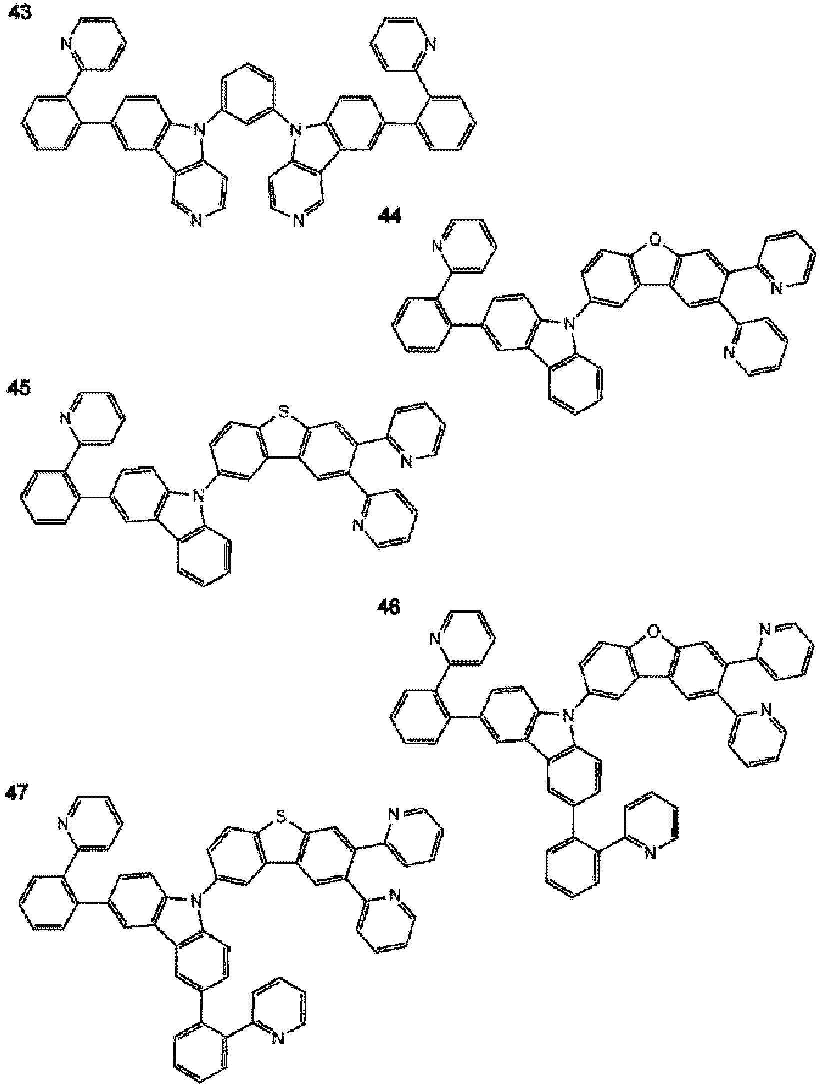

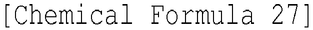

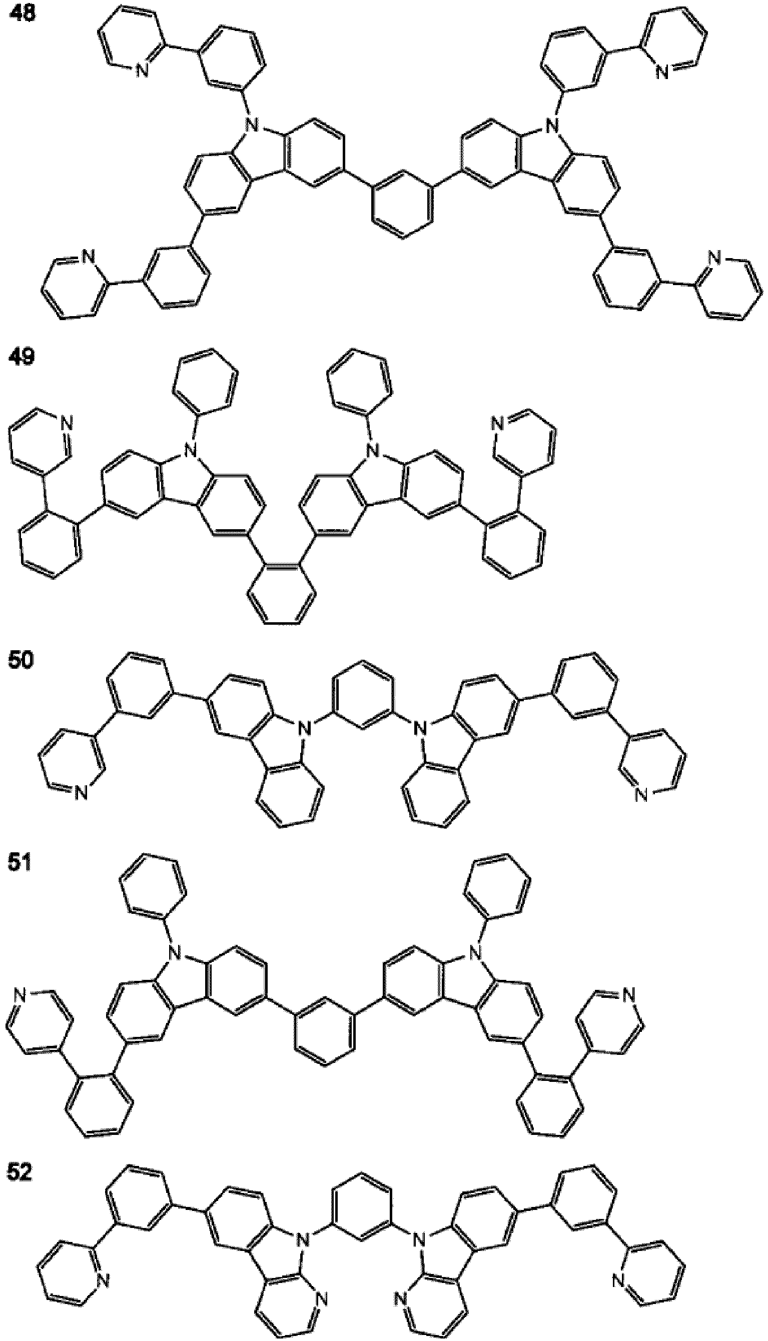

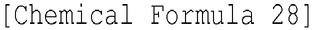

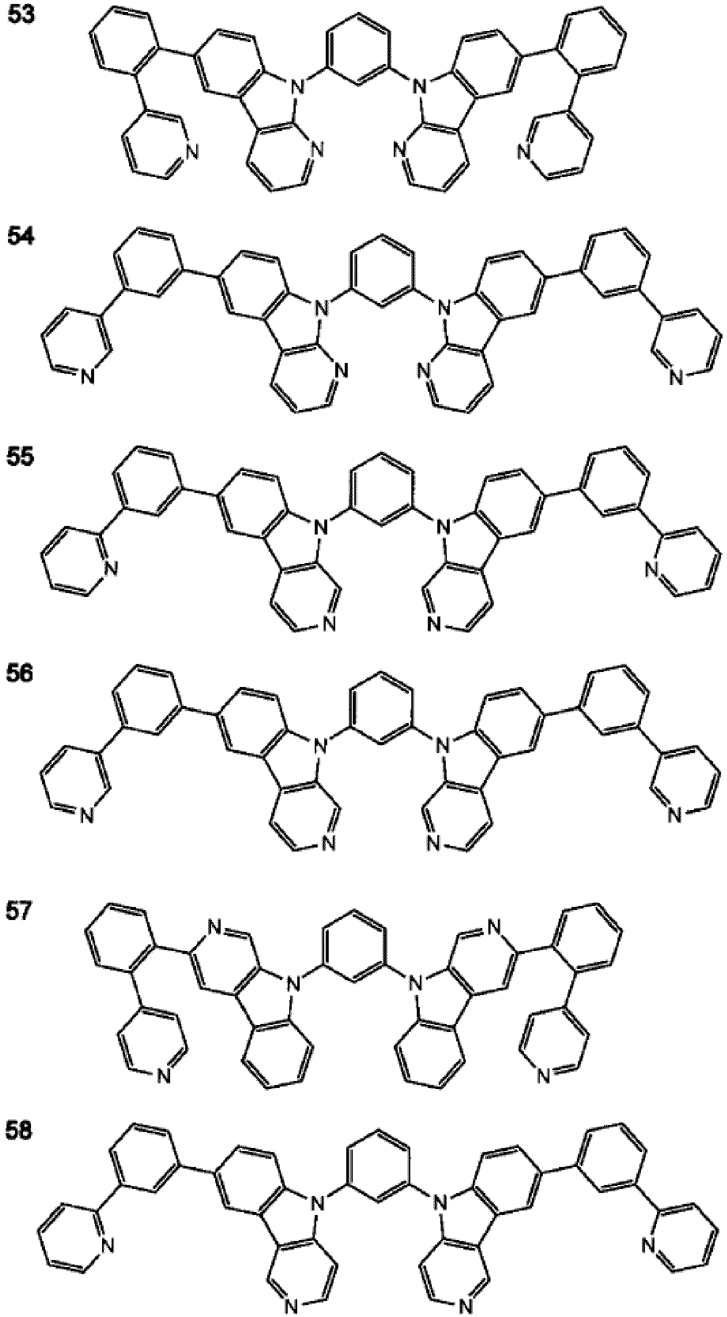

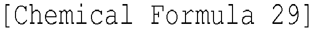

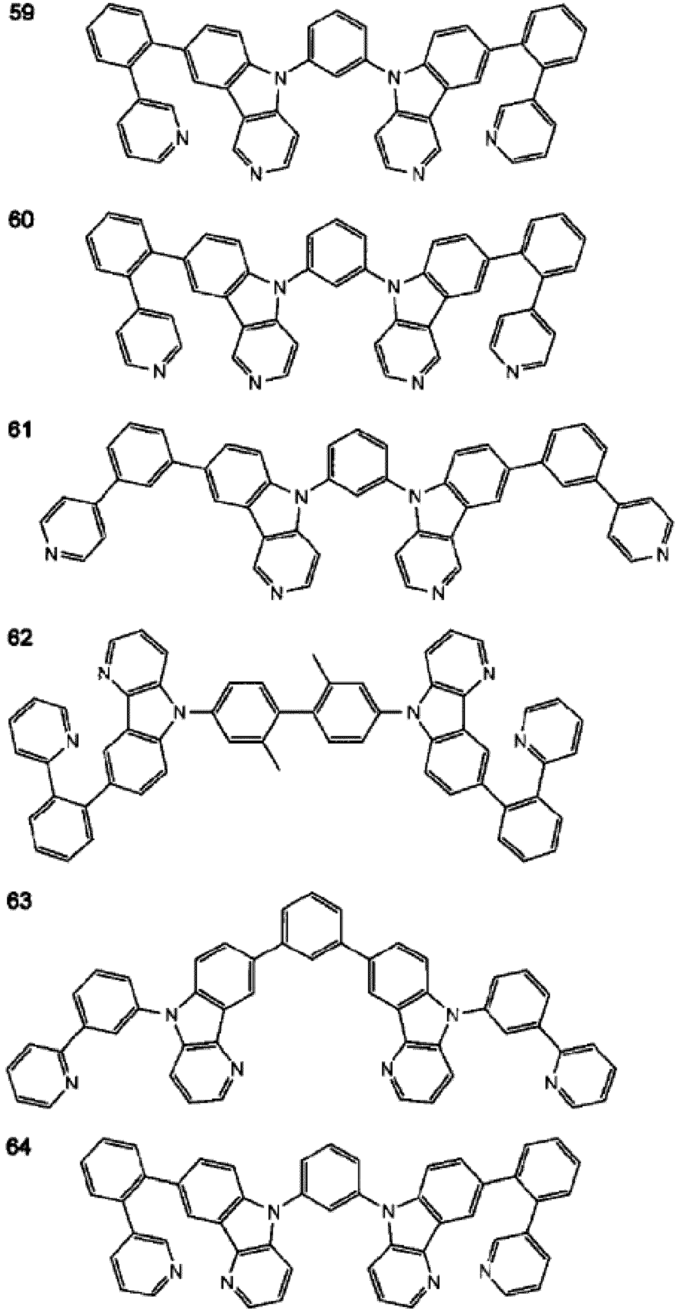

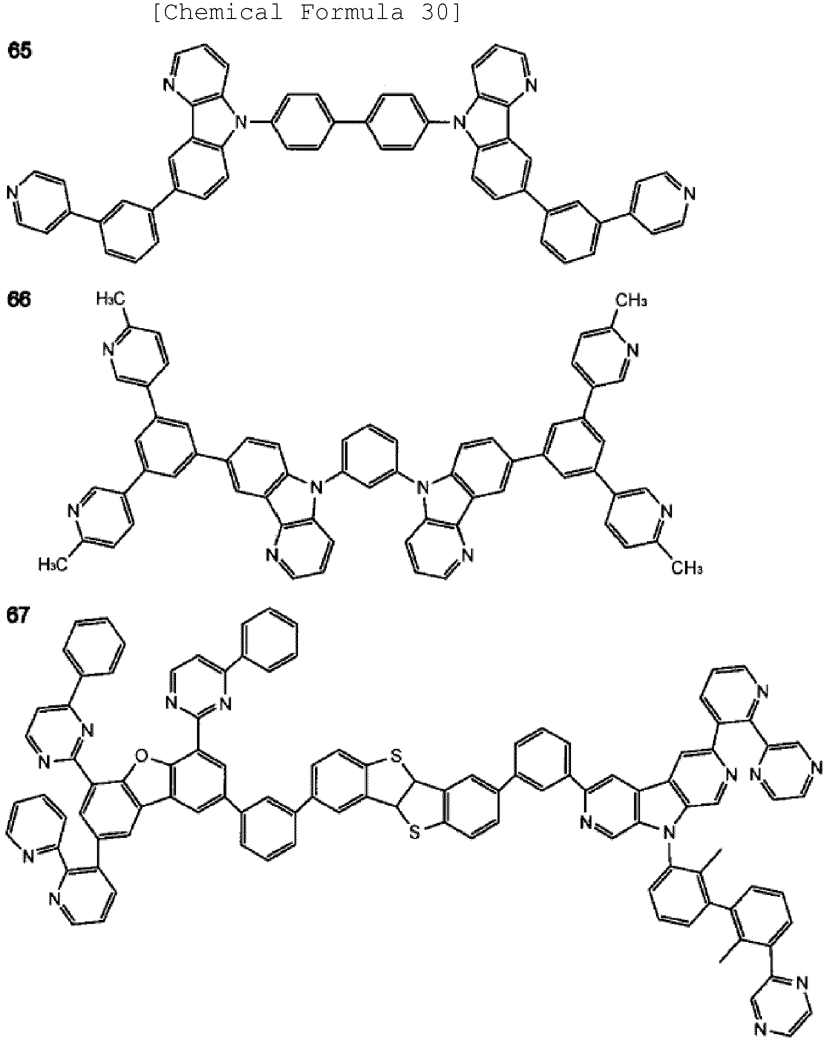

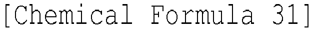

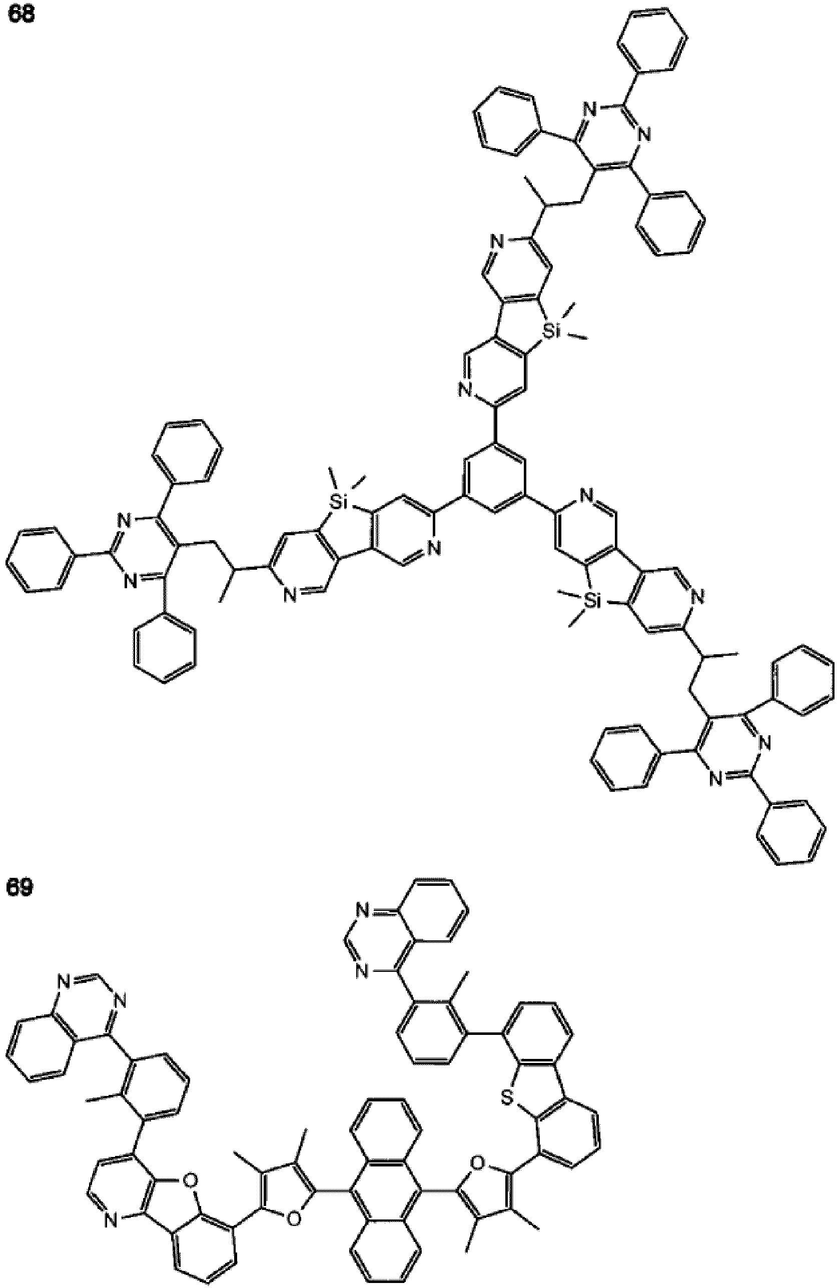

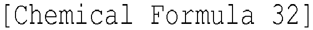

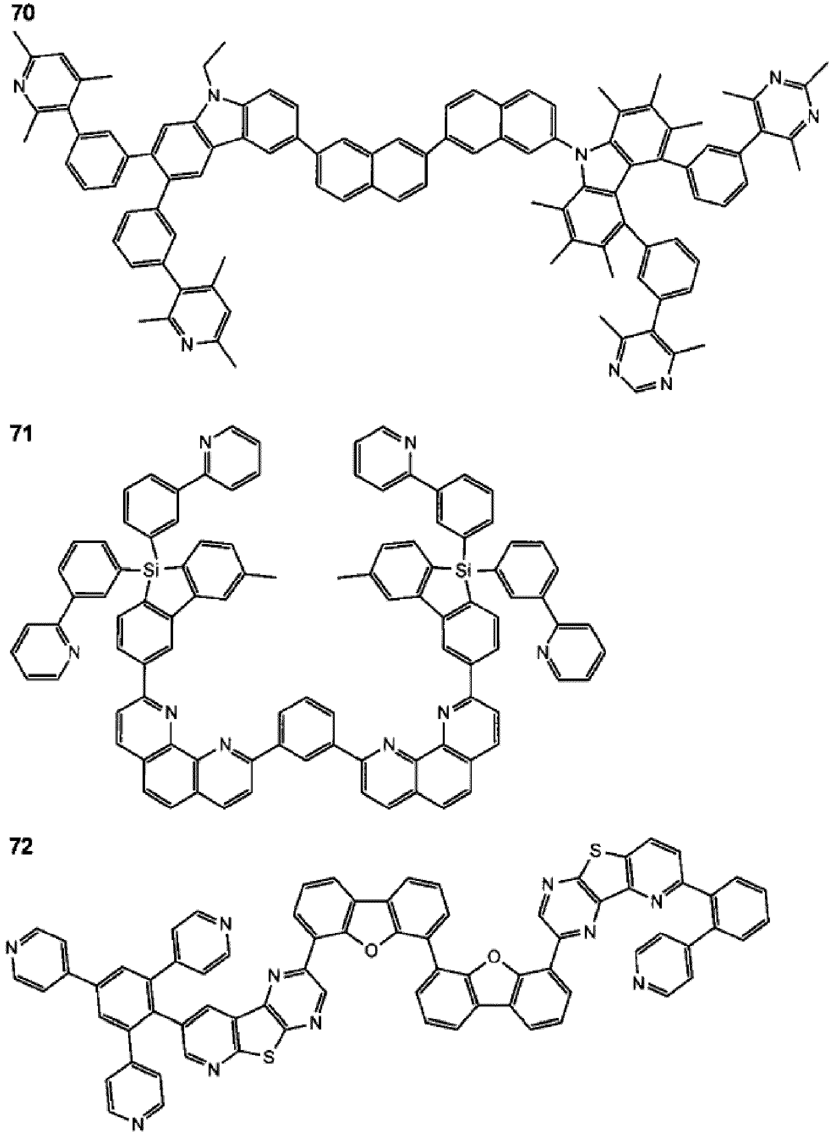

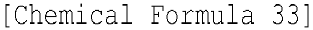

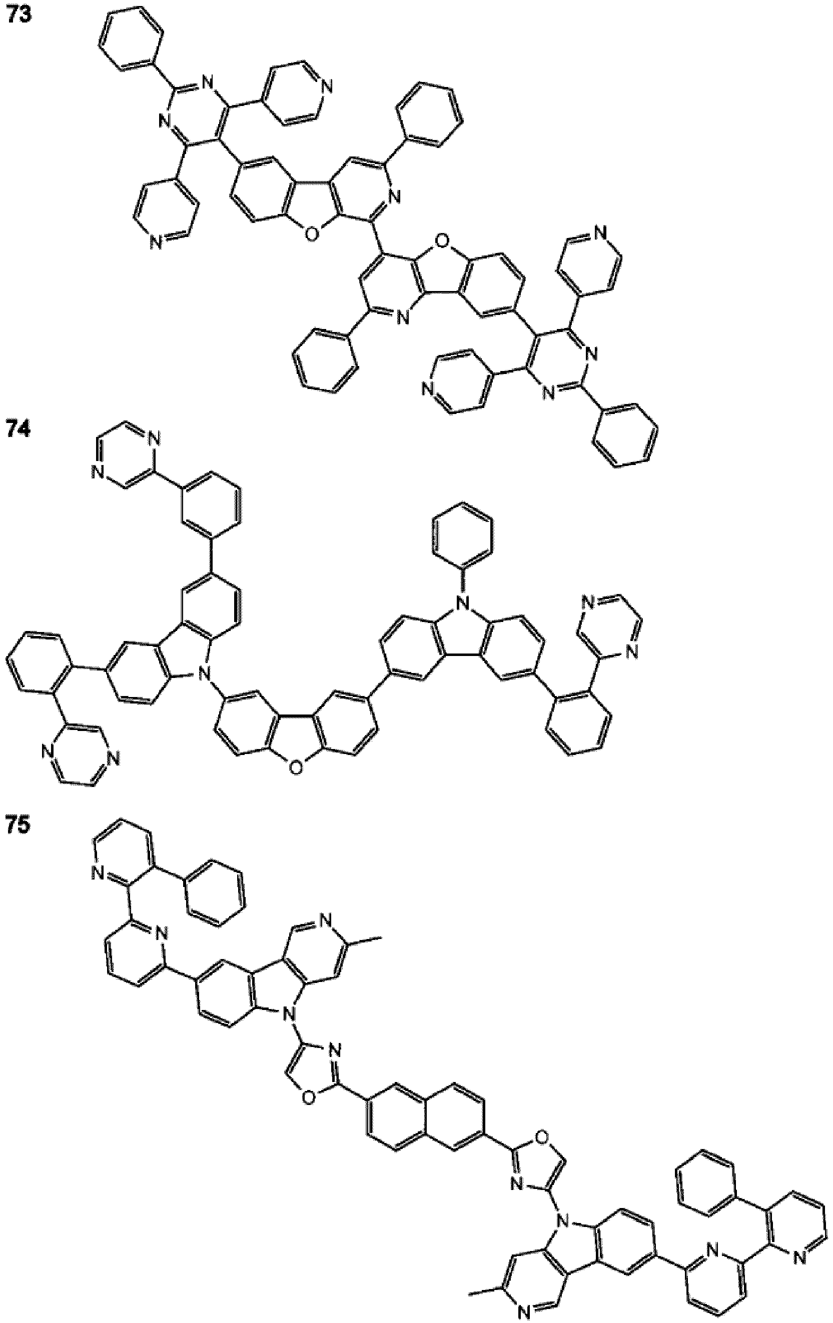

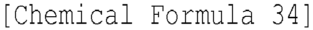

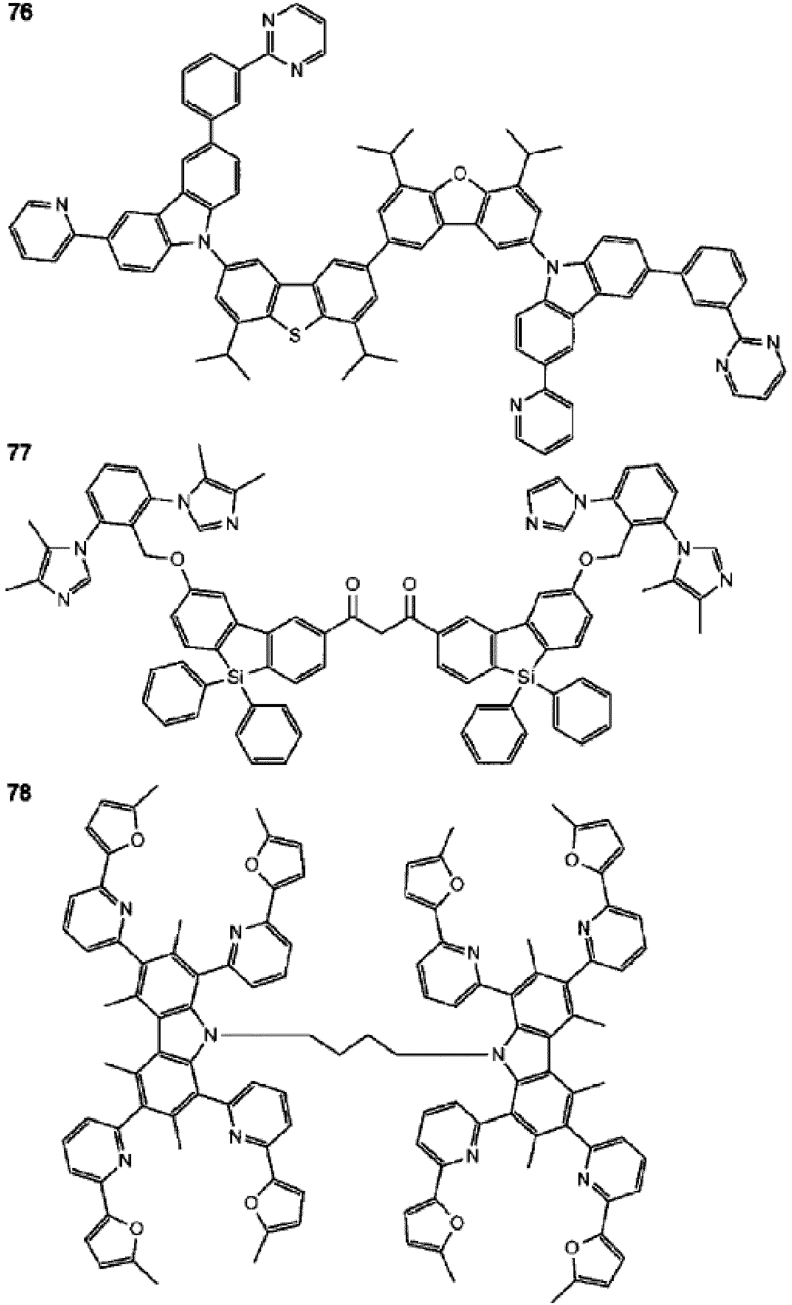

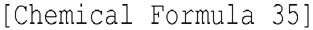

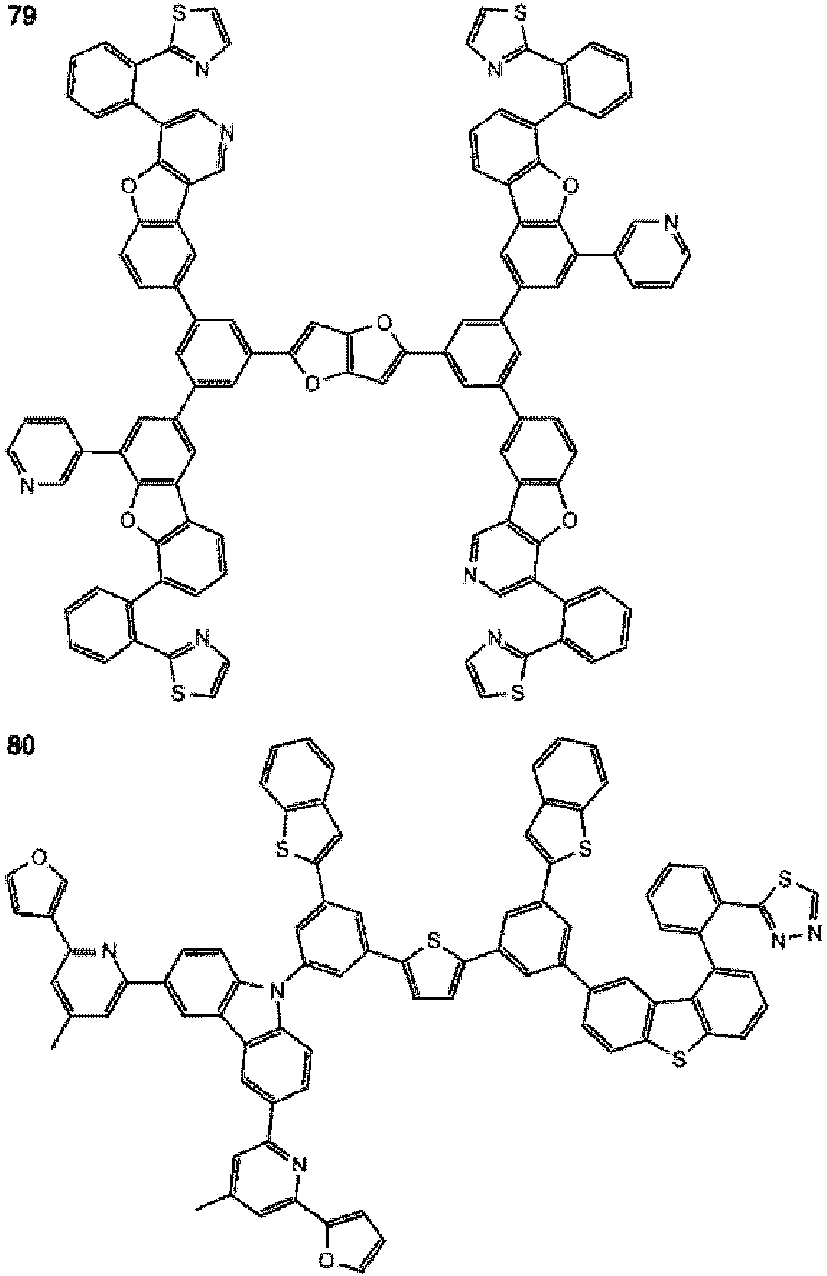

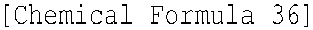

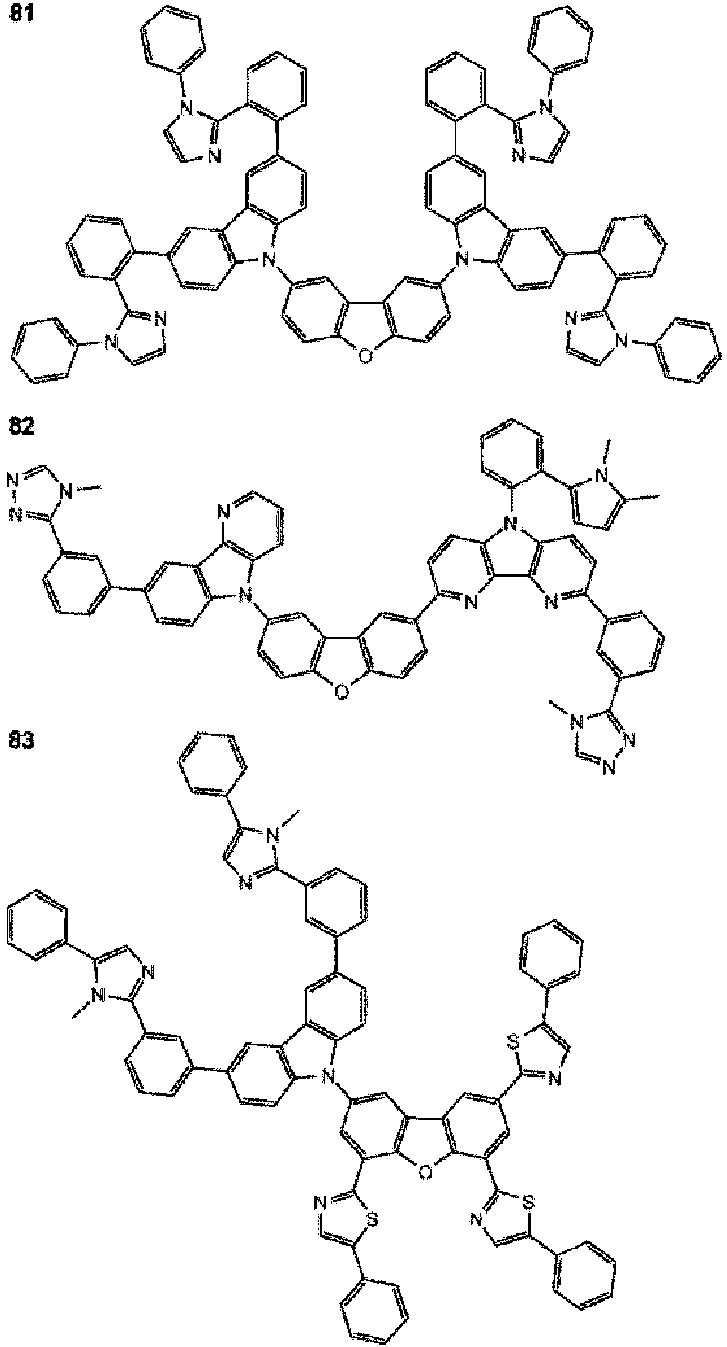

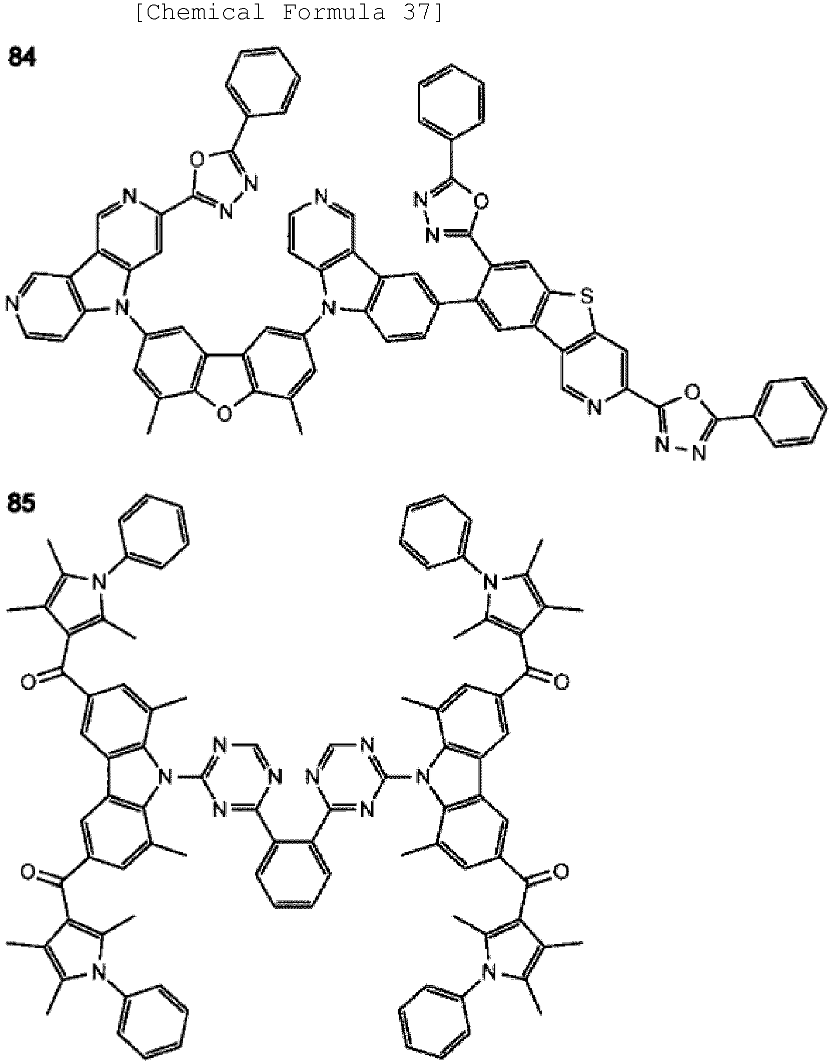

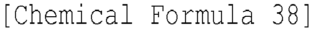

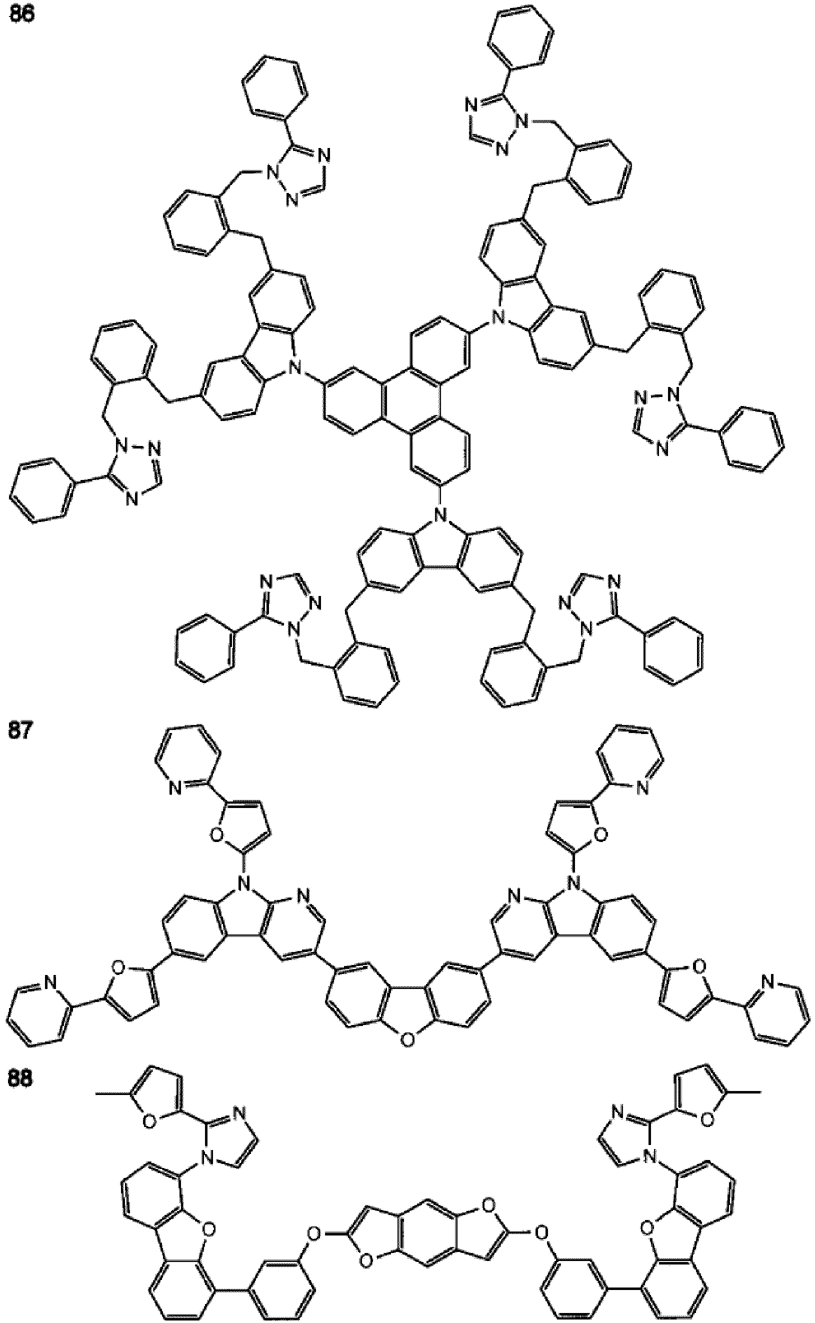

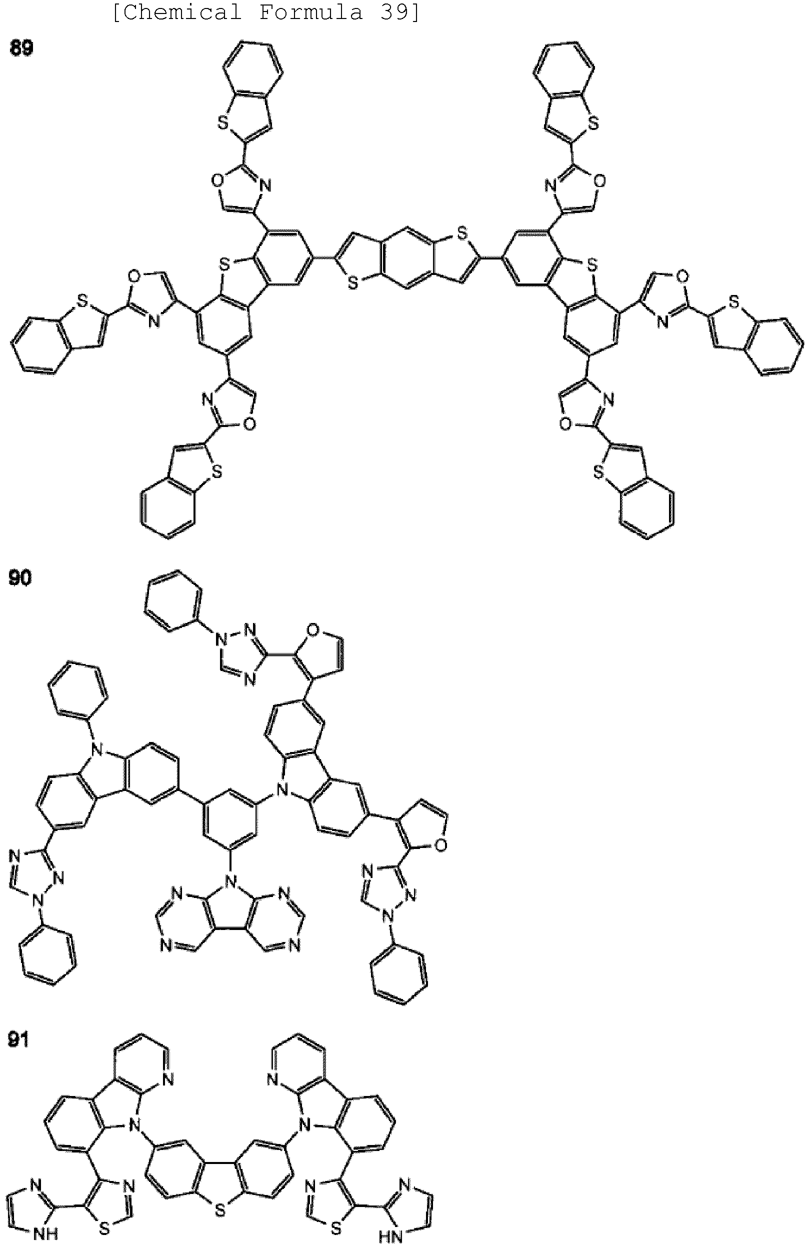

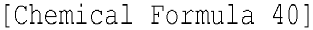

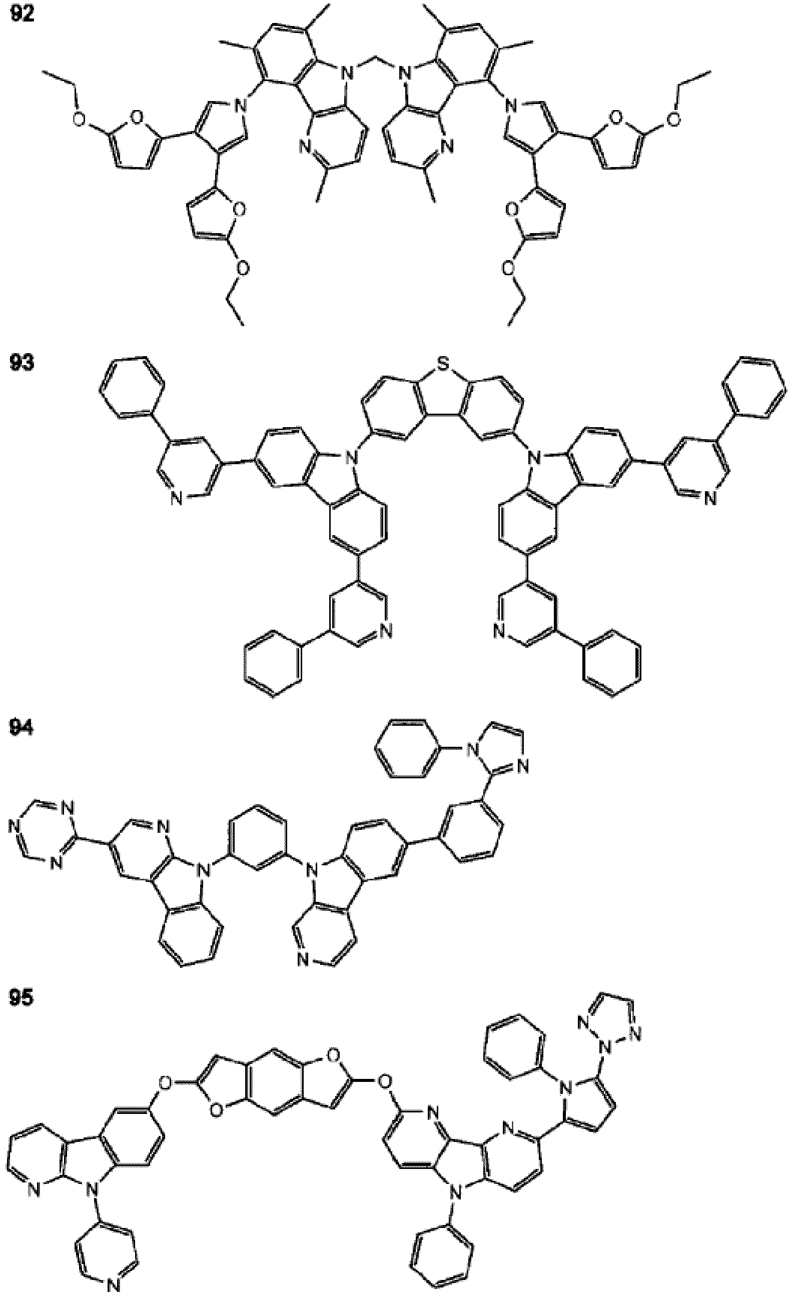

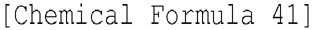

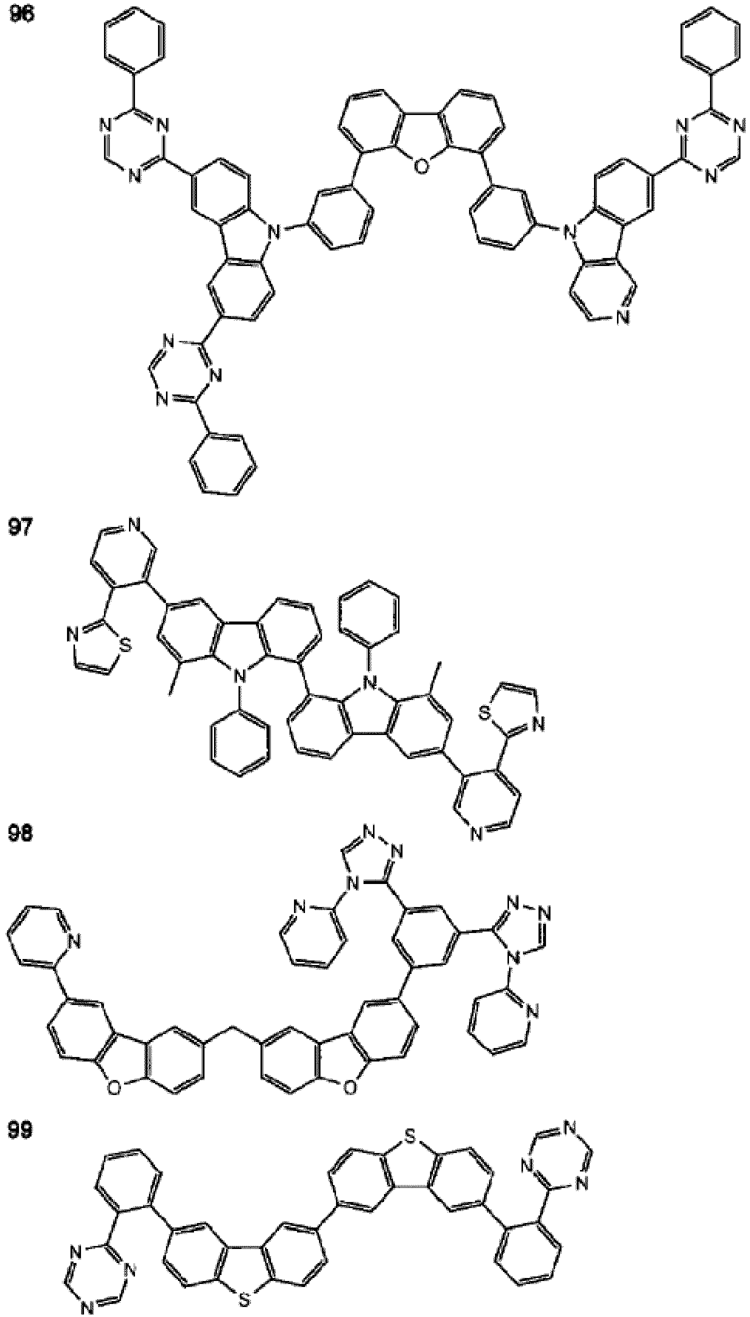

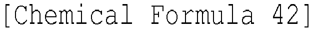

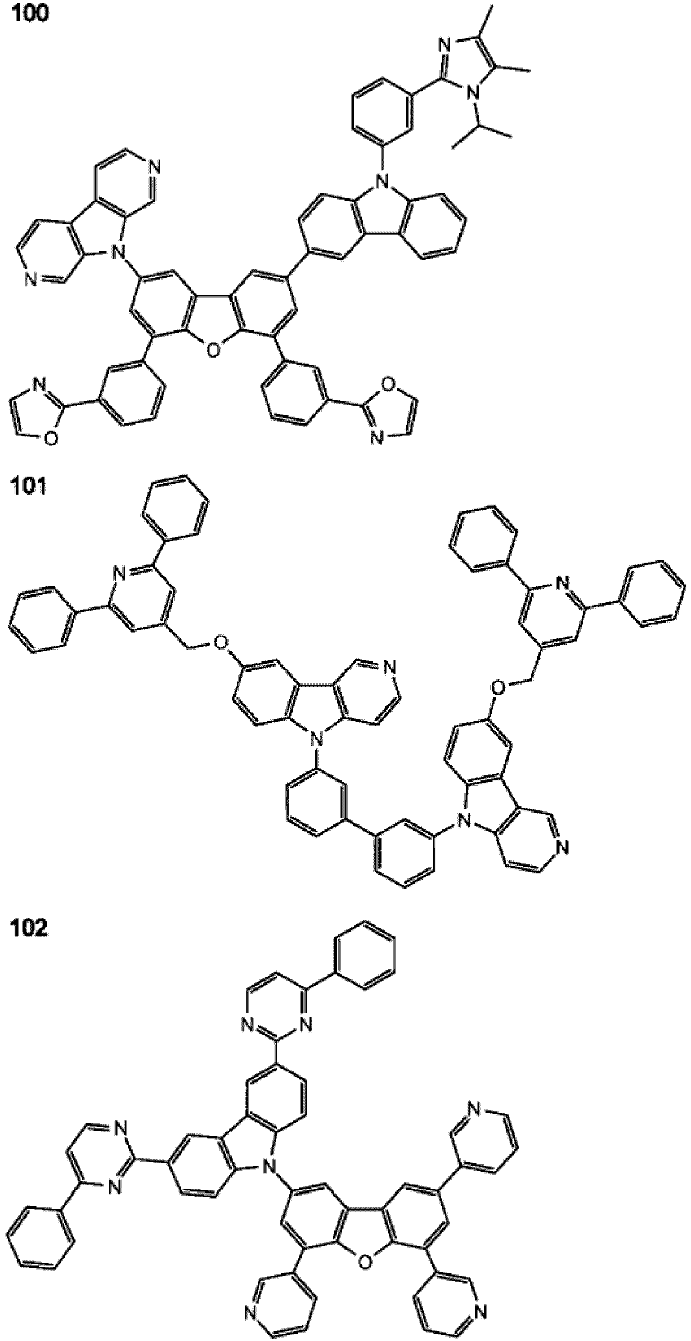

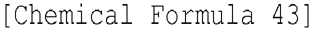

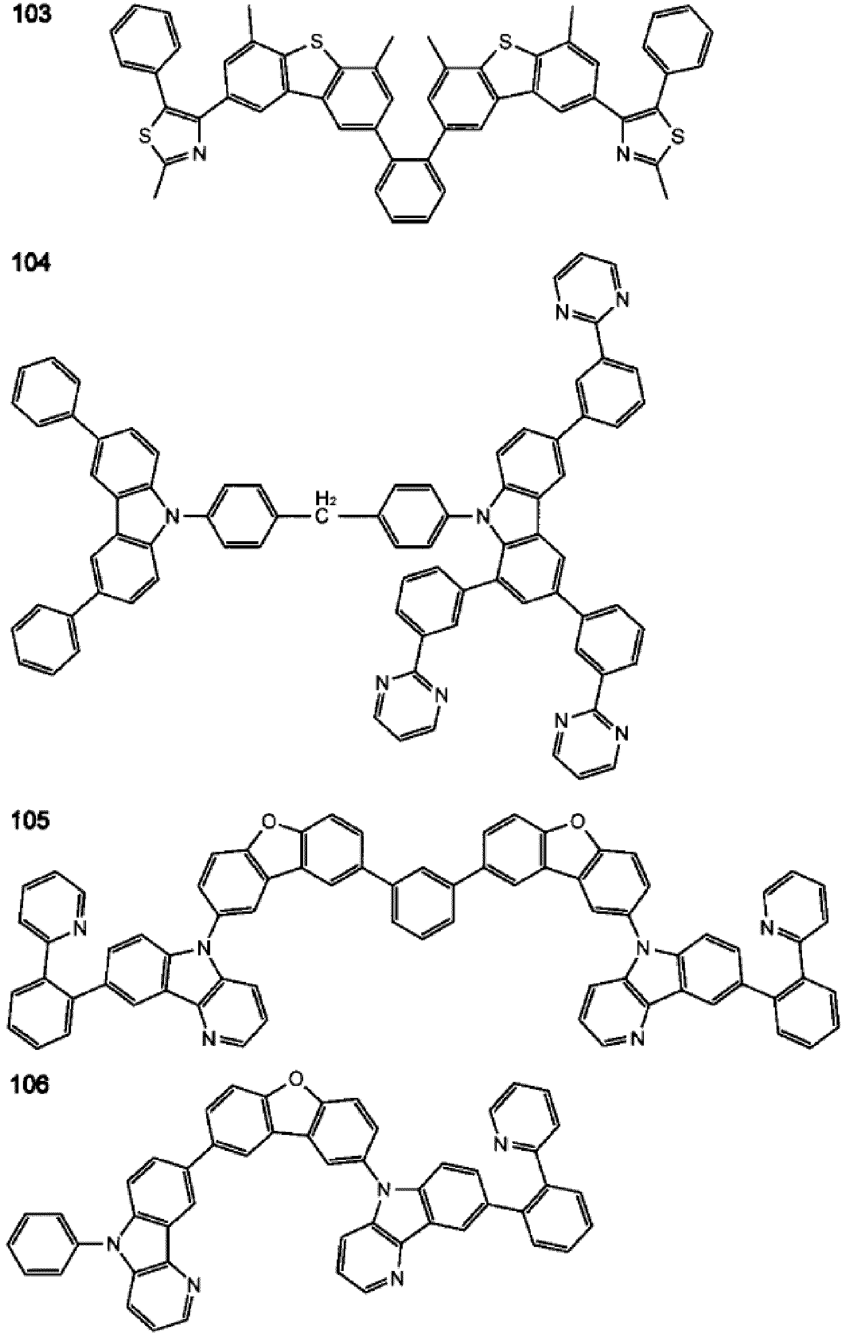

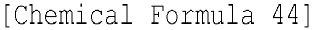

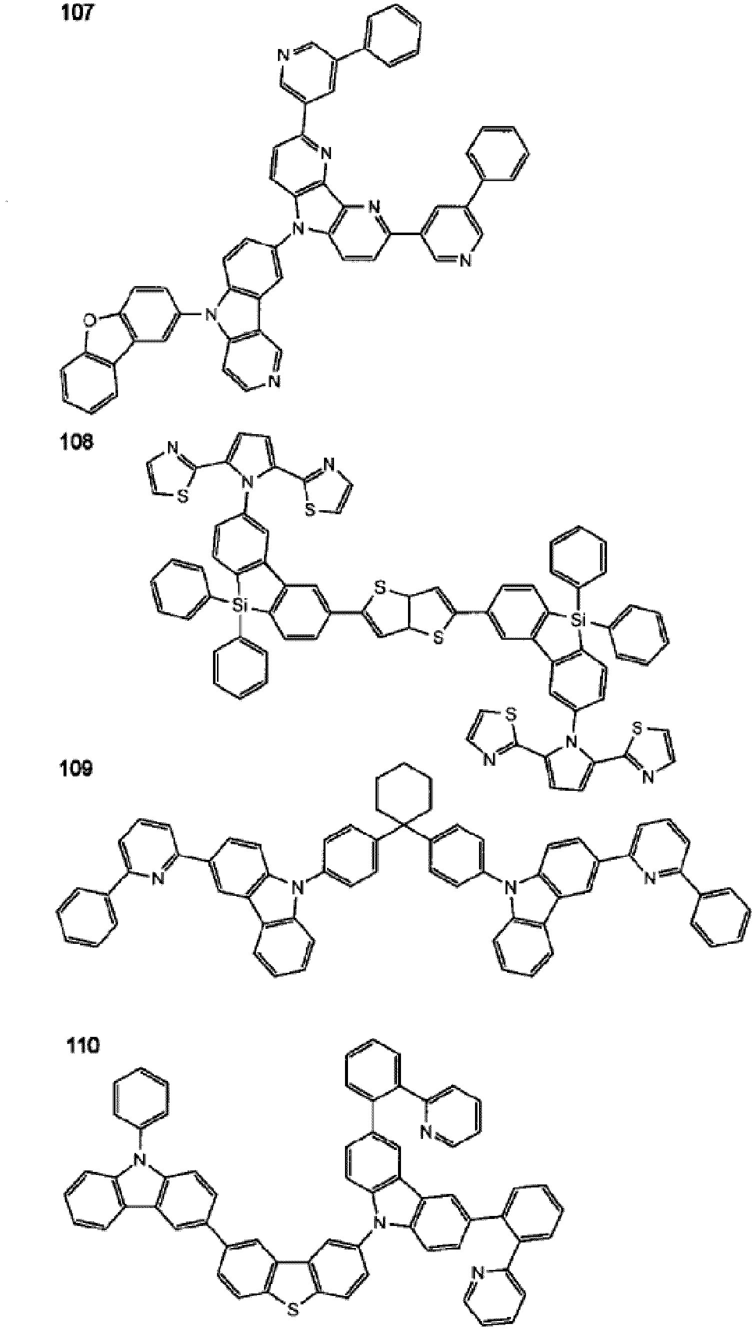

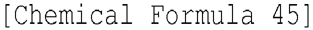

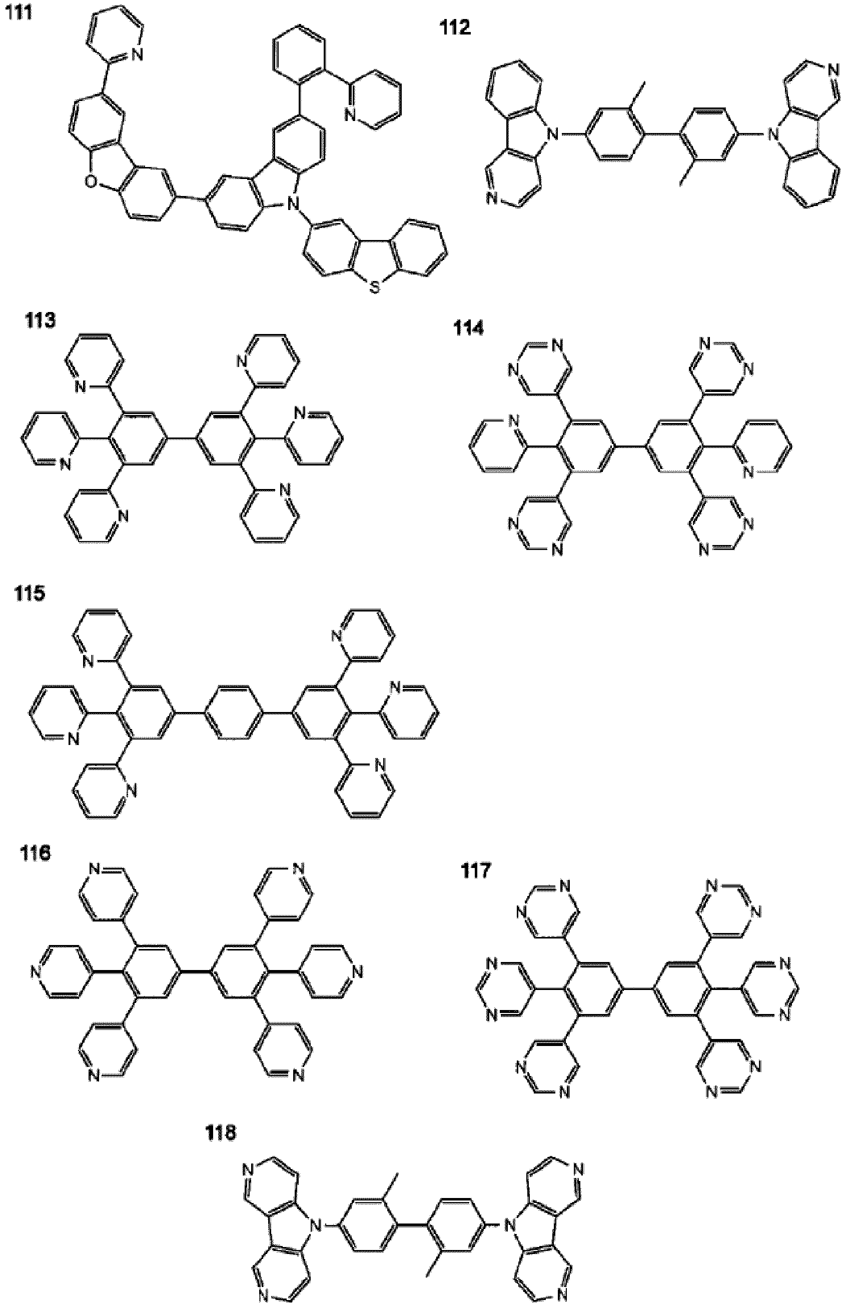

### [Synthesis example of compound]

A specific synthesis example of Compound 5 (not according to the invention) is shown below as the typical synthesis example of the compound; however, the present invention is not limited thereto.

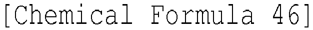

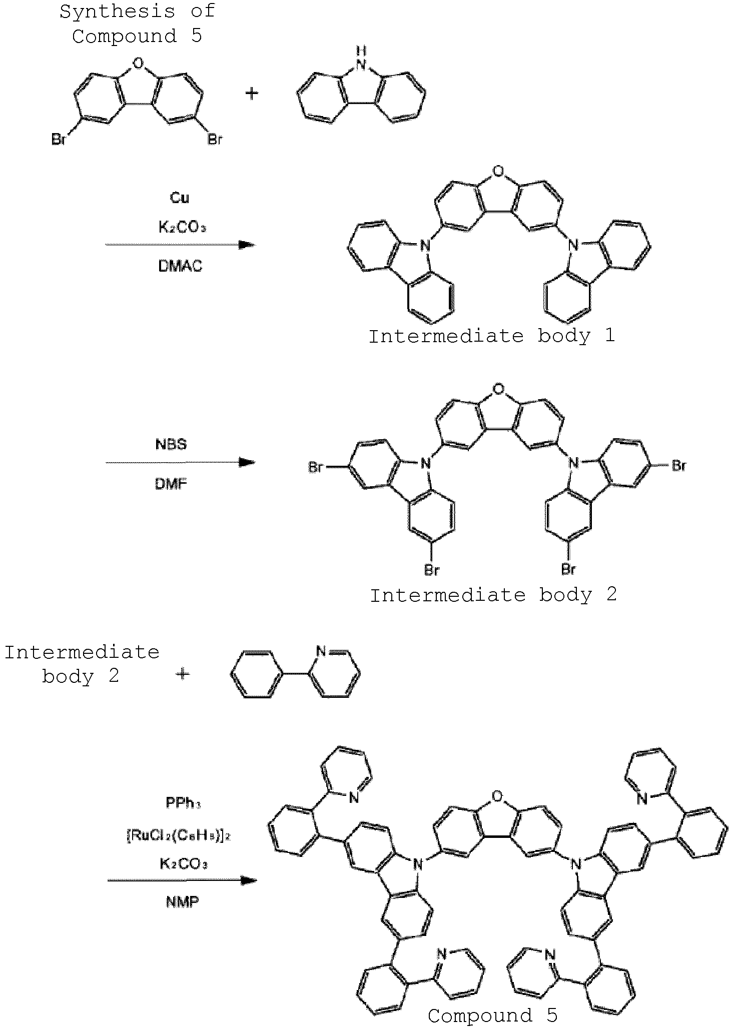

### Step 1: (Synthesis of intermediate body 1)

In 300 ml of DMAc (dimethyl acetamide), 2,8-dibromobenzofuran (1.0 mol), carbazole (2.0 mol), copper powder (3.0 mol), and potassium carbonate (1.5 mol) were mixed under a nitrogen atmosphere, and then the mixture was stirred for 24 hours at 130°C. The reaction liquid obtained thereby was cooled down to room temperature; then, 1 L of toluene was added thereto and washing was conducted three times with distilled water. Then, the solvent was removed from the washed product at the reduced-pressure atmosphere. The residue was purified with silica gel flash chromatography (n-heptane: toluene = 4:1 to 3:1), whereby the intermediate body 1 was obtained at a yield of 85%.

### Step 2: (Synthesis of intermediate body 2)

The intermediate body 1 (0.5 mol) was dissolved in 100 ml of DMF (dimethylformamide) in the air atmosphere at room temperature and NBS (N- bromosuccinimide) (2.0 mol) was added thereto; then, the mixture was stirred overnight at room temperature. The obtained precipitate was filtered and washed with methanol, whereby the intermediate body 2 was obtained at a yield of 92%.

### Step 3: (Synthesis of Compound 5)

In 3 L of NMP (N-methyl-2-pyrolidone), the intermediate body 2 (0.25 mol), 2-phenylpyridine (1.0 mol), a ruthenium complex [(η₆-C₆H₆)RuCl₂]₂ (0.05 mol), triphenylphosphine (0.2 mol), and potassium carbonate (12 mol) were mixed under a nitrogen atmosphere and stirred overnight at 140°C.

After the reaction liquid was cooled down to room temperature, 5 L of dichloromethane was added thereto and the reaction liquid was filtered. Next, the solvent was removed from the filtrate under the reduced-pressure atmosphere (800 Pa, 80°C) . The residue was purified with the silica gel flash chromatography (CH₂Cl₂:Et₃N = 20:1 to 10:1).

After the solvent was removed from the purified product under the reduced-pressure atmosphere, the residue was dissolved again in dichloromethane and washing was conducted with water three times. The substance obtained after the washing was dried with anhydrous magnesium sulfate and the solvent was removed from the dried substance under the reduced-pressure atmosphere, whereby Compound 5 was obtained at a yield of 68%.

### [Film-formation method of nitrogen-containing layer 3]

In the case where the nitrogen-containing layer 3 as above is formed on the transparent substrate 11, the film-formation method thereof may be a method employing a wet process, such as an application method, an inkjet method, a coating method, or a dipping method, or a method employing a dry process, such as an evaporation method (resistive heating, EB method, or the like), a sputtering method, or a CVD method. Above all, the evaporation method is preferable.

In particular, in the case of forming the nitrogen-containing layer 3 using a plurality of compounds, the co-evaporation in which a plurality of compounds is supplied at the same time from a plurality of evaporation sources is applied. In the case of using a polymer compound as the compound, the application method is preferably employed. In this case, an application liquid in which the compound is dissolved in a solvent is used. The solvent for dissolving the compound is not limited. Moreover, in the case of forming the nitrogen-containing layer 3 using a plurality of compounds, the application liquid may be manufactured using a solvent capable of dissolving the plural compounds.

### <Electrode layer 5>

The electrode layer 5 is a layer mainly containing silver, formed using silver or an alloy mainly containing silver, and provided adjacent to the nitrogen-containing layer 3. As the film-formation method for such an electrode layer 5, a method using a wet process, such as an application method, an inkjet method, a coating method, or a dipping method, or a method using a dry process, such as an evaporation method (resistive heating, EB method, or the like), a sputtering method, or a CVD method is given. Above all, the evaporation method is preferably employed. The electrode layer 5 has the sufficient conductivity even though the high-temperature annealing or the like is not performed after the film-formation, because the electrode layer 5 is formed on the nitrogen-containing layer 3. As necessary, however, the high-temperature annealing or the like may be performed after the film formation.

As the alloy mainly containing silver (Ag) to be included in the electrode layer 5, for example, silver magnesium (AgMg), silver copper (AgCu), silver palladium (AgPd), silver palladium copper (AgPdCu), silver indium (AgIn), or the like is given.

The electrode layer 5 as above may have a structure in which a plurality of layers of silver or the alloy mainly containing silver is stacked as necessary.

Moreover, the electrode layer 5 preferably has a thickness of 4 to 12 nm. With a thickness of 12 nm or less, the absorbing component or reflecting component of the film can be suppressed low and the light transmission of the transparent barrier film is maintained, which is preferable. Moreover, with a thickness of 4 nm or more, the layer conductivity is secured.

The transparent electrode 1 with the multilayer structure including the nitrogen-containing layer 3 and the electrode layer 5 provided adjacent thereto may have the upper part of the electrode layer 5 covered with a protective film or have another conductive layer stacked thereon. In this case, the protective film or the conductive layer preferably has light-transmitting property so that the light-transmitting property of the transparent electrode 1 is not deteriorated. Below the nitrogen-containing layer 3, i.e., between the nitrogen-containing layer 3 and the transparent substrate 11 may be provided a layer as necessary.

### <Effect of transparent electrode 1>

The transparent electrode 1 has the structure in which the electrode layer 5 mainly containing silver is provided adjacent to the nitrogen-containing layer 3 formed using the compound containing nitrogen atoms. Thus, when the electrode layer 5 is formed adjacent to the nitrogen-containing layer 3, the silver atom included in the electrode layer 5 mutually operates with the compound containing the nitrogen atom included in the nitrogen-containing layer 3, whereby the diffusion distance of the silver atom on the surface of the nitrogen-containing layer 3 is reduced to suppress the aggregation of silver. Therefore, a silver thin film, which is easily isolated in an island-shape due to the film growth through the nucleus growth type (Volume-Weber: VW type), comes to be formed through the film growth of the single-layer growth type (Frank-van der Merwe: FM type) . As a result, the electrode layer 5 mainly containing silver, which is thin but has the uniform thickness, can be obtained on the nitrogen-containing layer 3. Thus, the electrode layer 5 mainly containing silver whose conductivity is secured because the thickness is uniform, while the light-transmitting property is secured because the thickness is small, can be obtained.

It has been confirmed that the transparent electrode 1 as above has the low sheet resistance despite the small thickness as compared to the transparent electrode including ITO as described in the example below. Moreover, this transparent electrode 1 has the multilayer structure of the uniform nitrogen-containing layer 3 and electrode layer 5, so that the light scattering is also suppressed. As a result, when the transparent electrode 1 is used as the transparent electrode for a touch panel, the electrode 1 does not deteriorate the visibility of a displayed image in the base, and the electrode 1 can even be used as the transparent electrode for a large-sized touch panel.

The transparent electrode 1 as above costs low because indium (In), which is a rare metal, is not used. Moreover, since a chemically instable material such as ZnO is not used, the long-term reliability thereof is excellent.

### <<2. First modified example of transparent electrode for touch panel>>

### (Example of providing intermediate layer)

Fig. 2 is a schematic sectional diagram for describing a first modified example of the transparent electrode for a touch panel according to this embodiment. As illustrated in this drawing, a transparent electrode 1a of the first modified example has a structure in which the nitrogen-containing layer 3 and the electrode layer 5 are stacked with an intermediate layer A interposed therebetween, and the structure except the intermediate layer A is similar to that of the transparent electrode for a touch panel according to this embodiment described with reference to Fig. 1. Thus, description is made of only the structure of the intermediate layer A here.

### <Intermediate layer A>

The intermediate layer A is provided between and in contact with the nitrogen-containing layer 3 and the electrode layer 5. It is important that the intermediate layer A is thin enough not to deteriorate the light-transmitting property of the transparent electrode 1a and not to interrupt the influence of the nitrogen atom contained in the nitrogen-containing layer 3 on the electrode layer 5. Therefore, the intermediate layer A may have a thickness of 1 nm or less and is not necessarily the continuous film but may have an island-like shape or a shape with a plurality of holes. In this case, the nitrogen-containing layer 3 and the electrode layer 5 stacked with the intermediate layer A interposed therebetween are disposed partially adjacent to each other.

The intermediate layer A as above is the layer formed of an organic material or a conductive material. In the case of using the organic material, nitrogen may be omitted. Moreover, as the conductive material, magnesium, aluminum, copper, indium lithium, or an alloy containing any of these may be used.

### <Effect of transparent electrode 1a>

The transparent electrode 1a as above has the multilayer structure of the nitrogen-containing layer 3, the intermediate layer A, and the electrode layer 5, which has lower sheet resistance despite the smaller thickness than the transparent electrode including ITO and which is uniform differently from the metal nanowire, in a manner similar to the transparent electrode of the above described embodiment; therefore, the light scattering is also suppressed. As a result, when the transparent electrode 1a is used as the transparent electrode for a touch panel, the electrode 1a does not deteriorate the visibility of a displayed image in the base, and the electrode 1a can even be used as the transparent electrode for a large-sized touch panel.

### <<3. Second modified example of transparent electrode for touch panel>>

### (Example of providing high-refractive-index layer)

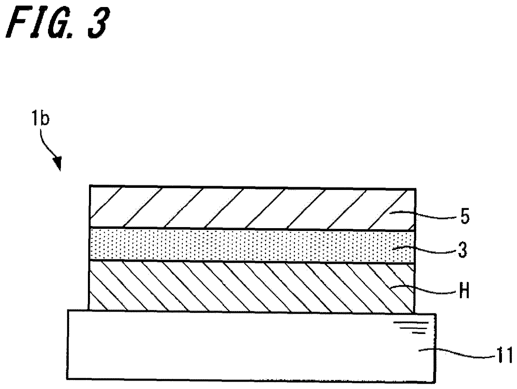

Fig. 3 is a schematic sectional diagram for describing a second modified example of the transparent electrode for a touch panel according to this embodiment. As illustrated in this drawing, a transparent electrode 1b of the second modified example has a structure in which a high-refractive-index layer H has the nitrogen-containing layer 3 held between the layer H and the electrode layer 5. The high-refractive-index layer H, the nitrogen-containing layer 3, and the electrode layer 5 are stacked in this order from the transparent substrate 11 side. The structure of the transparent electrode 1b except the high-refractive-index layer H is similar to the transparent electrode for a touch panel according to this embodiment described with reference to Fig. 1. Therefore, the description of the transparent electrode 1b is mainly made of the structure of the high-refractive-index layer H here.

### <High-refractive-index layer H>

The high-refractive-index layer H is a layer having a higher refractive index than the above nitrogen-containing layer 3. The refractive index of the high-refractive-index layer H at a wavelength of 550 nm is preferably 0.1 or more, preferably 0.3 or more, higher than that of the nitrogen-containing layer 3 (n = 1.7 to 1.8). The high-refractive-index layer H as above includes the material with the high refractive index and the light-transmitting property, and for example, a high-refractive-index material generally used for an optical film, such as titanium oxide (TiO₂: n = 2.3 to 2.4), zirconium oxide (ZrO: n = 2.4), cadmium oxide (CdO: n = 2.49), indium tin oxide (ITO: n = 2.1 to 2.2), hafnium oxide (HfO₂: n = 1.9 to 2.1), tantalum pentoxide (Ta₂O₅: n=2.16), niobium oxide (Nb₂O₅: n = 2.2 to 2.4), can be used.

Note that in the transparent electrode 1b having such a high-refractive-index layer H, the nitrogen-containing layer 3 preferably has a thickness of 5 nm or less. As described below in the example, when the nitrogen-containing layer 3 has a thickness of 5 nm or less, i.e., when the distance between the high-refractive-index layer H and the electrode layer 5 is 5 nm or less, the letters formed in the base is easily visible through the transparent electrode 1b and thus it has been confirmed that the transparent electrode 1b has the high light-transmitting property. The lower-limit value of the film thickness of the nitrogen-containing layer 3, however, is the thickness of such a degree that the film growth in the FM type of the electrode layer 5 formed on the nitrogen-containing layer 3 is not interrupted, i.e., such a degree that the nitrogen-containing layer 3 is formed as the continuous film covering the high-refractive-index layer H without being the island-like shape.

### <Effect of transparent electrode 1b>

The transparent electrode 1b as above has the multilayer structure of the high-refractive-index layer H, the nitrogen-containing layer 3, and the electrode layer 5, which has lower sheet resistance despite the smaller thickness than the transparent electrode including ITO and which is uniform, in a manner similar to the transparent electrode of the above described embodiment; therefore, the light scattering is also suppressed. In addition, the transparent electrode 1b has the high-refractive-index layer H, and the high-refractive-index layer H, the nitrogen-containing layer 3, and the electrode layer 5 are stacked in this order. Thus, the reflection on the electrode layer 5 mainly containing silver is suppressed and the light-transmitting property of the transparent electrode 1b is further improved. As a result, when the transparent electrode 1b is used as the transparent electrode for a touch panel, the electrode 1b further improves the visibility of a displayed image in the base, and the electrode 1b can even be used as the transparent electrode for a large-sized touch panel.

The transparent electrode 1b of the second modified example may be combined with the structure of the transparent electrode 1a of the first modified example described with reference to Fig. 2. In this case, the intermediate layer A described with reference to Fig. 2 is provided between the electrode layer 5 and the nitrogen-containing layer 3 in the transparent electrode 1b illustrated in Fig. 3.

### <<4. Touch panel>>

### (Structure 1 in which two-layer transparent electrode is provided on transparent substrate)

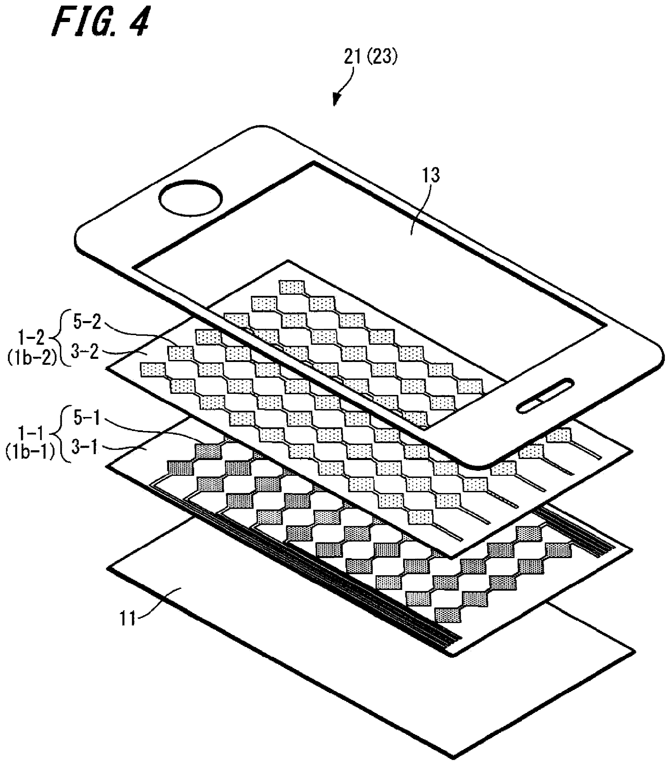

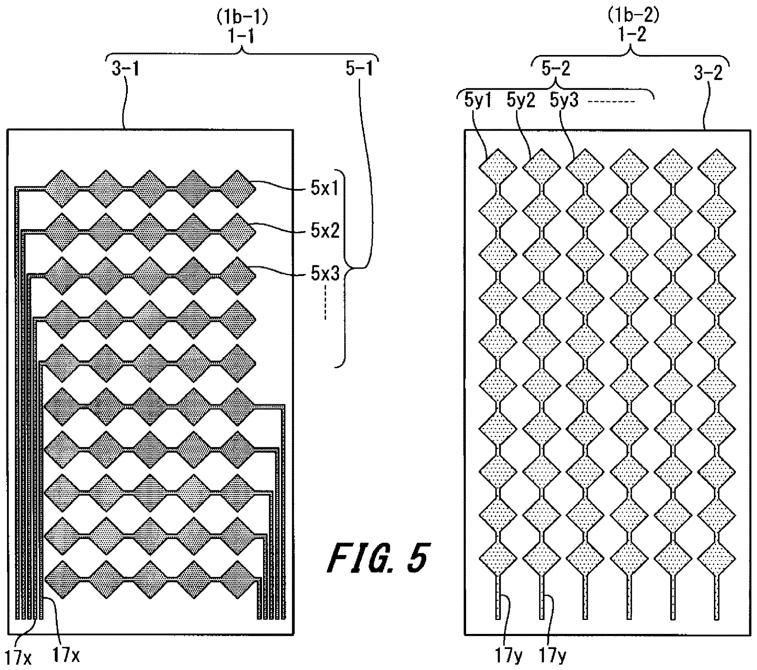

Fig. 4 is a perspective diagram illustrating a schematic structure of a touch panel 21 including the aforementioned transparent electrode for a touch panel. Moreover, Fig. 5 is a plan diagram of two transparent electrodes 1-1 and 1-2, which illustrates the electrode structure of the touch panel 21.

The touch panel 21 in the drawings corresponds to a projected capacitance type touch panel. In this touch panel 21, a first transparent electrode 1-1 and a second transparent electrode 1-2 are provided on a main plane of the transparent substrate 11 in this order, and an upper part thereof is covered with a front plate 13. Each of the first transparent electrode 1-1 and the second transparent electrode 1-2 is any of the transparent electrodes for a touch panel described with reference to Fig. 1 or Fig. 2. Therefore, the first transparent electrode 1-1 includes a first nitrogen-containing layer 3-1 and a first electrode layer 5-1 stacked thereon. Similarly, the second transparent electrode 1-2 includes a second nitrogen-containing layer 3-2 and a second electrode layer 5-2 stacked thereon.

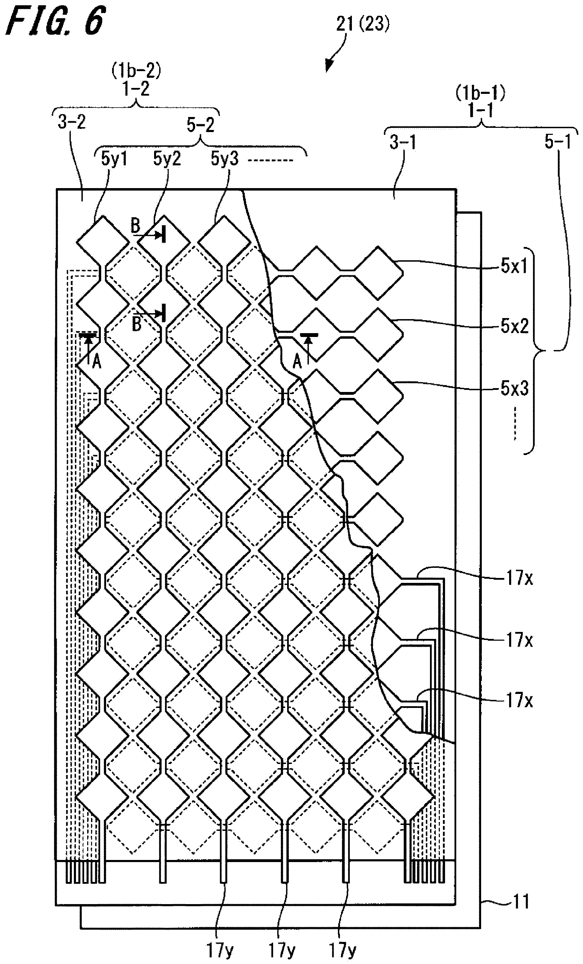

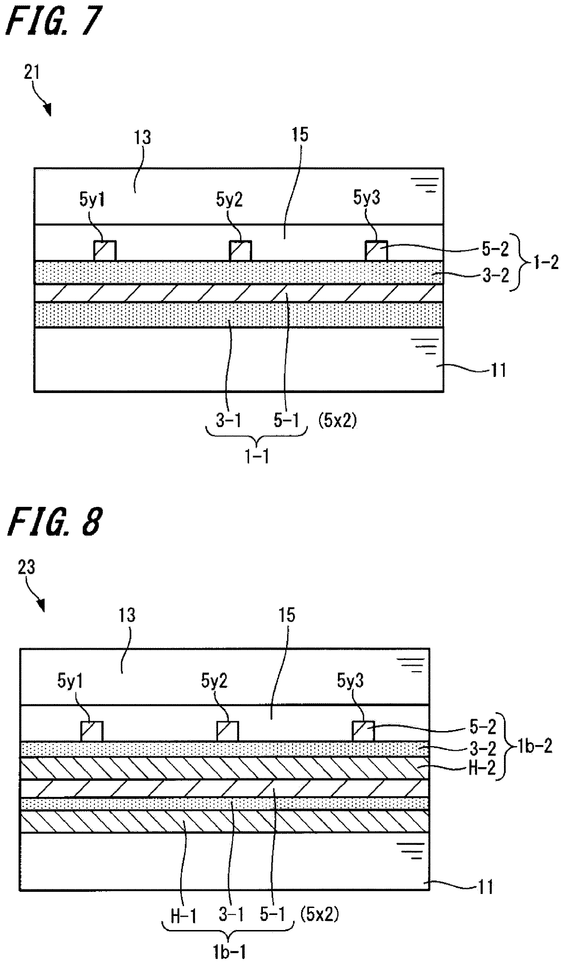

The details of the main layers included in the touch panel 21 are hereinafter described in the order from the transparent substrate 11 side. Note that description is made with reference to Fig. 4, Fig. 5, the schematic plan diagram of the electrode portion of Fig. 6 and the schematic sectional diagram of Fig. 7 corresponding to the A-A section. Note that the component similar to that of Fig. 1 is denoted by the same reference symbol and the overlapping description is omitted.

### <Transparent substrate 11>

The transparent substrate 11 illustrated in Fig. 4, Fig. 6, and Fig. 7 is the transparent substrate 11 used in the transparent electrode for a touch panel described above.

### <First nitrogen-containing layer 3-1 (first transparent electrode 1-1)>

The first nitrogen-containing layer 3-1 is the nitrogen-containing layer used in the transparent electrode for a touch panel described above, and is formed on one main plane of the transparent substrate 11. Here, for example, the first nitrogen-containing layer 3-1 is provided covering the entire surface of the one main plane of the transparent substrate 11; however, the first nitrogen-containing layer 3-1 may be patterned in the same shape as the first electrode layer 5-1 to be described next.

### <First electrode layer 5-1 (first transparent electrode 1-1)>

The first electrode layer 5-1 is the electrode layer used in the transparent electrode for a touch panel described above, and is structured as a plurality of x electrode patterns 5x1, 5x2, ... patterned on the first nitrogen-containing layer 3-1. The x electrode patterns 5x1, 5x2, ... are disposed in parallel to each other with a space therebetween while extending in the x direction. The x electrode patterns 5x1, 5x2, ... have the shape that the rhomboid patterned shape disposed in the x direction is connected to another rhomboid shape linearly in the x direction near the apex portion of the rhomboid shape.

Each of the x electrode patterns 5x1, 5x2, ... has the end thereof connected to an x wire 17x. Each of the x wires 17x is wired in the peripheral region on the transparent substrate 11, and led out of the edge of the transparent substrate 11. In a manner similar to the x electrode patterns 5x1, 5x2, ..., each of the x wires 17x may be formed as the first electrode layer 5-1 mainly containing silver or may be formed using a separately formed electrode layer.

### <Second nitrogen-containing layer 3-2 (second transparent electrode 1-2)>

The second nitrogen-containing layer 3-2 is the nitrogen-containing layer used in the transparent electrode for a touch panel described above, and is formed on one main plane of the transparent substrate 11 in the state of covering the first electrode layer 5-1. The second nitrogen-containing layer 3-2 is provided to expose at least the terminal portion of the x wire 17x while covering the first electrode layer 5-1. Here, as an example, the second nitrogen-containing layer 3-2 is provided to expose the terminal portion of the x wire 17x while the other portion covers the entire surface of the one main plane of the transparent substrate 11. However, the second nitrogen-containing layer 3-2 may be patterned into the same shape as the second electrode layer 5-2 to be described next.

### <Second electrode layer 5-2 (second transparent electrode 1-2)>

The second electrode layer 5-2 is the electrode layer used in the transparent electrode for a touch panel described above, and is structured as a plurality of y electrode patterns 5y1, 5y2, ... patterned on the second nitrogen-containing layer 3-2. The y electrode patterns 5y1, 5y2, ... are disposed in parallel to each other with a space therebetween in the state of extending in a y direction, which is orthogonal to the x electrode patterns 5x1, 5x2, .... The y electrode patterns 5y1, 5y2, ... have the shape that the rhomboid patterned shape disposed in the y direction is connected to another rhomboid shape linearly in the y direction near the apex portion of the rhomboid shape.

As illustrated in Fig. 6, the rhomboid patterned portion of each of the y electrode patterns 5y1, 5y2, ... is disposed not overlapping with the rhomboid patterned portion of each of the x electrode patterns 5x1, 5x2, ... in plan view, and has the shape occupying as large area as possible in the range of avoiding the overlap. Thus, the central region of the transparent substrate 11 has the structure in which the x electrode patterns 5x1, 5x2, ... formed of the first electrode layer 5-1 and the y electrode patterns 5y1, 5y2, ... formed of the second electrode layer 5-2 are less visible.

The y electrode patterns 5y1, 5y2, ... are stacked on the x electrode patterns 5x1, 5x2, ... only in the connecting portion of the rhomboid electrode patterns. In the stacked portion, the second nitrogen-containing layer 3-2 is held and this secures the insulating property between the x electrode patterns 5x1, 5x2, ... and the y electrode patterns 5y1, 5y2, ....

At an end of each of the y electrode patterns 5y1, 5y2, ..., a y wire 17y is connected. The y wires 17y are wired in the peripheral region on the transparent substrate 11 and led out of the edge of the transparent substrate 11 beside the x wires 17x. Such y wires 17y may be formed as the second electrode layer 5-2 mainly containing silver in a manner similar to the y electrode patterns 5y1, 5y2, ... or may be formed using a separately formed electrode layer.

Note that the x wires 17x and the y wires 17y led out of the edge of the transparent substrate 11 are connected to a flexible printed board or the like.

### <Front plate 13>

The front plate 13 illustrated in Fig. 4 and Fig. 7 is a plate material whose portion corresponding to an input position in the touch panel 21 is pressed. The front plate 13 as above is the plate material with the light-transmitting property, and is similar to the transparent substrate 11. For the front plate 13, the material with the necessary optical characteristic may be selected and used. The front plate 13 as above is attached to the second transparent electrode 1-2 side with an adhesive 15 (see Fig. 7), for example. The material of the adhesive 15 is not particularly limited as long as the material has the light-transmitting property.

The front plate 13 is provided with a light-blocking film that covers the periphery of the transparent substrate 11, which prevents the x wires 17x led out of the x electrode patterns 5x1, 5x2, ... and the y wires 17y led out of the y electrode patterns 5y1, 5y2, ... from being viewed from the front plate 13 side.

Note that the first transparent electrode 1-1 and the second transparent electrode 1-2 may be configured as the transparent electrode 1a for a touch panel described with reference to Fig. 2. In this case, in regard to the first electrode layer 1-1, the intermediate layer patterned to be the same shape as the first electrode layer 5-1 may be held between the first nitrogen-containing layer 3-1 and the first electrode layer 5-1. Similarly, in regard to the second transparent electrode 1-2, the intermediate layer patterned into the same shape as the second electrode layer 5-2 may be held between the second nitrogen-containing layer 3-2 and the second electrode layer 5-2.

### <Operation of touch panel>

In the case of operating the touch panel 21 as above, voltage is applied to the x electrode patterns 5x1, 5x2, ... and the y electrode patterns 5y1, 5y2, ... from the flexible printed board connected to the x wires 17x and y wires 17y or the like. If a finger or a touch pen touches the surface of the front plate 13 in the state, the capacitance of each portion within the touch panel 21 is changed to cause the voltage in the x electrode patterns 5x1, 5x2, ... and the y electrode patterns 5y1, 5y2, ... to change. This change varies depending on the distance from the position where the finger or touch pen is in contact, and is the largest at the position where the finger or touch pen is in contact. Therefore, the position addressed by the x electrode patterns 5x1, 5x2, ... and the y electrode patterns 5y1, 5y2, ..., where the voltage change is the maximum, is detected as the position where the finger or the touch pen is in contact.

### <Effect of touch panel 21>

The aforementioned touch panel 21 includes the transparent electrode for a touch panel with the low sheet resistance despite the small thickness described above as the two-layer transparent electrodes 1-1 and 1-2, whereby the voltage drop of the transparent electrode for a touch panel can be suppressed, and therefore the size of the touch panel 21 can be increased.

In particular, this touch panel 21 is the projected capacitance type having the x electrode patterns 5x1, 5x2, ... and the y electrode patterns 5y1, 5y2, ... provided orthogonal thereto. Therefore, the x electrode patterns 5x1, 5x2, ... and the y electrode patterns 5y1, 5y2, ... are required to have high conductivity. In this regard, the x electrode patterns 5x1, 5x2, ... and the y electrode patterns 5y1, 5y2, ... are formed of the electrode layer 5 for the transparent electrode for a touch panel described above; therefore, the thickness can be reduced while the conductivity is maintained. Accordingly, the x electrode patterns 5x1, 5x2, ... and the y electrode patterns 5y1, 5y2, ... are hardly visible and the deterioration in visibility of the displayed image in the base through the touch panel 21 can be prevented.

### <<5. First modified example of touch panel>>

### (Structure 2 in which two-layer transparent electrode is provided on transparent substrate)

Fig. 8 is a schematic sectional diagram for describing a first modified example of the touch panel according to this embodiment, and corresponds to the A-A section of Fig. 6. A touch panel 23 in this drawing has a structure on the transparent substrate 11 in which the two-layer transparent electrode 1b with the high-refractive-index layer H described above with reference to Fig. 3 is provided, and the other structure is similar to that of the touch panel 21 of this embodiment described with reference to Fig. 4 to Fig. 7. Accordingly, the detailed structure of the touch panel 23 of the first modified example is described by applying the reference symbols similar to those of Fig. 4 to Fig. 6 and the sectional diagram of Fig. 8, which are used in the description of the touch panel 21 of this embodiment, and the overlapping description is omitted.

In the touch panel 23 of the first modified example, a first transparent electrode 1b-1 and a second transparent electrode 1b-2 are disposed in this order on one main plane of the transparent substrate 11, and an upper part thereof is covered with the front plate 13. Each of the first transparent electrode 1b-1 and the second transparent electrode 1b-2 is the transparent electrode for a touch panel described with reference to Fig. 3. Therefore, the first transparent electrode 1b-1 has a structure in which a first high-refractive-index layer H-1, a first nitrogen-containing layer 3-1, and a first electrode layer 5-1 are stacked in this order. Similarly, the second transparent electrode 1-2 has a structure in which a second high-refractive-index layer H-2, a second nitrogen-containing layer 3-2, and a second electrode layer 5-2 are stacked in this order.

The touch panel 23 as above is different from the touch panel 21 in this embodiment only in that the first high-refractive-index layer H-1 and the second high-refractive-index layer H-2 are provided in the multilayer structure.

### <First high-refractive-index layer H-1 (first transparent electrode 1b-1)>

The first high-refractive-index layer H-1 (see Fig. 8) is the high-refractive-index layer used in the transparent electrode for a touch panel described above, and is formed on one main plane of the transparent substrate 11. Here, as an example, the first high-refractive-index layer H-1 is provided covering the entire surface of the one main plane of the transparent substrate 11; however, the first high-refractive-index layer H-1 may alternatively be patterned into the same shape as the first electrode layer 5-1 together with the first nitrogen-containing layer 3-1.

### <Second high-refractive-index layer H-2 (second transparent electrode 1-2)>

The second high-refractive-index layer H-2 (see Fig. 8) is the high-refractive-index layer used in the transparent electrode for a touch panel described above, and is formed on one main plane of the transparent substrate 11 while covering the first electrode layer 5-1. The second high-refractive-index layer H-2 as above is provided in the state of exposing at least the terminal portion of the x wire 17x while covering the first electrode layer 5-1. Here, as an example, the second high-refractive-index layer H-2 is provided to expose the terminal portion of the x wire 17x in a manner similar to the second nitrogen-containing layer 3-2 while the other portion covers the entire one main plane of the transparent substrate 11; however, the second high-refractive-index layer H-2 may alternatively be patterned into the same shape as the second electrode layer 5-2 together with the second nitrogen-containing layer 3-2.

The touch panel 23 with the high-refractive-index layers H-1 and H-2 as above can be operated in a manner similar to the touch panel of this embodiment.

### <Effect of touch panel 23>

The aforementioned touch panel 23 includes the transparent electrode for a touch panel having the aforementioned light-transmitting property and sufficient conductivity as the two-layer transparent electrodes 1b-1 and 1b-2. Thus, the voltage drop that would occur if the transparent electrode for a touch panel were increased in size can be suppressed while the visibility of the displayed image in the base is maintained, and the size of the touch panel 23 can be increased.

In particular, this touch panel 23 is the projected capacitance type having the x electrode patterns 5x1, 5x2, ... and the electrode patterns 5y1, 5y2, ... disposed orthogonal thereto. Therefore, the x electrode patterns 5x1, 5x2, ... and the y electrode patterns 5y1, 5y2, ... are required to have high conductivity. In this regard, the x electrode patterns 5x1, 5x2, ... and the y electrode patterns 5y1, 5y2, ... can be reduced in thickness while the conductivity is maintained because these patterns are the electrode layer 5 of the transparent electrode for a touch panel described above. Accordingly, the x electrode patterns 5x1, 5x2, ... and the y electrode patterns 5y1, 5y2, ... become less visible and the deterioration in visibility of the displayed image in the base through the touch panel 23 can be prevented.

### <<6. Second modified example of touch panel>>

### (Structure in which two transparent substrates are used)

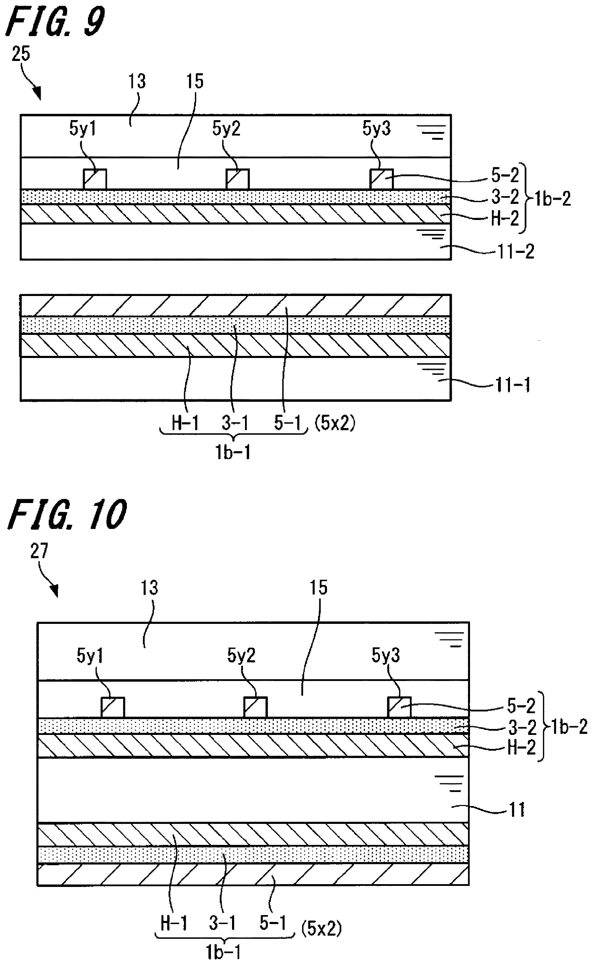

Fig. 9 is a schematic sectional diagram for describing a second modified example of the touch panel according to this embodiment, and corresponds to the A-A section of Fig. 6. A touch panel 25 illustrated in this drawing has a structure in which the first transparent electrode 1b-1 and the second transparent electrode 1b-2 are provided on one main plane of two transparent substrates 11-1 and 11-2, and the other structure is similar to that of this embodiment described above. Thus, the structure similar to that of the touch panel of this embodiment is denoted by the same reference symbol and the overlapping description is omitted.

That is to say, the touch panel 25 of the second modified example includes the first transparent substrate 11-1 provided with the first transparent electrode 1b-1 and the second transparent substrate 11-2 provided with the second transparent electrode 1b-2. These transparent substrates 11-1 and 11-2 are disposed so that the surfaces thereof provided with the transparent electrodes 1b-1 and 1b-2 face in the same direction and the second transparent substrate 11-2 is positioned on the surface of the first transparent substrate 11-1 where the first transparent electrode 1b-1 is formed.

The first transparent substrate 11-1 and the second transparent substrate 11-2 are the transparent substrate 11 similar to the substrate used in the transparent electrode for a touch panel described above. The first transparent electrode 1b-1 and the second transparent electrode 1b-2 each have the structure similar to that of the first modified example described with reference to Fig. 8, and respectively have the high-refractive-index layers H-1 and H-2, the nitrogen-containing layers 3-1 and 3-2, and the electrode layers 5-1 and 5-2 on the transparent substrates 11-1 and 11-2.

The structure of each of the electrode layers 5-1 and 5-2 is similar to that of this embodiment described above, and the x electrode patterns 5x1, 5x2, ... formed of the first electrode layer 5-1 and the y electrode patterns 5y1, 5y2, ... formed of the second electrode layer 5-2 have the less visible pattern structure and arrangement. Note that the insulating property between the first electrode layer 5-1 and the second electrode layer 5-2 is secured by the second transparent substrate 11-2, the second high-refractive-index layer H-2, and the second nitrogen-containing layer 3-2.

The first transparent substrate 11-1 and the second transparent substrate 11-2 that are stacked are bonded to each other with an adhesive that is not illustrated here. With this adhesive, the first electrode layer 5-1 and the second electrode layer 5-2 are electrically disconnected from each other.

The touch panel 25 as above can be operated in a manner similar to the touch panel according to this embodiment described above.

### <Effect of touch panel 25>

By the use of the transparent electrode for a touch panel having the aforementioned light-transmitting property and sufficient conductivity, the touch panel 25 according to the second modified example can be increased in size in a manner similar to the touch panel of this embodiment described above, and moreover, the deterioration in visibility of the displayed image in the base through the touch panel 25 can be prevented.

### <<7. Third modified example of touch panel>>

### (Structure in which single layer of transparent electrode is provided on each surface of transparent substrate)

Fig. 10 is a schematic sectional diagram for describing a modified example of the touch panel of this embodiment described above, and corresponds to the A-A section of Fig. 6. A touch panel 27 illustrated in this drawing has a structure in which one surface of the transparent substrate 11 is provided with the first transparent electrode 1b-1 and the other surface thereof is provided with the second transparent electrode 1b-2 . The other structure is similar to that of this embodiment described above. Therefore, the structure similar to that of the touch panel of this embodiment is denoted by the same reference symbol and the overlapping description is omitted.

In other words, the touch panel 27 according to the third modified example includes one transparent substrate 11, the first transparent electrode 1b-1 provided on one main plane side of the transparent substrate 11, and the second transparent electrode 1b-2 provided on the other main plane side of the transparent substrate 11. Among these, the first transparent electrode 1b-1 has the structure in which the high-refractive-index layer H-1, the nitrogen-containing layer 3-1, and the electrode layer 5-1 are stacked on one main plane of the transparent substrate 11 in this order. On the other hand, the second transparent electrode 1b-2 has the structure in which the high-refractive-index layer H-2, the nitrogen-containing layer 3-2, and the electrode layer 5-2 are stacked on the other main plane of the transparent substrate 11 in this order.

The transparent substrate 11 is similar to that of the transparent electrode for a touch panel described above. The above layers included in the first transparent electrode 1b-1 and the second transparent electrode 1b-2 are similar to those of the first modified example, and have a structure in which the high-refractive-index layers H-1 and H-2, the nitrogen-containing layers 3-1 and 3-2, and the electrode layers 5-1 and 5-2 are stacked in this order from the transparent substrate 11 side.

Moreover, the structure of each of the electrode layers 5-1 and 5-2 is similar to that of this embodiment described above and the x electrode patterns 5x1, 5x2, ... formed of the first electrode layer 5-1 and the y electrode patterns 5y1, 5y2, ... formed of the second electrode layer 5-2 have the less visible pattern structure and arrangement. Note that the insulating property between the first electrode layer 5-1 and the second electrode layer 5-2 is secured by the first nitrogen-containing layer 3-1, the first high-refractive-index layer H-1, the transparent substrate 11, the second high-refractive-index layer H-2, and the second nitrogen-containing layer 3-2.

The touch panel 27 as above can be operated in a manner similar to the touch panel of this embodiment described above.

### <Effect of touch panel 27>

By the use of the transparent electrode for a touch panel having the aforementioned light-transmitting property and sufficient conductivity, the touch panel 27 according to the third modified example can be increased in size in a manner similar to the touch panel of this embodiment described above, and moreover, the deterioration in visibility of the displayed image in the base through the touch panel 27 can be prevented.

### <<8. Fourth modified example of touch panel>>

### (Structure in which two patterns of transparent electrodes are provided on one plane of transparent substrate)

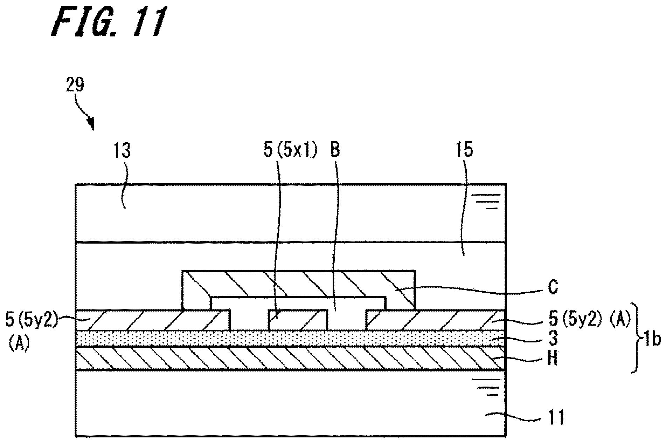

Fig. 11 is a schematic sectional diagram for describing a fourth modified example of the touch panel according to this embodiment described above, and corresponds to the B-B section of Fig. 6. The multilayer structure of a touch panel 29 of the fourth modified example and the multilayer structure of the touch panel 21 of Fig. 6 are different from each other in the following point: the touch panel 29 of Fig. 11 has the electrode layer 5 having both the x electrode patterns 5x1, 5x2, ... and the y electrode patterns 5y1, 5y2, ... on the same plane of the transparent substrate 11. Here, the structure similar to that of the touch panel of this embodiment described above is denoted by the same reference symbol and the overlapping description is omitted.

The touch panel 29 of the fourth modified example includes the transparent substrate 11, and the transparent electrode 1b including the high-refractive-index layer H, the nitrogen-containing layer 3, and the electrode layer 5 stacked in order on the transparent substrate 11. Moreover, an interlayer insulation film B and a connection electrode C are stacked in order on the electrode layer 5. Among those, the electrode layer 5 has both the x electrode patterns 5x1, 5x2, ... and the y electrode patterns 5y1, 5y2, ... that are electrically insulated from each other on the nitrogen-containing layer 3, which is characteristic.

### (Transparent substrate 11, high-refractive-index layer H, and nitrogen-containing layer 3)

The transparent substrate 11, the high-refractive-index layer H, and the nitrogen-containing layer 3 are similar to those of the transparent electrode for a touch panel described with reference to Fig. 3. As an example, the high-refractive-index layer H, and the nitrogen-containing layer 3 are provided covering the entire one main plane of the transparent substrate 11 but alternatively may be patterned into the same shape as the electrode layer 5 in a manner similar to the first modified example.

### (Electrode layer 5)

The electrode layer 5 is the electrode layer used in the transparent electrode for a touch panel described above, and has the structure in which the plural x electrode patterns 5x1, 5x2, ... and the plural y electrode patterns 5y1, 5y2, ... are patterned on the nitrogen-containing layer 3.

The x electrode patterns 5x1, 5x2, ... are arranged in parallel to each other with a space therebetween while extending in the x direction. The x electrode patterns 5x1, 5x2, ... have the shape that the rhomboid patterned shape disposed in the x direction is connected to another rhomboid shape linearly in the x direction near the apex portion of the rhomboid shape. This is common to this embodiment and other modified examples 1 to 3.

The y electrode patterns 5y1, 5y2, ... are arranged in parallel to each other with a space therebetween while extending in the y direction that is orthogonal to the x electrode patterns 5x1, 5x2, .... Each of the y electrode patterns 5y1, 5y2, ... is formed by a plurality of patterns A arranged in the y direction.

The pattern A has a rhomboid shape, for example, and is disposed spaced from the x electrode patterns 5x1, 5x2, ... so as to be electrically disconnected from the x electrode patterns. Thus, the insulating property is secured between the x electrode patterns 5x1, 5x2, ... and the patterns A forming the y electrode patterns 5y1, 5y2, .... Moreover, the rhomboidal pattern A has the shape as large as possible in the range of having a space of such a degree that the insulated state from the x electrode patterns 5x1, 5x2, ... can be maintained. Thus, in the central region of the transparent substrate 11, the x electrode patterns 5x1, 5x2, ... and the patterns A forming the y electrode patterns 5y1, 5y2, ... are made less visible.

The x electrode patterns 5x1, 5x2, ... and the pattern A that forms the y electrode patterns 5y1, 5y2, ... are connected to the x wires or the y wires at the end, in a manner similar to this embodiment.

### (Interlayer insulation film B, connection electrode C)

The connection electrode C is the electrode formed of a conductive layer separate from the electrode layer 5, and connects the patterns A that form the y electrode patterns 5y1, 5y2, ... linearly in the y direction near the apex of the rhomboidal pattern A. The connection electrode C is disposed at each position intersecting with the portion connecting the rhomboid patterns of the x electrode patterns 5x1, 5x2, ... in a plan view. At these intersecting portions, the interlayer insulation film B covers the portion connecting the rhomboid pattern of the x electrode patterns 5x1, 5x2, ... and the connection electrode C is stacked on the x electrode patterns 5x1, 5x2, ... with the interlayer insulation film B interposed therebetween. This secures the insulating property between the x electrode patterns 5x1, 5x2, ... and the y electrode patterns 5y1, 5y2, .... Note that the connection electrode C may be formed of a general electrode material such as silver, or an electrode material with the light-transmitting property such as ITO. From the viewpoint of the visibility of the displayed image in the base through the touch panel 29, the electrode material with the light-transmitting property is preferably used.

Note that in this fourth modified example, the connection electrode C is provided above the electrode layer 5; however, the connection electrode C may be provided below the electrode layer 5. In this case, in the transparent electrode 1b in which the high-refractive-index layer H, the nitrogen-containing layer 3, and the electrode layer 5 are stacked in this order on the transparent substrate 11, the connection electrode C is provided between the transparent substrate 11 and the high-refractive-index layer H or between the high-refractive-index layer H and the nitrogen-containing layer 3. The connection electrode C as above and the patterns A that form the y electrode patterns 5y1, 5y2, ... are connected to each other through the connection hole provided in the high-refractive-index layer H and the nitrogen-containing layer 3, or in the nitrogen-containing layer 3. The connection electrode C in this case is disposed at each position intersecting with the portion connecting the rhomboid patterns of the x electrode patterns 5x1, 5x2, ... in a plan view. At these intersecting portions, the high-refractive-index layer H and the nitrogen-containing layer 3 or the nitrogen-containing layer 3 is held between the connection electrode C and the portion connecting the rhomboid pattern of the electrode patterns 5x1, 5x2, .... Therefore, even in the example in which the connection electrode C is provided below the electrode layer 5, the insulating property is secured between the x electrode patterns 5x1, 5x2, ... and the y electrode patterns 5y1, 5y2, ....

The touch panel 29 as above can be operated in a manner similar to the touch panel of this embodiment.

### <Effect of touch panel 29>

By the use of the transparent electrode for a touch panel having the aforementioned light-transmitting property and sufficient conductivity, the touch panel 29 according to the second modified example can be increased in size in a manner similar to the touch panel of this embodiment described above, and moreover, the deterioration in visibility of the displayed image in the base through the touch panel 29 can be prevented.

The structure of each touch panel including the transparent electrode 1b described with reference to Fig. 3 has been described in the first to fourth modified examples. However, in the touch panel of these first to fourth modified examples, the transparent electrode 1b may be replaced by the transparent electrode 1 described with reference to Fig. 1 or the transparent electrode 1a described with reference to Fig. 2.

The touch panel according to the present invention is widely applicable to any structure including the transparent electrode without being limited to the structures of this embodiment and the modified examples above. The transparent electrode for a touch panel according to the present invention may be used as the transparent electrode. For example, in the case of the projected capacitance type touch panel, the x electrode patterns 5x1, 5x2, ... and the y electrode patterns 5y1, 5y2, ... disposed orthogonal thereto may be arranged while maintaining the insulating property, and the pattern shape is not limited. Moreover, the touch panel may be a resistive film type in which the two transparent electrodes 1-1 and 1-2 with the solid film shaped electrode layer 5 are disposed with a spacer therebetween, or a surface capacitance type; in any way, the effect of increasing the size can be obtained similarly.

### <<9. Display device>>

### (Structure including touch panel)

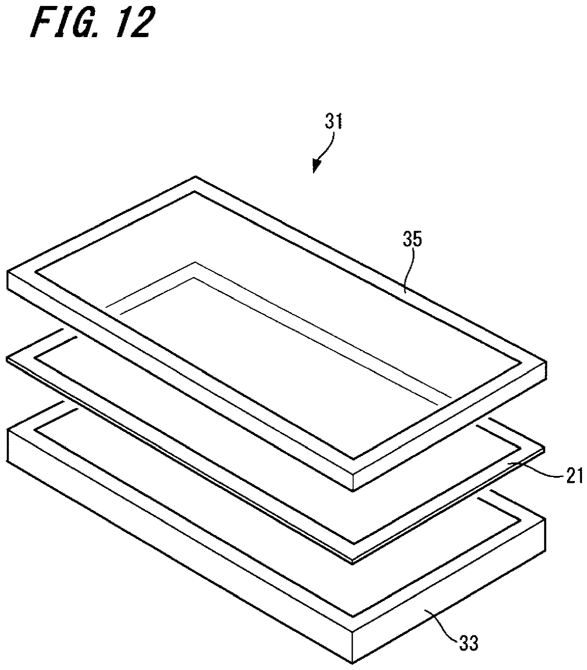

Fig. 12 is a perspective diagram illustrating a structure of a display device of the present invention. A display device 31 in this drawing is a display device having a function of information input provided with the touch panel according to the present invention, on the display surface of the display panel 33. Any of the touch panels according to this embodiment and the first to fourth modified examples can be applied as the touch panel as the present invention; here, the touch panel 21 described with reference to Fig. 4 to Fig. 7 is used.

A display panel 33 may be, for example, a planar display panel such as a liquid crystal display panel or a display panel formed using an organic electroluminescent element or a CRT (Cathode Ray Tube) display without particular limitation. Moreover, the display panel 33 may be a display panel displaying a still image without being limited to the display panel displaying a motion image.

The touch panel 21 is disposed overlapping the display surface of the image in the display panel 33. The touch panel 21 and the display panel 33 may be housed in a frame-shaped case member 35 as necessary, or this case member 35 may be provided with a front plate made of a transparent plate member.

Thus, by bringing a finger or a touch pen in contact with a part of the displayed image on the display panel 33 through the touch panel 21, a user can input the positional information of the contact portion in the touch panel 21.

### <Effect of display device>

The display device 31 with the structure as above can be reduced in thickness by the use of the touch panel 21 as above. Example 1

### <<Manufacture of transparent electrode for touch panel>>

A transparent electrode for a touch panel in each of Samples 1 to 27 (hereinafter referred to as a transparent electrode) was manufactured on a transparent substrate so as to have an area of 5 cm × 5 cm. As the transparent substrate, a polyethylene terephthalate (PET) substrate was prepared. Table 2 below shows the structure of each layer in each of the transparent electrodes of Samples 1 to 27. Description is hereinafter made of a procedure of manufacturing each of the transparent electrodes of Samples 1 to 27.

### <Manufacture of transparent electrode of Sample 1>

An ITO film (with a thickness of 100 nm) was formed on one main plane of the transparent substrate by a sputtering method. Thus, the transparent electrode with a single-layer structure in which the ITO film served as the electrode layer was manufactured.

### <Manufacture of transparent electrode of Samples 2 to 4>

In each of Samples 2 to 4, an electrode layer made of the silver nanowire was formed on one main plane of the transparent substrate by an application method. Thus, the transparent electrode with the single layer structure including just the electrode layer made of the silver nanowire was manufactured. Here, a dispersion liquid of the silver nanowire was applied and the application film thickness of the dispersion liquid of the silver nanowire was adjusted so that the thickness of the film obtained by the drying became 50 nm in Sample 2, 150 nm in Sample 3, and 200 nm in Sample 4.

### <Manufacture of transparent electrode of Sample 5>

An electrode layer (with a thickness of 8 nm) of silver (Ag) was formed on one main plane of the transparent substrate by an evaporation method. Thus, the transparent electrode with the single layer structure in which the silver was used for the electrode layer 5 was manufactured. On this occasion, the electrode was fixed to a base material holder of a commercial vacuum evaporation device, and attached to a vacuum tank of the vacuum evaporation device. Moreover, silver (Ag) was input to a resistive heating boat made of tungsten, and attached to the inside of the vacuum tank. Next, after the pressure in the vacuum tank was reduced to 4 × 10⁻⁴ Pa, electricity was supplied to the resistive heating boat so that heat can be applied thereto; thus, the electrode layer made of silver was formed with a thickness of 8 nm at an evaporation speed of 0.1 nm/sec to 0.2 nm/sec.

### <Manufacture of transparent electrode of Samples 6 to 25>

With reference to Fig. 1, the nitrogen-containing layer 3 (with a thickness of 25 nm) including each of the compounds shown in Table 2 below was formed by an evaporation method on one main plane of the transparent substrate 11, and then the electrode layer 5 made of silver (Ag) (with a thickness of 8 nm) was formed by the evaporation method in each of Samples 6 to 25. Thus, the transparent electrode 1 with the two-layer structure of the nitrogen-containing layer 3 and the electrode layer 5 was manufactured.

On this occasion, first, the transparent substrate 11 was fixed to the base material holder of the commercial evaporation device. Moreover, each of the compounds shown in Table 2 below was put into a resistive heating boat made of tantalum in the manufacture of each transparent electrode of Samples 6 to 25. These substrate holders and heating boat were attached to a first vacuum tank of the vacuum evaporation device. Moreover, silver (Ag) was put into a resistive heating boat made of tungsten and attached to a second vacuum tank.

Among the compounds used here, Compounds No.-1 to No.-3 are the ones shown below. Among these compounds, Compound No.-1 is anthracene not containing nitrogen atoms. Compounds No.-2 and No.-3 contain nitrogen but their effective unshared electron pair content ratio [n/M] is less than 2.0 × 10⁻³. In Compounds No.-2 and No.-3, a nitrogen atom having [effective unshared electron pair] is marked with a circle. The compounds No.-1 and No.-2 are not according to the invention.

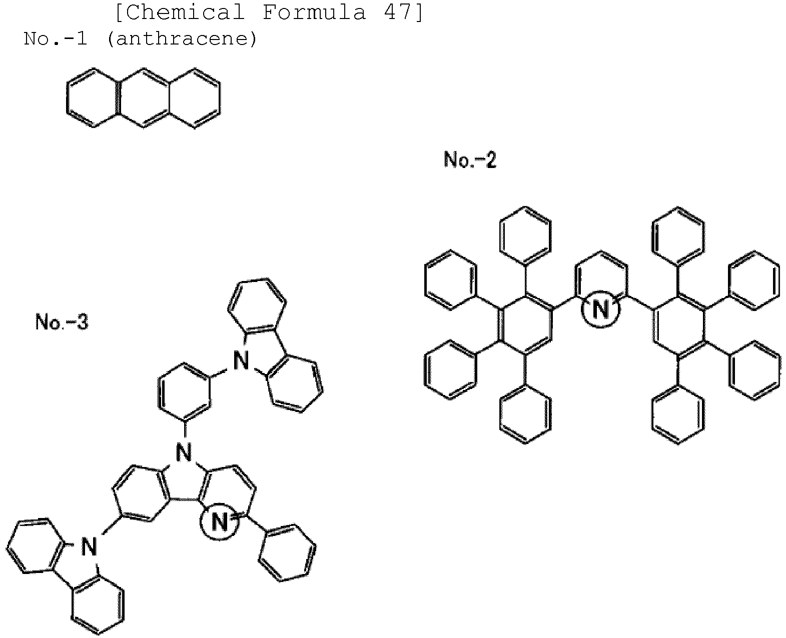

On the other hand, each of Compounds No. 1 to No. 16 and No. 18 is the compound containing nitrogen atoms described in this embodiment. Table 2 also shows the number [n] of effective unshared electron pairs, the molecular weight [M], and the effective unshared electron pair content ratio [n/M] of the compounds used here.

Next, after the pressure in the first vacuum tank was reduced to 4 × 10⁻⁴ Pa, electricity was supplied to the heating boat containing each compound so that heat can be applied thereto, and the nitrogen-containing layer 3 (base layer not containing nitrogen in Sample 6) was formed of each compound with a thickness of 25 nm on the transparent substrate 11 at an evaporation speed of 0.1 nm/sec to 0.2 nm/sec.

Next, while the atmosphere was maintained vacuum, the transparent substrate 11 with the nitrogen-containing layer 3 (base layer) formed thereon was transferred to the second vacuum tank. Then, after it was confirmed that the pressure was reduced to 4 × 10⁻⁴ Pa, electricity was supplied to the heating boat containing silver so that heat was applied thereto . Thus, the electrode layer 5 including silver with a thickness of 8 nm was formed at an evaporation speed of 0.1 nm/sec to 0.2 nm/sec, and the transparent electrode 1 of each of Samples 6 to 25 having the multilayer structure of the nitrogen-containing layer 3 (base layer) and the electrode layer 5 thereon was obtained.

### <Manufacture of transparent electrode of Samples 26 and 27>

With reference to Fig. 2, in each of Samples 26 and 27, the nitrogen-containing layer 3 (with a thickness of 25 nm) including Compound No. 7 was formed on one main plane of the transparent substrate 11 and the intermediate layer A including each material was formed subsequently by an evaporation method. In addition, the electrode layer 5 (with a thickness of 8 nm) including silver (Ag) was formed by an evaporation method. Thus, the transparent electrode 1a with the three-layer structure of the nitrogen-containing layer 3, the intermediate layer A, and the electrode layer 5 was manufactured.

On this occasion, the step of evaporating the intermediate layer A was added between the evaporation of the nitrogen-containing layer 3 and the evaporation of the electrode layer 5 described in the manufacture of the transparent electrode of Sample 15. In the added step of evaporating the intermediate layer A, magnesium (Mg) was evaporated with a thickness of 0.5 nm in Sample 26 and anthracene was evaporated with a thickness of 0.5 nm in Sample 27. Thus, the transparent electrode 1a in each of Samples 26 and 27, which has the three-layer structure of the nitrogen-containing layer 3, the intermediate layer A, and the electrode layer 5 continuously formed, was obtained.

### <Evaluation of each sample of Example 1>

The sheet resistance (surface resistance) and the visibility of letters of each transparent electrode of Samples 1 to 27 manufactured as above were evaluated. The sheet resistance was measured using a resistance meter (MCP-T610 manufactured by Mitsubishi Chemical Corporation) by the four-terminal and four-probe constant-current application method. In regard to the visibility of letters, the transparent substrate provided with the transparent electrode formed was overlapped on the image representing letters and the visibility therethrough was evaluated in five grades. In the five-grade evaluation, the normal line direction relative to the electrode surface of the transparent electrode was set to 0°, and the evaluation was conducted from two angles of 0° and 45° and their average value was used as the evaluation result. The evaluation results are shown below in Table 2.

**[Table 2]**

| Sample | Transparent substrate 11 | Transparent electrode for touch panel | | | | | | | Evaluation result | | Others |
|---|---|---|---|---|---|---|---|---|---|---|---|
| | Material | Nitrogen-containing layer 3 (thickness: 25 nm) | | | | Intermediate layer A | Electrode layer 5 | | Sheet resistance Ω/sq. | Visibility of letters | |
| | | Compound | Number [n] of effective unshared electron pairs | Molecular weight [M] | [n/M] | Compound | Compound | Thickness nm | | | |
| 1 | PET | - | - | - | - | - | ITO | 100 | 154 | 4.2 | Comparison |
| 2 | | - | - | - | - | - | Ag nanowire | 50 | 150 | 3.2 | |
| 3 | | - | - | - | - | - | | 150 | 43.3 | 2.1 | |
| 4 | | - | - | - | - | - | | 200 | 15.2 | 1.5 | |
| 5 | | - | - | - | - | - | Ag | 8 | Unmeasurable | 3.3 | |
| 6 | PET | No.-1 (anthracene) | 0 | 178.23 | 0.0E+00 | - | Ag | 8 | Unmeasurable | 3.1 | |
| 7 | PET | No.-2 | 1 | 839.00 | 1.2E-03 | - | Ag | 8 | 98.4 | 4.5 | Comparison |
| 8 | | No.-3 | 1 | 650.77 | 1.5E-03 | - | | | 95.4 | 4.6 | * |
| 9 | | No.1 | 1 | 500.55 | 2.0E-03 | - | | | 45.8 | 4.5 | Comparison |
| 10 | | No.2 | 2 | 790.95 | 2.5E-03 | - | | | 40.1 | 4.6 | Comparison |
| 11 | | No.3 | 2 | 655.81 | 3.0E-03 | - | | | 32.7 | 4.5 | Comparison |
| 12 | | No.4 | 2 | 655.81 | 3.0E-03 | - | | | 29.4 | 4.7 | Comparison |
| 13 | | No. 5 | 3 | 974.18 | 3.1E-03 | - | | | 24.5 | 4.5 | Comparison |
| 14 | | No.6 | 3 | 808.99 | 3.7E-03 | - | | | 16.3 | 4.6 | Comparison |
| 15 | | No.7 | 4 | 716.83 | 5.6E-03 | - | | | 6.8 | 4.8 | * |
| 16 | | No.8 | 6 | 1036.19 | 5.8E-03 | - | | | 11.9 | 4.5 | * |
| 17 | PET | No.9 | 4 | 551.64 | 7.3E-03 | - | Ag | 8 | 11.3 | 4.6 | Comparison |
| 18 | | No.10 | 4 | 516.60 | 7.7E-03 | - | | | 7.1 | 4.7 | * |
| 19 | | No.11 | 5 | 539.63 | 9.3E-03 | - | | | 9.5 | 4.7 | Comparison |
| 20 | | No.12 | 6 | 646.76 | 9.3E-03 | - | | | 9.8 | 4.5 | Comparison |
| 21 | | No.13 | 4 | 412.45 | 9.7E-03 | - | | | 9.4 | 4.7 | * |
| 22 | | No.14 | 6 | 616.71 | 9.7E-03 | - | | | 7.2 | 4.7 | Comparison |
| 23 | | No.15 | 5 | 463.53 | 1.1E-02 | - | | | 8.3 | 4.5 | Comparison |
| 24 | | No.16 | 6 | 540.62 | 1.1E-02 | - | | | 8.9 | 4.6 | Comparison |
| 25 | | No.18 | 6 | 312.33 | 1.9E-02 | - | | | 9.9 | 4.6 | Comparison |
| 26 | PET | No.7 | 4 | 716.83 | 5.6E-03 | Mg(0.5nm) | Ag | 8 | 9.5 | 4.6 | * |
| 27 | | | | | | Anthracene (0.5nm) | | | 11.5 | 4.7 | * |

| | | | | | | | | | | | |
|---|---|---|---|---|---|---|---|---|---|---|---|
| * = according to the present invention | | | | | | | | | | | |

### <Evaluation result of Example 1>

As is clear from Table 2, in the transparent electrodes of Samples 7 to 27, i.e., the transparent electrode in which the electrode layer 5 mainly containing silver was stacked on the nitrogen-containing layer 3, the electrode layer 5 including silver, which substantially served as the conductive layer, was as thin as 8 nm but the sheet resistance value thereof can be measured, and it has been confirmed that the thickness is substantially uniform by the film growth of the single layer growth type (Frank-van der Merwe: FM type). This result similarly applies to the transparent electrodes of Samples 26 and 27 in which the extremely thin intermediate layer A is provided between the nitrogen-containing layer 3 and the electrode layer 5.

On the other hand, the transparent electrode of Sample 1, which had the single layer structure including ITO, had a thickness as large as 100 nm but the sheet resistance was higher than that of the transparent electrodes of Samples 7 to 27. In the transparent electrode of Samples 2 to 4, which had the single layer structure including the silver nanowire, the sheet resistance relative to the thickness was higher than that of the transparent electrode of Samples 7 to 27 and moreover, in Sample 4, where the sheet resistance was the lowest, the visibility of letters was as low as 1.5 due to the light scattering.

Moreover, in regard to the transparent electrode of Samples 5 and 6, which had the single layer structure of just the electrode layer of silver without the nitrogen-containing layer, and the transparent electrode in which the electrode layer including silver was stacked on the base layer of Compound No.-1 (anthracene) not containing nitrogen atoms, the sheet resistance was unable to be measured and the use as the electrode was impossible.

In particular, in the transparent electrode of Samples 9 to 27, in which the nitrogen-containing layer was formed using any of Compounds No. 1 to No. 16 and No. 18 whose effective unshared electron pair content ratio [n/M] was in the range of 2.0 × 10⁻³ ≤ [n/M] ≤ 1.9 × 10⁻², the electrode layer including silver, which substantially served as the conductive layer, was as thin as 8 nm but the sheet resistance was as low as 45 Ω/sq. or less. Thus, it has been confirmed that the thickness is substantially uniform due to the film growth of the single layer growth type (Frank-van der Merwe: FM type).

Moreover, the transparent electrodes of Samples 9 to 27 whose effective unshared electron pair content ratio [n/M] was in the predetermined range had a letter visibility of 4.5 or more.

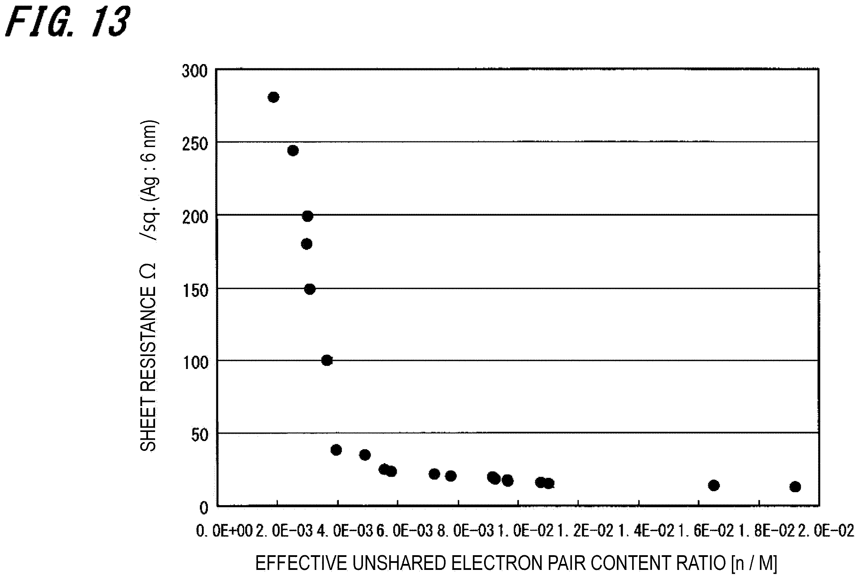

Note that Fig. 13 is the graph obtained by plotting the values of the sheet resistance measured in regard to the transparent electrodes in which the electrode layer with a thickness of 6 nm was provided on the nitrogen-containing layer including Compounds No. 1 to No. 20 whose effective unshared electron pair content ratio [n/M] was 2.0 × 10⁻³ ≤ [n/M] ≤ 1.9 × 10⁻², and the effective unshared electron pair content ratio [n/M] of the compound included in the nitrogen-containing layer.

The graph of Fig. 13 indicates that the sheet resistance of the transparent electrode tends to decrease as the value of the effective unshared electron pair content ratio [n/M] increases when the effective unshared electron pair content ratio [n/M] is in the range of 2.0 × 10⁻³ ≤ [n/M] ≤ 1.9 × 10⁻². Beyond the effective unshared electron pair content ratio [n/M] of 3.9 × 10⁻³, it has been confirmed that the sheet resistance was able to be reduced drastically as long as the effective unshared electron pair content ratio [n/M] was in the range of 3.9 × 10⁻³ ≤ [n/M].

The above results also applied to the case in which the nitrogen-containing layer was formed by the applied film.

Thus, by selecting and using the compound to be included in the nitrogen-containing layer provided adjacent to the electrode layer based on the effective unshared electron pair content ratio [n/M], the electrode film (i.e., transparent electrode) with the low resistance despite of having the thickness small enough to secure the light-transmitting property can be obtained.

### Example 2

### <Manufacture of transparent electrode for touch panel>

The transparent electrode for a touch panel with the high-refractive-index layer of each of Samples 206 to 334 (hereinafter referred to as the transparent electrode) was manufactured on the transparent substrate to have an area of 5 cm × 5 cm. A polyethylene terephthalate (PET) substrate was prepared as the transparent substrate. Table 3 below shows the structure of each layer of each transparent electrode of Samples 206 to 234 and the structure of each layer of each transparent electrode of Samples 1 to 5 manufactured to be compared in Example 1 above. Description is hereinafter made of the procedure of manufacturing each transparent electrode of Samples 206 to 234.

### <Manufacture of transparent electrode of Sample 206>

The high-refractive-index layer made of titanium oxide (TiO₂) was formed with a thickness of 30 nm on one main plane of the transparent substrate and the electrode layer made of silver was formed thereon with a thickness of 8 nm. Thus, the transparent electrode with the multilayer structure of the high-refractive-index layer and the electrode layer thereon was manufactured.

On this occasion, the transparent substrate was fixed to the base material holder of the commercial electron beam evaporation device, and titanium oxide (TiO₂) was put into the heating boat and these substrate holder and heating boat were attached to the vacuum tank of the electron beam evaporation device. Moreover, silver (Ag) was put into the resistive heating boat made of tungsten and attached to the vacuum tank of the vacuum evaporation device.

Next, after the pressure in the vacuum tank of the electron beam evaporation device was reduced to 4 × 10⁻⁴ Pa, the heating boat containing titanium oxide was heated by being irradiated with the electron beam and the high-refractive-index layer made of titanium oxide was provided with a thickness of 30 nm on the transparent substrate at an evaporation speed of 0.1 nm/sec to 0.2 nm/sec.

Next, the transparent substrate on which the high-refractive-index layer has been formed was transferred to the vacuum tank of the vacuum evaporation device while the atmosphere was maintained vacuum, and the pressure in the vacuum tank was reduced to 4 × 10⁻⁴ Pa. Subsequently, electricity was supplied to the heating boat containing silver so that heat was applied thereto. Thus, the electrode layer made of silver with a thickness of 8 nm was formed at an evaporation speed of 0.1 nm/sec to 0.2 nm/sec.

### <Manufacture of transparent electrode of Samples 207 to 231>

With reference to Fig. 3, in each of Samples 207 to 231, the high-refractive-index layer H made of titanium oxide (TiO₂) was formed with a thickness of 30 nm on one main plane of the transparent substrate 11, and then the nitrogen-containing layer 3 including each compound shown in Table 3 was formed with the thickness shown in Table 3 by the evaporation method. Subsequently, the electrode layer 5 made of silver (Ag) was formed in each thickness shown in Table 3 by the evaporation method. Thus, the transparent electrode 1b with the three-layer structure including the high-refractive-index layer H, the nitrogen-containing layer 3, and the electrode layer 5 was manufactured. Note that in Sample 207, the base layer not containing nitrogen was formed instead of the nitrogen-containing layer.

On this occasion, first, the transparent substrate 11 was fixed to the base material holder of the commercial electron beam evaporation device and titanium oxide (TiO₂) was put into the heating boat. Then, these substrate holder and heating boat were attached to the vacuum tank of the electron beam evaporation device. Moreover, each compound shown in Table 3 below was put into the resistive heating boat made of tantalum and attached to the first vacuum tank of the vacuum evaporation device. Moreover, silver (Ag) was put into the resistive heating boat made of tungsten and attached to the second vacuum tank of the vacuum evaporation device.

Among the compounds used here, Compounds No.-1, No.-2, and No.-3 are the compounds represented by the above structure formulae. On the other hand, Compounds No. 1 to No. 18 are the compounds containing nitrogen atoms shown in this embodiment. Table 3 below also shows the number [n] of effective unshared electron pairs, the molecular weight [M], and the effective unshared electron pair content ratio [n/M] of the compounds used here.

Subsequently, after the pressure in the vacuum tank of the electron beam evaporation device was reduced to 4 × 10⁻⁴ Pa, the heating boat containing titanium oxide was heated by being irradiated with the electron beam. Thus, the high-refractive-index layer H made of titanium oxide with a thickness of 30 nm was formed on the transparent substrate at an evaporation speed of 0.1 nm/sec to 0.2 nm/sec.

Next, the transparent substrate on which the high-refractive-index layer has been formed was transferred to the first vacuum tank of the vacuum evaporation device while the atmosphere was maintained vacuum. After the pressure in the first vacuum tank was reduced to 4 × 10⁻⁴ Pa, electricity was supplied to the heating boat containing each compound so that heat was applied thereto. Thus, the nitrogen-containing layer 3 (base layer not containing nitrogen in Sample 207) including each compound with each film thickness was formed on the high-refractive-index layer H at an evaporation speed of 0.1 nm/sec to 0.2 nm/sec.

Next, the transparent substrate 11 on which the nitrogen-containing layer 3 (base layer) has been formed was transferred to the second vacuum tank while the atmosphere was maintained vacuum, and then the pressure in the second vacuum tank was reduced to 4 × 10⁻⁴ Pa. Then, electricity was supplied to the heating boat containing silver so that heat was applied thereto. Thus, the electrode layer 5 made of silver with a thickness of 8 nm was formed at an evaporation speed of 0.1 nm/sec to 0.2 nm/sec. As a result, each transparent electrode 1b of Samples 207 to 231 with the multilayer structure of the high-refractive-index layer H, the nitrogen-containing layer 3 (base layer), and the electrode layer 5 was obtained.

### <Manufacture of transparent electrode of Samples 232 to 234>

The transparent electrode 1b of each of Samples 232 to 234 was obtained through a procedure similar to that of Sample 230 except the compound shown in Table 3 below was used for the high-refractive-index layer H.

### <Evaluation of each sample of Example 2>

The sheet resistance (surface resistance) and the visibility of letters of each transparent electrode of Samples 206 to 234 manufactured as above in Example 2 were evaluated in a manner similar to Example 1. The evaluation results of these are shown below in Table 3 in addition to the evaluation results of Samples 1 to 5 as the comparative examples manufactured in Example 1.

**[Table 3]**

| Sample | Transparent substrate 11 | Transparent electrode for touch panel | | | | | | | | Evaluation result | | Others |
|---|---|---|---|---|---|---|---|---|---|---|---|---|
| | | High-refractive-index layer H (thickness 30 nm) | Nitrogen-containing layer 3 | | | | | Electrode layer 5 | | | | |
| | Material | Compound | Compound | Number [n] of effective unshared .. electron pairs | Molecular weight [M] | [n/M] | Thickness nm | Compound | Thickness nm | Sheet resistance Ω/sq. | Visibility of letters | |
| 1 | PET | - | - | - | - | - | - | ITO | 100 | 154.0 | 4.2 | Comparison |
| 2 | | - | - | - | - | - | - | Ag nanowire | 50 | 150 | 3.2 | |
| 3 | | - | - | - | - | - | - | | 100 | 43.3 | 2.1 | |
| 4 | | - | - | - | - | - | - | | 150 | 15.2 | 1.5 | |
| 5 | | - | - | - | - | - | - | Ag | 8 | Unmeasurale | 3.3 | |
| 206 | PET | TᵢO₂ | - | - | - | - | - | Ag | 8 | Unmeasurale | 3.1 | |
| 207 | | | No.-1 | 0 | 178.23 | 0.0E+00 | 10 | | | Unmeasurable | 3.2 | |
| 208 | PET | TᵢO₂ | **No.**-2 | 1 | 839.00 | 1.2E-03 | 10 | Ag | 8 | 102.0 | 3.9 | Comparison |
| 209 | | | | | | | 5 | | | 101.0 | 3.6 | Comparison |
| 210 | | | | | | | 3 | | | 103.0 | 4.2 | Comparison |
| 211 | PET | TᵢO₂ | No.-3 | 1 | 850.77 | 1.5E-03 | 3 | Ag | 8 | 102.0 | 4.2 | * |
| 212 | | | No.1 | 1 | 500.55 | 2.0E-03 | | | | 76.6 | 4.5 | Comparison |
| 213 | | | No.2 | 2 | 790.95 | 2.5E-03 | | | | 70.3 | 4.5 | Comparison |
| 214 | | | No.3 | 2 | 655.81 | 3.0E-03 | | | | 57.1 | 4.5 | Comparison |
| 215 | | | No.4 | 2 | 655.81 | 3.0E-03 | | | | 51.4 | 4.5 | Comparison |
| 216 | | | No.5 | 3 | 974.14 | 3.1E-03 | | | | 42.9 | 4.5 | Comparison |
| 217 | | | No.6 | 3 | 808.99 | 3.7E-03 | | | | 30.3 | 4.5 | Comparison |
| 218 | | | No.7 | 4 | 716.83 | 5.6E-03 | | | | 16.7 | 4.8 | * |
| 219 | | | No.8 | 6 | 1036.19 | 5.8E-03 | | | | 15.9 | 4.7 | * |
| 220 | | | No.9 | 4 | 551.64 | 7.3E-03 | | | | 15.0 | 4.7 | Comparison |
| 221 | PET | TᵢO₃ | No.10 | 4 | 516.60 | 7.7E-03 | 3 | Ag | 8 | 13.5 | 4.7 | * |
| 222 | | | No.11 | 5 | 539.63 | 9.3E-03 | | | | 12.7 | 4.7 | Comparison |
| 223 | | | No.12 | 6 | 646.76 | 9.3E-03 | | | | 13.0 | 4.7 | Comparison |
| 224 | | | No.13 | 4 | 412.45 | 9.7E-03 | | | | 12.5 | 4.8 | * |
| 225 | | | No.14 | 6 | 616.71 | 9.7E-03 | | | | 12.3 | 4.8 | Comparison |
| 226 | | | No.15 | 5 | 463.53 | 1.1E-02 | | | | 10.7 | 4.8 | Comparison |
| 227 | | | No.16 | 6 | 540.62 | 1.1E-02 | | | | 10.0 | 4.8 | Comparison |
| 228 | | | No.17 | 9 | 543.58 | 1.7E-02 | | | | 9.3 | 4.7 | Comparison |
| 229 | | | No. 18 | 6 | 312.33 | 1.9E-02 | | | | 8.0 | 4.7 | Comparison |
| 230 | PET | TᵢO₃ | No.7 | 4 | 716.83 | 5.6E-03 | 3 | Ag | 10 | 8.3 | 4.8 | * |
| 231 | | | No.14 | 6 | 616.71 | 9.7E-03 | | | | 6.2 | 4.8 | Comparison |
| 232 | PET | Nb₂O₅ | No.7 | 4 | 716.83 | 5.6E-03 | 3 | Ag | 10 | 8.4 | 4.5 | * |
| 233 | | CdO | | | | | | | | 8.5 | 4.5 | * |
| 234 | | ITO | | | | | | | | 8.2 | 4.5 | * |

| | | | | | | | | | | | | |
|---|---|---|---|---|---|---|---|---|---|---|---|---|
| * = according to the present invention | | | | | | | | | | | | |

### <Evaluation result of Example 2>

As is clear from Table 3, in the transparent electrodes of Samples 208 to 234, i.e., the transparent electrode 1b in which the high-refractive-index layer H, the nitrogen-containing layer 3, and the electrode layer 5 were stacked in this order, the electrode layer 5 including silver, which substantially served as the conductive layer, was as thin as 8 nm or 10 nm but the sheet resistance value thereof was measurable, and it has been confirmed that the thickness is substantially uniform due to the film growth of the single layer growth type (Frank-van der Merwe: FM type).

In contrast to this, the transparent electrode of Sample 1 with the single layer structure of ITO had higher sheet resistance than the transparent electrode of Samples 208 to 234 despite of having the thickness as large as 100 nm. Further, the transparent electrode of Samples 2 to 4 with the single layer structure including the silver nanowire had sheet resistance to thickness higher than the transparent electrode of Samples 208 to 234, and Sample 4 with the lowest sheet resistance had the visibility of letters as low as 1.5 because of light scattering.

Moreover, in regard to the transparent electrode of Samples 5, 206, and 207, i.e., the transparent electrode with the single layer structure not including the nitrogen-containing layer but including only the electrode layer made of silver, the transparent electrode formed by stacking the electrode layer made of silver without having the nitrogen-containing layer on the high-refractive-index layer, and the transparent electrode formed by stacking the high-refractive-index layer, the base layer of Compound No.-1 (anthracene) not containing nitrogen atoms, and the electrode layer made of silver in this order, the sheet resistance was unable to be measured and the use as the electrode was impossible.

In particular, the transparent electrode of Samples 212 to 234, i.e., the transparent electrode in which the nitrogen-containing layer was formed using any of Compounds No.1 to No. 18 whose effective unshared electron pair content ratio [n/M] was in the predetermined range of 2.0 × 10⁻³ ≤ [n/M] 1.9 × 10⁻² had a sheet resistance as low as 76 Ω/sq. despite of having the thickness of the electrode layer, which was formed of silver and substantially served as a conductive layer, as small as 8 nm or 10 nm. Thus, it has been confirmed that the thickness is substantially uniform due to the film growth of the single layer growth type (Frank-van der Merwe: FM type).

In addition, the transparent electrode of Samples 212 to 234 whose effective unshared electron pair content ratio [n/M] was in the predetermined range had a visibility of letters of 4.5 or more.

Moreover, the transparent electrodes of Samples 208 to 210 are different only in the film thickness of the nitrogen-containing layer, and the comparison among these indicates that the transparent electrodes of Samples 209 and 210 whose thickness of nitrogen-containing layer was 5 nm or less had a visibility of letters as high as 3.6 or more. Note that the sheet resistance of these transparent electrodes was substantially the same.

The transparent electrodes of Samples 230 and 232 to 234 are different only in the compound used for the high-refractive-index layer, and the compounds thereof have a refractive index of TiO₂: n = 2.3 to 2.4, Nb₂O₅: n = 2.3, CdO: n = 2.49, and ITO: n = 2.1 to 2.2. In this manner, it has been confirmed that any compound had 0.3 or more higher refractive index than the nitrogen-containing layer (n = 1.7 to 1.8) and a visibility of letters of 4.5 or more and a sheet resistance that is one digit, which is very low.

### Example 3

### <<Manufacture of touch panel>>

Each of the transparent electrodes of Samples 7 to 27 manufactured in Example 1 and each of the transparent electrodes of Samples 208 to 234 manufactured in Example 2 were overlapped to manufacture each simple touch panel.

### <Evaluation and evaluation result of each sample of Example 3>

The visibility of letters of each touch panel was evaluated by a method similar to Example 1. As a result, it has been confirmed that the visibility of letters was as high as the transparent electrodes of Samples 7 to 27 and the transparent electrodes of Samples 208 to 234.

### Reference Signs List

1, 1a, 1b transparent electrode for touch panel (transparent electrode)
1-1, 1b-1 first transparent electrode
1-2, 1b-2 second transparent electrode
3 nitrogen-containing layer
3-1 first nitrogen-containing layer
3-2 second nitrogen-containing layer
5 electrode layer
5-1 first electrode layer
5-2 second electrode layer
5x1, 5x2, 5x3, ... x electrode pattern (first electrode layer)
5y1, 5y2, 5y3, ... y electrode pattern (second electrode layer)
21, 23, 25, 27, 29 ... touch panel
31 display device
33 display panel
A intermediate layer
H high-refractive-index layer
H-1 first high-refractive-index layer
H-2 second high-refractive-index layer

## Claims

1. A transparent electrode (1, 1a, 1b) for a touch panel, comprising:
a nitrogen-containing layer (3) formed using a compound containing nitrogen atoms; and
an electrode layer (5) mainly containing silver and provided stacking on the nitrogen-containing layer (3),
wherein the transparent electrode (1, 1a, 1b) for a touch panel is **characterized in that** the nitrogen-containing layer (3) contains a compound represented by General Formula (1) below 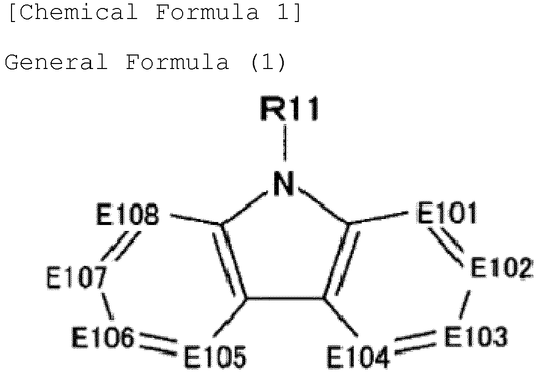
[In General Formula (1), each of E101 to E108 represents -C(R12)= or -N=, at least one of E101 to E108 represents -N=, and R11 and R12 represent a hydrogen atom or a substituent.]

2. The transparent electrode (1, 1a, 1b) for a touch panel according to claim 1, **characterized in that** the nitrogen-containing layer (3) contains a compound represented by General Formula (2) below. 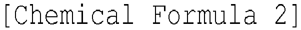 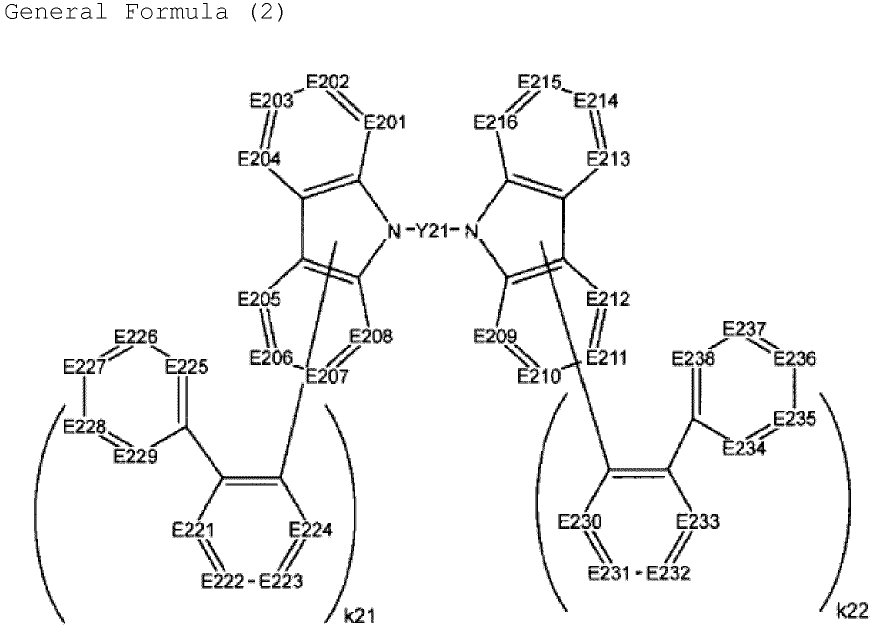 [In General Formula (2), Y21 represents an arylene group, a heteroarylene group, or a divalent connecting group including a combination thereof, each of E201 to E216 and E221 to E238 represents -C(R21)= or -N=, R21 represents a hydrogen atom or a substituent, at least one of E221 to E229 and at least one of E230 to E238 represent -N=, k21 and k22 represent an integer of 0 to 4 and k21 + k22 is an integer of 2 or more.]

3. The transparent electrode (1, 1a, 1b) for a touch panel according to claim 1, **characterized in that** the nitrogen-containing layer (3) contains a compound represented by General Formula (3) below. 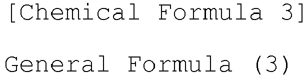 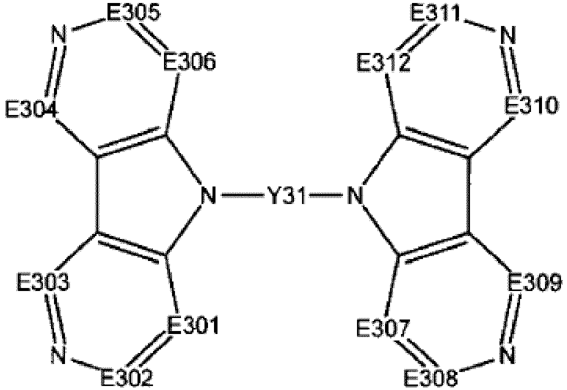 [In General Formula (3), each of E301 to E312 represents -C(R31)=, R31 represents a hydrogen atom or a substituent, and Y31 represents an arylene group, a heteroarylene group, or a divalent connecting group including a combination thereof.]

4. The transparent electrode (1, 1a, 1b) for a touch panel according to claim 1, **characterized in that** the nitrogen-containing layer (3) contains a compound represented by General Formula (4) below. 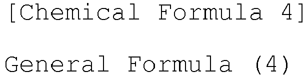 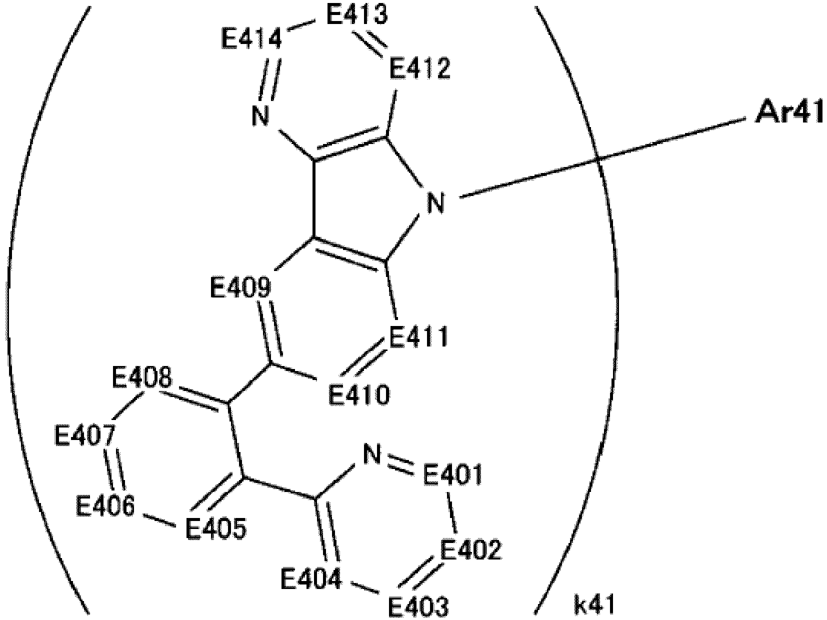 [In General Formula (4), each of E401 to E414 represents -C(R41) =, R41 represents a hydrogen atom or a substituent, Ar41 represents a substituted or unsubstituted aromatic hydrocarbon ring or heteroaromatic ring, and k41 represents an integer of 3 or more.]

5. The transparent electrode (1, 1a, 1b) for a touch panel according to any one of claims 1 to 4, **characterized in that** an effective unshared electron pair content ratio [n/M] of the compound containing nitrogen atoms satisfies 2.0 × 10⁻³ ≤ [n/M], where n represents the number of unshared electron pairs which are not related to an aromatic property and are not coordinated to metal among unshared electron pairs of the nitrogen atoms and M represents the molecular weight of the compound.

6. The transparent electrode (1, 1a, 1b) for a touch panel according to any one of claims 1 to 4, **characterized in that** the effective unshared electron pair content ratio [n/M] of the compound satisfies 3.9 × 10⁻³ ≤ [n/M].

7. The transparent electrode (1, 1a, 1b) for a touch panel according to one any of claims 1 to 4, **characterized in that** the nitrogen-containing layer (3) is formed using the compound containing nitrogen atoms and using a mixture of such compound with another compound, and an average value of the effective unshared electron pair content ratio [n/M] considering a mixing ratio of these compounds satisfies 2.0 × 10⁻³ ≤ [n/M].

8. The transparent electrode (1, 1a, 1b) for a touch panel according to any of claims 1 to 4, **characterized in that** it further comprises a high-refractive-index layer (H) provided with the nitrogen-containing layer (3) interposed between the electrode layer (5) and the high-refractive-index layer (H) and having a higher refractive index than the nitrogen-containing layer (3).

9. The transparent electrode (1, 1a, 1b) for a touch panel according to any one of claims 1 to 4, **characterized in that** the high-refractive-index layer (H) is formed of titanium oxide or niobium oxide.

10. The transparent electrode (1, 1a, 1b) for a touch panel according to any one of claim 1 to 4, **characterized in that** the nitrogen-containing layer (3) has a thickness of 5 nm or less.

11. The transparent electrode (1, 1a, 1b) for a touch panel according to any of claims 1 to 4, **characterized in that** the nitrogen-containing layer (3) and the electrode layer (5) are provided adjacent to each other.

12. A touch panel (21, 23, 25, 27, 29) **characterized in that** it comprises the transparent electrode (1, 1a, 1b) for a touch panel according to any of claims 1 to 4.

13. A display device (31) **characterized in that** it comprises:
the touch panel (21, 23, 25, 27, 29) according to any one of claims 1 to 4; and
a display panel (33) disposed overlapping with the touch panel.

## Patentansprüche

1. Transparente Elektrode (1, 1a, 1b) für ein Touch Panel, mit:
einer stickstoffhaltigen Schicht (3), die unter Verwendung einer Stickstoffatome enthaltenden Verbindung ausgebildet wird; und
einer hauptsächlich Silber enthaltenden Elektrodenschicht (5), die auf der stickstoffhaltigen Schicht (3) aufgetragen ist,
wobei die transparente Elektrode (1, 1a, 1b) für ein Touch Panel **dadurch gekennzeichnet ist, dass** die stickstoffhaltige Schicht (3) eine durch die unten gezeigte allgemeine Formel (1) dargestellte Verbindung enthält, 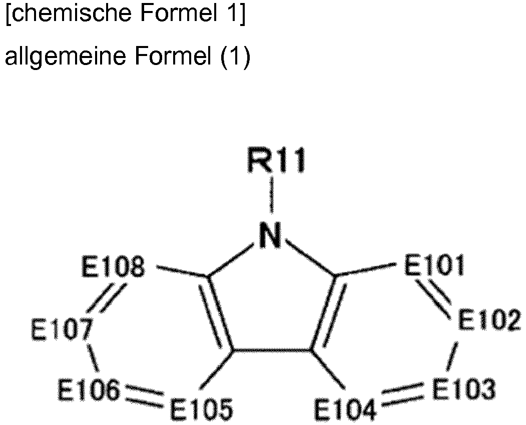
[In der allgemeinen Formel (1) stellt jedes E aus E101 bis E108 -C (R12) = oder -N= dar, zumindest ein E aus E101 bis E108 stellt -N= dar und R11 und R12 stellen ein Wasserstoffatom oder einen Substituenten dar.]

2. Transparente Elektrode (1, 1a, 1b) für ein Touch Panel gemäß Anspruch 1, **dadurch gekennzeichnet, dass** die stickstoffhaltige Schicht (3) eine durch die unten stehende allgemeine Formel (2) dargestellte Verbindung enthält. 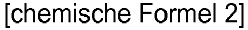 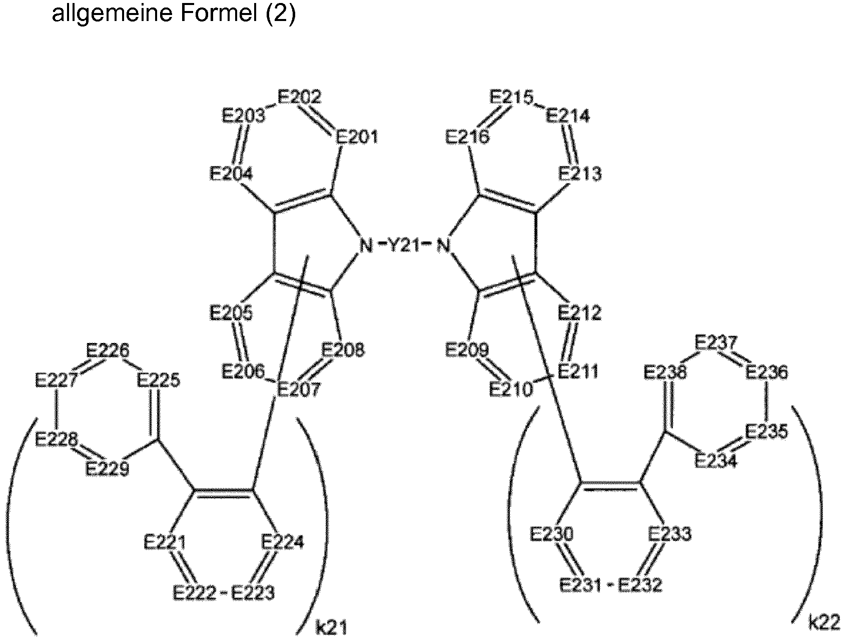 [In der allgemeinen Formel (2) stellt Y21 eine Arylen-Gruppe, eine Heteroarylen-Gruppe oder eine zweiwertige Verbindungsgruppe einschließlich einer Kombination daraus dar, jedes E aus E201 bis E216 und E221 bis E238 stellt -C (R21)= oder -N= dar, R21 stellt ein Wasserstoffatom oder einen Substituenten dar, zumindest ein E aus E221 bis E229 und zumindest ein E aus E230 bis E238 stellen -N= dar, k21 und k22 stellen eine ganze Zahl von 0 bis 4 dar und k21 + k22 ergibt eine ganze Zahl von 2 oder mehr.]

3. Transparente Elektrode (1, 1a, 1b) für ein Touch Panel gemäß Anspruch 1, **dadurch gekennzeichnet, dass** die stickstoffhaltige Schicht (3) eine durch die unten stehende allgemeine Formel (3) dargestellte Verbindung enthält. 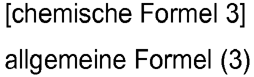 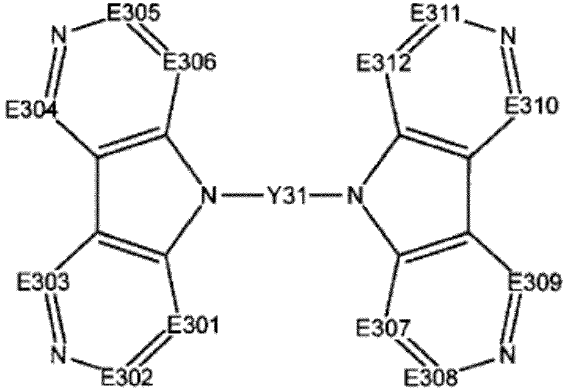 [In der allgemeinen Formel (3) stellt jedes E aus E301 bis E312 -C (R31)= dar, R31 stellt ein Wasserstoffatom oder einen Substituenten dar und Y31 stellt eine Arylen-Gruppe, eine Heteroarylen-Gruppe oder eine zweiwertige Verbindungsgruppe einschließlich einer Kombination daraus dar.]

4. Transparente Elektrode (1, 1a, 1b) für ein Touch Panel gemäß Anspruch 1, **dadurch gekennzeichnet, dass** die stickstoffhaltige Schicht (3) eine durch die unten stehende allgemeine Formel (4) dargestellte Verbindung enthält. 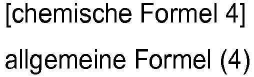 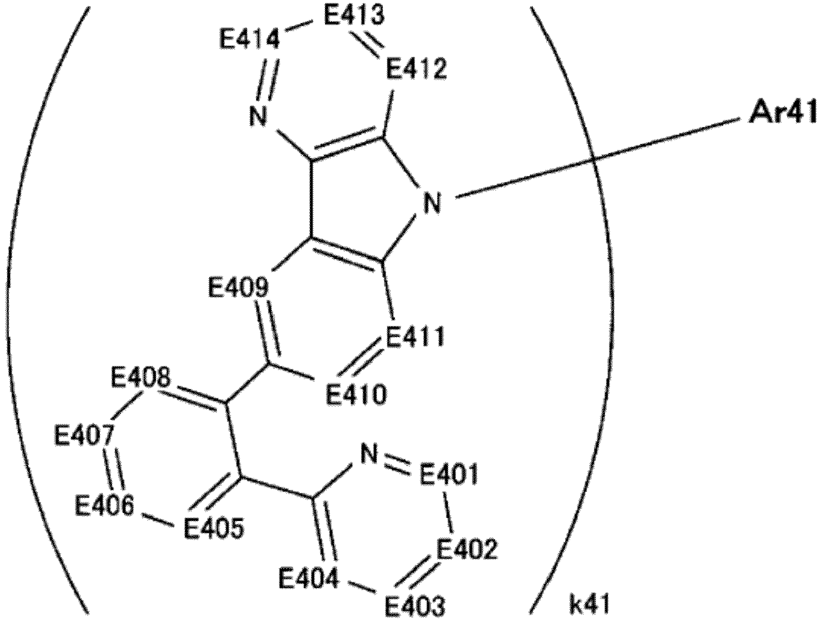 [In der allgemeinen Formel (4) stellt jedes E aus E401 bis E414 -C(R41)= dar, R41 stellt ein Wasserstoffatom oder einen Substituenten dar, Ar41 stellt einen substituierten oder nicht substituierten aromatischen Kohlenwasserstoffring oder heteroaromatischen Ring dar und k41 stellt eine ganze Zahl von 3 oder mehr dar.]

5. Transparente Elektrode (1, 1a, 1b) für ein Touch Panel gemäß irgendeinem der Ansprüche 1 bis 4, **dadurch gekennzeichnet, dass** ein effektiver Anteil an ungeteiltem Elektronenpaargehalt [n/M] der stickstoffhaltigen Verbindung 2,0 x 10⁻³ ≤ [n/M] genügt, wobei n die Anzahl an ungeteilten Elektronenpaaren darstellt, die nicht mit einer aromatischen Eigenschaft zusammenhängen und nicht mit Metall koordiniert sind, unter den ungeteilten Elektronenpaaren der Stickstoffatome und M das Molekulargewicht der Verbindung darstellt.

6. Transparente Elektrode (1, 1a, 1b) für ein Touch Panel gemäß irgendeinem der Ansprüche 1 bis 4, **dadurch gekennzeichnet, dass** der effektive Anteil an ungeteiltem Elektronenpaargehalt [n/M] der Verbindung 3,9 x 10⁻³ ≤ [n/M] genügt.

7. Transparente Elektrode (1, 1a, 1b) für ein Touch Panel gemäß irgendeinem der Ansprüche 1 bis 4, **dadurch gekennzeichnet, dass** die stickstoffhaltige Schicht (3) unter Verwendung der Stickstoffatome enthaltenden Verbindung und unter Verwendung eines Gemisches aus solch einer Verbindung mit einer anderen Verbindung gebildet wird, und dass ein Durchschnittswert des effektiven Anteils an ungeteiltem Elektronenpaargehalt [n/M] unter Berücksichtigung eines Mischungsverhältnisses dieser Verbindungen 2,0 x 10⁻³ ≤ [n/M] genügt.

8. Transparente Elektrode (1, 1a, 1b) für ein Touch Panel gemäß irgendeinem der Ansprüche 1 bis 4, **dadurch gekennzeichnet, dass** sie ferner eine Schicht (H) mit hohem Brechungsindex aufweist, die mit der zwischen der Elektrodenschicht (5) und der Schicht (H) mit hohem Brechungsindex angeordneten stickstoffhaltigen Schicht (3) bereitgestellt ist und einen höheren Brechungsindex aufweist als die stickstoffhaltige Schicht (3).

9. Transparente Elektrode (1, 1a, 1b) für ein Touch Panel gemäß irgendeinem der Ansprüche 1 bis 4, **dadurch gekennzeichnet, dass** die Schicht (H) mit hohem Brechungsindex aus Titanoxid oder Nioboxid gebildet ist.

10. Transparente Elektrode (1, 1a, 1b) für ein Touch Panel gemäß irgendeinem der Ansprüche 1 bis 4, **dadurch gekennzeichnet, dass** die stickstoffhaltige Schicht (3) eine Dicke von 5 nm oder weniger ausfweist.

11. Transparente Elektrode (1, 1a, 1b) für ein Touch Panel gemäß irgendeinem der Ansprüche 1 bis 4, **dadurch gekennzeichnet, dass** die stickstoffhaltige Schicht (3) und die Elektrodenschicht (5) angrenzend aneinander vorgesehen sind.

12. Touch Panel (21, 23, 25, 27, 29), **dadurch gekennzeichnet, dass** es die transparente Elektrode (1, 1a, 1b) für ein Touch Panel gemäß irgendeinem der Ansprüche 1 bis 4 aufweist.

13. Anzeigevorrichtung (31), **dadurch gekennzeichnet, dass** sie aufweist:
das Touch Panel (21, 23, 25, 17, 29) gemäß irgendeinem der Ansprüche 1 bis 4; und
ein Anzeigefeld (33), das in Überlappung mit dem Touch Panel angeordnet ist.

## Revendications

1. Electrode transparente (1, 1a, 1b) destinée à un écran tactile, comprenant :
une couche contenant de l'azote (3) formée en utilisant un composé contenant des atomes d'azote ; et
une couche d'électrode (5) principalement contenant de l'argent et disposée sur la couche contenant de l'azote (3),
dans lequel l'électrode transparente (1, 1a, 1b) destinée à un écran tactile est **caractérisée en ce que** la couche contenant de l'azote (3) contient un composé représenté par la formule générale (1) ci-dessous 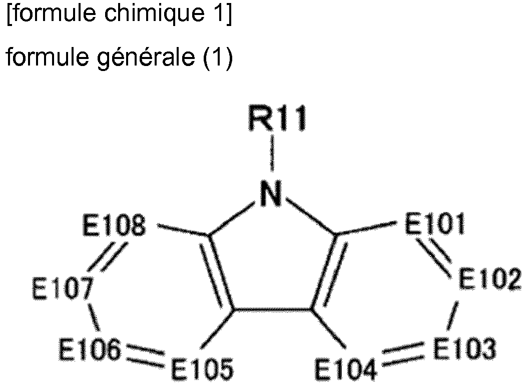
[Dans la formule générale (1) chacun de E101 à E108 représente -C(R12)= ou -N=, au moins un de E101 à E108 représente -N=, et R11 et R12 représentent un atome d'hydrogène ou un substituant.)

2. Electrode transparente (1, 1a, 1b) destinée à un écran tactile selon la revendication 1, **caractérisée en ce que** la couche contenant de l'azote (3) contient un composé représenté par la formule générale (2) ci-dessous 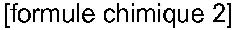 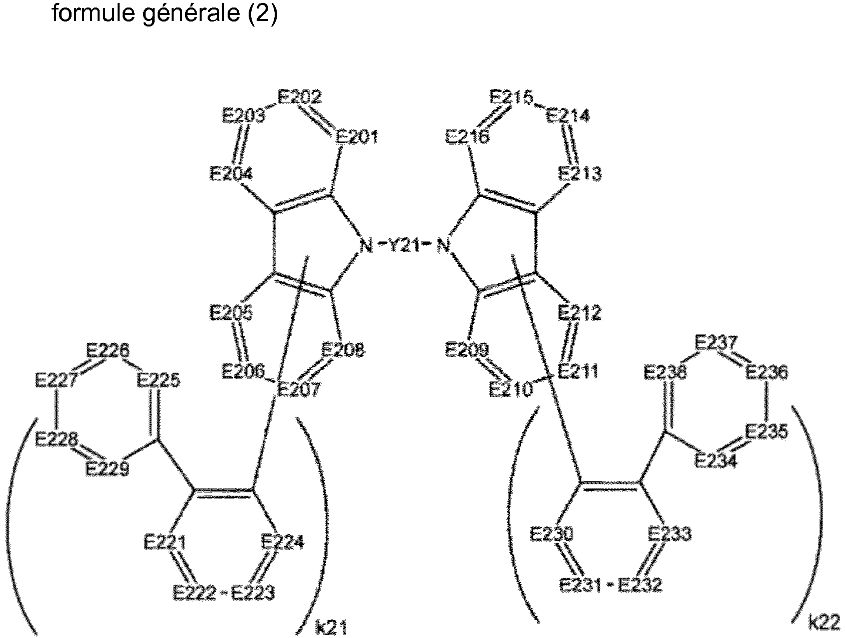 [dans la formule générale (2) Y21 représente un groupe arylène, un groupe hétéroarylène ou un groupe de liaison divalent, une combinaison de ceux-ci y compris, chacun de E201 à E216 et de E221 à E238 représente -C(R21)= ou -N=, R21 représente un atome d'hydrogène ou un substituant, au moins un de E221 à E229 et au moins un de E230 à E238 représentent -N=, k21 et k22 représentent un nombre entier de 0 à 4 et k21 + k22 est un nombre entier de 2 ou plus.]

3. Electrode transparente (1, 1a, 1b) destinée à un écran tactile selon la revendication 1, **caractérisée en ce que** la couche contenant de l'azote (3) contient un composé représenté par la formule générale (3) ci-dessous 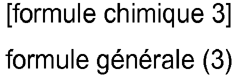 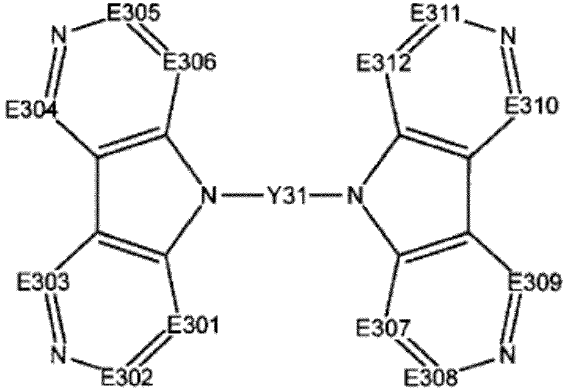 [dans la formule générale (3), chacun de E301 à 312 représente -C(R31)=, R31 représente un atome d'hydrogène ou un substituant et Y31 représente un groupe arylène, un groupe hétéroarylène ou un groupe de liaison divalent, une combinaison de ceux-ci y compris.]

4. Electrode transparente (1, 1a, 1b) destinée à un écran tactile selon la revendication 1, **caractérisée en ce que** la couche contenant de l'azote (3) contient un composé représenté par la formule générale (4) ci-dessous 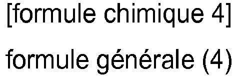 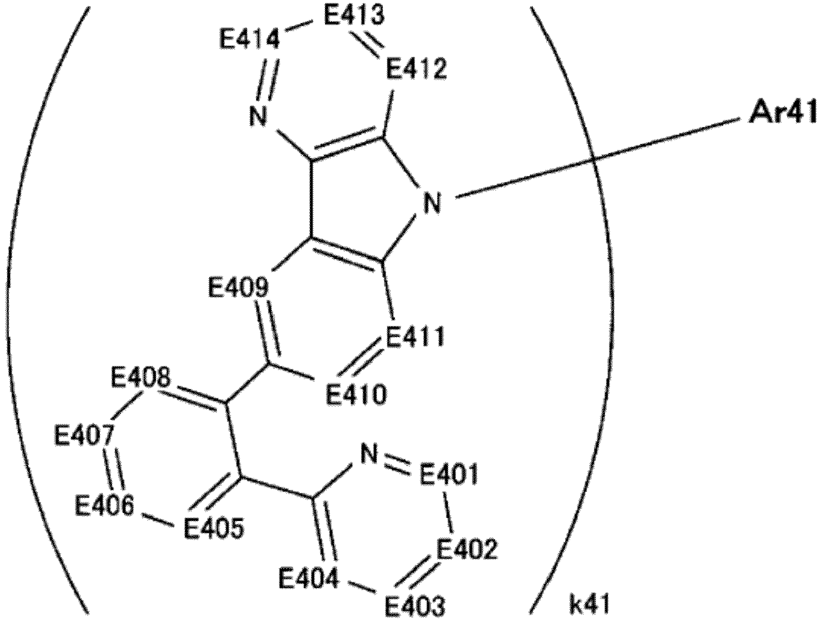 [dans la formule générale (4) chacun de E401 à E414 représente -C(41)=, R41 représente un atome d'hydrogène ou un substituant, Ar41 représente un cycle hydrocarboné aromatique ou un cycle hétéroaromatique substitué ou non substitué et k41 représente un nombre entier de 3 ou plus.]

5. Electrode transparente (1, 1a, 1b) destinée à un écran tactile selon l'une quelconque des revendications 1 à 4, **caractérisée en ce qu'**un rapport effectif de teneur en paires d'électrons non partagées [n/M] du composé contenant de l'azote satisfait à 2,0 x 10⁻³ ≤ [n/M], dans lequel n représente le nombre de paires d'électrons non partagées, qui ne sont pas associées à une propriété aromatique et ne sont pas coordonnées à un métal parmi des paires d'électrons non partagées des atomes d'azote et M représente le poids moléculaire du composé.

6. Electrode transparente (1, 1a, 1b) destinée à un écran tactile selon l'une quelconque des revendications 1 à 4, **caractérisée en ce que** le rapport effectif de teneur en paires d'électrons non partagées [n/M] du composé satisfait à 3,9 x 10⁻³ ≤ [n/M].

7. Electrode transparente (1, 1a, 1b) destinée à un écran tactile selon l'une quelconque des revendications 1 à 4, **caractérisée en ce que** la couche contenant de l'azote (3) est formée en utilisant le composé contenant des atomes d'azote et en utilisant un mélange d'un tel composé avec un autre composé, et une valeur moyenne du rapport effectif de teneur en paires d'électrons non partagées [n/M], étant donné un rapport de mélange de ces composés, satisfait à 2,0 x 10⁻³ ≤ [n/M].

8. Electrode transparente (1, 1a, 1b) destinée à un écran tactile selon l'une quelconque des revendications 1 à 4, **caractérisée en ce qu'**elle comprend en outre une couche à indice de réfraction élevé (H) prévue avec la couche contenant de l'azote (3) interposée entre la couche d'électrode (5) et la couche à indice de réfraction élevé (H) et ayant un indice de réfraction plus élevé que la couche contenant de l'azote (3).

9. Electrode transparente (1, 1a, 1b) destinée à un écran tactile selon l'une quelconque des revendications 1 à 4, **caractérisée en ce que** la couche à indice de réfraction élevé (H) est formée à partir d'oxyde de titane ou à partir d'oxyde de niobium.

10. Electrode transparente (1, 1a, 1b) destinée à un écran tactile selon l'une quelconque des revendications 1 à 4, **caractérisée en ce que** la couche contenant de l'azote (3) comprend une épaisseur de 5 nm ou moins.

11. Electrode transparente (1, 1a, 1b) destinée à un écran tactile selon l'une quelconque des revendications 1 à 4, **caractérisée en ce que** la couche contenant de l'azote (3) et la couche d'électrode sont disposées de manière adjacente l'une à l'autre.

12. Ecran tactile (21, 23, 25, 27, 29) **caractérisé en ce qu'**il comprend l'électrode transparente (1, 1a, 1b) destinée à un écran tactile selon l'une quelconque des revendications 1 à 4.

13. Dispositif d'affichage (31) **caractérisé en ce qu'**il comprend :
l'écran tactile (21, 23, 25, 27, 29) selon l'une des revendications 1 à 4 ; et un panneau d'affichage (33) disposé de manière chevauchante avec l'écran tactile.
